# FASCICULE DE BREVET EUROPEEN

(11) **EP 1 268 784 B1**
(45) Date de publication et mention de la délivrance du brevet: **16.03.2005**
(21) Numéro de dépôt: 01919586.6
(22) Date de dépôt: 27.03.2001
(51) Int. Cl.: C12N 15/12, C07K 14/47, C07K 16/18, C12Q 1/68, G01N 33/53, G01N 33/68, A61K 38/17, A61P 1/00

(54) **GENES IMPLIQUES DANS LES MALADIES INFLAMMATOIRES DE L'INTESTIN ET LEUR UTILISATION**
AN ENTZÜNDLICHEN DARMERKRANKUNGEN BETEILIGTE GENE UND DEREN VERWENDUNG
GENES INVOLVED IN INTESTINAL INFLAMMATORY DISEASES AND USE THEREOF

(30) Priorité: 27.03.2000 FR 0003832
(43) Date de publication de la demande: 02.01.2003
(73) Titulaire: Fondation Jean Dausset-Ceph, 75010 Paris (FR)
(72) Inventeur: HUGOT, Jean-Pierre, F-75019 Paris (FR); THOMAS, Gilles, F-75005 Paris (FR); ZOUALI, Mohamed, F-92220 Bagneux (FR); LESAGE, Suzanne, F-78700 Conflans-Sainte-Honorine (FR); CHAMAILLARD, Mathias, F-37300 Joue-les-Tours (FR)
(74) Mandataire: Ahner, Francis
(86) Numéro de dépôt international: PCT/FR2001/000935
(87) Numéro de publication internationale: WO 2001/072822

(56) Documents cités:
- EP-A- 1 074 617
- WO-A-00/58473
- WO-A-99/23255
- WO-A-99/40102
- WO-A-99/64576
- DATABASE EMBL [en ligne] ACCESSION NO:AC007728, 7 juin 1999 (1999-06-07) DOE JOINT GENOME INSTITUTE: "Homo sapiens chromosome 16 clone RP11-327F22, WORKING DRAFT SEQUENCE, 1 ordered pieces." XP002156657
- DATABASE EMBL [en ligne] ACCESSION NO: AC007608, 21 mai 1999 (1999-05-21) DOE JOINT GENOME INSTITUTE: "Homo sapiens chromosome 16 clone RP11-401P9, WORKING DRAFT SEQUENCE, 8 ordered pieces." XP002156658
- DATABASE EMBL [en ligne] ACCESSION NO: AQ534686, 18 mai 1999 (1999-05-18) ZHAO, S., ET AL.: "RPCI-11-384F21.TJ RPCI-11 Homo sapiens genomic clone RPCI-11-384F21,genomic survey sequence." XP002156659
- DATABASE EMBL [en ligne] ACCESSION NO: AI681116, 27 mai 1999 (1999-05-27) NCI-CGAP: "tx44b02.x1 NCI_CGAP_Lu24 Homo sapiens cDNA clone IMAGE:2272395 3', mRNA sequence." XP002156660
- DATABASE EMBL [en ligne] ACCESSION NO: AQ585409, 9 juin 1999 (1999-06-09) ZHAO, S., ET AL.: "RPCI-11-459C5.TV RPCI-11 Homo sapiens genomic clone RPCI-11-459C5, genomic survey sequence." XP002156661
- DATABASE EMBL [en ligne] ACCESSION NO:AI090427, 19 août 1998 (1998-08-19) NCI-CGAP: "oy82d10.s1 NCI_CGAP_CLL1 Homo sapiens cDNA clone IMAGE:1672339 3', mRNA sequence." XP002156662
- DATABASE EMBL [en ligne] ACCESSION NO: AQ176547, 21 septembre 1998 (1998-09-21) MAHAIRAS, G.G., ET AL.: "HS_3213_B1_C05_T7 CIT Approved Human Genomic Sperm Library D Homo sapiens genomic clone Plate=3213 Col=9 Row=F, genomic survey sequence." XP002156663
- DATABASE EMBL [en ligne] ACCESSION NO: AF178930, 23 novembre 2000 (2000-11-23) OGURA, Y., ET AL.: "Homo sapiens NOD2 protein (NOD2) mRNA, complete cds." XP002177310 -& OGURA, Y., ET AL.: "Nod2, a Nod1/Apaf-1 Family Member That Is Restricted to Monocytes and Activates NF-kappa B" THE JOURNAL OF BIOLOGICAL CHEMISTRY, vol. 276, no. 7, 16 février 2001 (2001-02-16), pages 4812-4818, XP002177307
- HUGOT JEAN-PIERRE ET AL: "Association of NOD2 leucine-rich repeat variants with susceptibility to Crohn's disease." NATURE (LONDON), vol. 411, no. 6837, 2001, pages 599-603, XP002177308 ISSN: 0028-0836
- DATABASE EMBL [en ligne] ACCESSION NO: AW082334, 18 octobre 1999 (1999-10-18) NCI-CGAP: "xb65f03.x1 Soares_NFL_T_GBC_S1 Homo sapiens cDNA clone IMAGE:2581181 3' similar to contains LTR1.t3 LTR1 repetitive element ;, mRNA sequence." XP002156664
- DATABASE EMBL [en ligne] ACCESSION NO: AA282390, 4 avril 1997 (1997-04-04) NCI-CGAP: "zs89a11.r1 NCI_CGAP_GCB1 Homo sapiens cDNA clone IMAGE:704636 5', mRNA sequence." XP002156665
- DATABASE EMBL [en ligne] ACCESSION NO: AA278249, 3 avril 1997 (1997-04-03) NCI-CGAP: "zs77c05.r1 NCI_CGAP_GCB1 Homo sapiens cDNA clone IMAGE:703496 5', mRNA sequence." XP002156666
- DATABASE EMBL [en ligne] ACCESSION NO: AW134842, 29 octobre 1999 (1999-10-29) NCI-CGAP: "UI-H-BI1-abs-e-09-0-UI.s1 NCI_CGAP_Sub3 Homo sapiens cDNA clone IMAGE:2713048 3', mRNA sequence." XP002156667
- DATABASE EMBL [en ligne] ACCESSION NO: AW104269, 21 octobre 1999 (1999-10-21) NCI-CGAP: "xd70h07.x1 Soares_NFL_T_GBC_S1 Homo sapiens cDNA clone IMAGE:2603005 3' similar to contains Alu repetitive element;contains element MER22 repetitive element ;, mRNA sequence." XP002156668
- DATABASE EMBL [en ligne] ACCESSION NO: AI377313, 28 janvier 1999 (1999-01-28) NCI-CGAP: "te60b02.x1 Soares_NFL_T_GBC_S1 Homo sapiens cDNA clone IMAGE:2091051 3' similar to contains element MSR1 MSR1 repetitive element ;, mRNA sequence." XP002156669
- HUGOT JEAN-PIERRE ET AL: "Mapping of a susceptibility locus for Crohn's disease on chromosome 16." NATURE (LONDON), vol. 379, no. 6568, 1996, pages 821-823, XP002156655 ISSN: 0028-0836 cité dans la demande
- MIRZA MUDDASSAR M ET AL: "Evidence of linkage of the inflammatory bowel disease susceptibility locus on chromosome 16 (IBD1) to ulcerative colitis." JOURNAL OF MEDICAL GENETICS, vol. 35, no. 3, mars 1998 (1998-03), pages 218-221, XP000971943 ISSN: 0022-2593
- HUGOT J P ET AL: "Fine mapping of the inflammatory bowel disease susceptibility locus 1 (IBD1) in the pericentromeric region of chromosome 16." GASTROENTEROLOGY, vol. 114, no. 4 PART 2, 15 avril 1998 (1998-04-15), page A999 XP000971941 Digestive Diseases Week and the 99th Annual Meeting of the American Gastroenterological Association;New Orleans, Louisiana, USA; May 16-22, 1998 ISSN: 0016-5085
- DATABASE SWISSPROT [en ligne] ACCESSION NO: Q9Y239, INOHARA, N., ET AL.: "NOD1 protein" XP002156670 -& INOHARA, N., ET AL.: "Nod 1, an Apaf-1-like activator of caspase-9 and nuclear factor-kappaB" THE JOURNAL OF BIOLOGICAL CHEMISTRY, vol. 274, no. 21, 21 mai 1999 (1999-05-21), pages 14560-14567, XP002156656

### LISTE DE SÉQUENCES

<110> Fondation Jean Dausset - CEPH
<120> Gènes impliqués dans les maladies inflammatoires de l'intestin et leur utilisation
<130> D18702
<160> 90
<170> PatentIn Ver. 2.1
<210> 1
   <211> 4322
   <212> ADN
   <213> Homo sapiens
<220>
   <221> CDS
   <222> (1)..(3123)
<400> 1
   atg gag aag aga agg ggt cta acc att gag tgc tgg ggc ccc caa agt    48
   Met Glu Lys Arg Arg Gly Leu Thr Ile Glu Cys Trp Gly Pro Gln Ser
    1               5                  10                  15
   ccc tca ctg acc ttg ttc tcc tcc cca ggt tgt gaa atg tgc tcg cag    96
   Pro Ser Leu Thr Leu Phe Ser Ser Pro Gly Cys Glu Met Cys Ser Gln
               20                  25                  30
   gag gct ttt cag gca cag agg agc cag ctg gtc gag ctg ctg gtc tca   144
   Glu Ala Phe Gln Ala Gln Arg Ser Gln Leu Val Glu Leu Leu Val Ser
           35                  40                  45
   ggg tcc ctg gaa ggc ttc gag agt gtc ctg gac tgg ctg ctg tcc tgg   192
   Gly Ser Leu Glu Gly Phe Glu Ser Val Leu Asp Trp Leu Leu Ser Trp
       50                  55                  60
   gag gtc ctc tcc tgg gag gac tac gag ggc ttc cac ctc ctg ggc cag   240
   Glu Val Leu Ser Trp Glu Asp Tyr Glu Gly Phe His Leu Leu Gly Gln
   65                  70                  75                  80
   cct ctc tcc cac ttg gcc agg cgc ctt ctg gac acc gtc tgg aat aag   288
   Pro Leu Ser His Leu Ala Arg Arg Leu Leu Asp Thr Val Trp Asn Lys
                   85                  90                  95
   ggt act tgg gcc tgt cag aag ctc atc gcg gct gcc caa gaa gcc cag   336
   Gly Thr Trp Ala Cys Gln Lys Leu Ile Ala Ala Ala Gln Glu Ala Gln
               100                 105                 110
   gcc gac agc cag tcc ccc aag ctg cat ggc tgc tgg gac ccc cac tcg   384
   Ala Asp Ser Gln Ser Pro Lys Leu His Gly Cys Trp Asp Pro His Ser
           115                 120                 125
   ctc cac cca gcc cga gac ctg cag agt cac cgg cca gcc att gtc agg   432
   Leu His Pro Ala Arg Asp Leu Gln Ser His Arg Pro Ala Ile Val Arg
       130                 135                 140
   agg ctc cac agc cat gtg gag aac atg ctg gac ctg gca tgg gag cgg   480
   Arg Leu His Ser His Val Glu Asn Met Leu Asp Leu Ala Trp Glu Arg
   145                 150                 155                 160
   ggt ttc gtc agc cag tat gaa tgt gat gaa atc agg ttg ccg atc ttc      528
   Gly Phe Val Ser Gln Tyr Glu Cys Asp Glu Ile Arg Leu Pro Ile Phe
                   165                 170                 175
   aca ccg tcc cag agg gca aga agg ctg ctt gat ctt gcc acg gtg aaa      576
   Thr Pro Ser Gln Arg Ala Arg Arg Leu Leu Asp Leu Ala Thr Val Lys
               180                 185                 190
   gcg aat gga ttg gct gcc ttc ctt cta caa cat gtt cag gaa tta cca      624
   Ala Asn Gly Leu Ala Ala Phe Leu Leu Gln His Val Gln Glu Leu Pro
           195                 200                 205
   gtc cca ttg gcc ctg cct ttg gaa gct gcc aca tgc aag aag tat atg      672
   Val Pro Leu Ala Leu Pro Leu Glu Ala Ala Thr Cys Lys Lys Tyr Met
       210                 215                 220
   gcc aag ctg agg acc acg gtg tct gct cag tct cgc ttc ctc agt acc      720
   Ala Lys Leu Arg Thr Thr Val Ser Ala Gln Ser Arg Phe Leu Ser Thr
   225                 230                 235                 240
   tat gat gga gca gag acg ctc tgc ctg gag gac ata tac aca gag aat      768
   Tyr Asp Gly Ala Glu Thr Leu Cys Leu Glu Asp Ile Tyr Thr Glu Asn
                   245                 250                 255
   gtc ctg gag gtc tgg gca gat gtg ggc atg gct gga tcc ccg cag aag      816
   Val Leu Glu Val Trp Ala Asp Val Gly Met Ala Gly Ser Pro Gln Lys
               260                 265                 270
   agc cca gcc acc ctg ggc ctg gag gag ctc ttc agc acc cct ggc cac      864
   Ser Pro Ala Thr Leu Gly Leu Glu Glu Leu Phe Ser Thr Pro Gly His
           275                 280                 285
   ctc aat gac gat gcg gac act gtg ctg gtg gtg ggt gag gcg ggc agt      912
   Leu Asn Asp Asp Ala Asp Thr Val Leu Val Val Gly Glu Ala Gly Ser
       290                 295                 300
   ggc aag agc acg ctc ctg cag cgg ctg cac ttg ctg tgg gct gca ggg      960
   Gly Lys Ser Thr Leu Leu Gln Arg Leu His Leu Leu Trp Ala Ala Gly
   305                 310                 315                 320
   caa gac ttc cag gaa ttt ctc ttt gtc ttc cca ttc agc tgc cgg cag     1008
   Gln Asp Phe Gln Glu Phe Leu Phe Val Phe Pro Phe Ser Cys Arg Gln
                   325                 330                 335
   ctg cag tgc atg gcc aaa cca ctc tct gtg cgg act cta ctc ttt gag     1056
   Leu Gln Cys Met Ala Lys Pro Leu Ser Val Arg Thr Leu Leu Phe Glu
               340                 345                 350
   cac tgc tgt tgg cct gat gtt ggt caa gaa gac atc ttc cag tta ctc     1104
   His Cys Cys Trp Pro Asp Val Gly Gln Glu Asp Ile Phe Gln Leu Leu
           355                 360                 365
   ctt gac cac cct gac cgt gtc ctg tta acc ttt gat ggc ttt gac gag     1152
   Leu Asp His Pro Asp Arg Val Leu Leu Thr Phe Asp Gly Phe Asp Glu
       370                 375                 380
   ttc aag ttc agg ttc acg gat cgt gaa cgc cac tgc tcc ccg acc gac     1200
   Phe Lys Phe Arg Phe Thr Asp Arg Glu Arg His Cys Ser Pro Thr Asp
   385                 390                 395                 400
   ccc acc tct gtc cag acc ctg ctc ttc aac ctt ctg cag ggc aac ctg     1248
   Pro Thr Ser Val Gln Thr Leu Leu Phe Asn Leu Leu Gln Gly Asn Leu
                   405                 410                 415
   ctg aag aat gcc cgc aag gtg gtg acc agc cgt ccg gcc gct gtg tcg     1296
   Leu Lys Asn Ala Arg Lys Val Val Thr Ser Arg Pro Ala Ala Val Ser
               420                 425                 430
   gcg ttc ctc agg aag tac atc cgc acc gag ttc aac ctc aag ggc ttc     1344
   Ala Phe Leu Arg Lys Tyr Ile Arg Thr Glu Phe Asn Leu Lys Gly Phe
           435                 440                 445
   tct gaa cag ggc atc gag ctg tac ctg agg aag cgt cat cat gag ccc     1392
   Ser Glu Gln Gly Ile Glu Leu Tyr Leu Arg Lys Arg His His Glu Pro
       450                 455                 460
   ggg gtg gcg gac cgc ctc atc cgc ctg ctc caa gag acc tca gcc ctg     1440
   Gly Val Ala Asp Arg Leu Ile Arg Leu Leu Gln Glu Thr Ser Ala Leu
   465                 470                 475                 480
   cac ggt ttg tgc cac ctg cct gtc ttc tca tgg atg gtg tcc aaa tgc     1488
   His Gly Leu Cys His Leu Pro Val Phe Ser Trp Met Val Ser Lys Cys
                   485                 490                 495
   cac cag gaa ctg ttg ctg cag gag ggg ggg tcc cca aag acc act aca     1536
   His Gln Glu Leu Leu Leu Gln Glu Gly Gly Ser Pro Lys Thr Thr Thr
               500                 505                 510
   gat atg tac ctg ctg att ctg cag cat ttt ctg ctg cat gcc acc ccc     1584
   Asp Met Tyr Leu Leu Ile Leu Gln His Phe Leu Leu His Ala Thr Pro
           515                 520                 525
   cca gac tca gct tcc caa ggt ctg gga ccc agt ctt ctt cgg ggc cgc     1632
   Pro Asp Ser Ala Ser Gln Gly Leu Gly Pro Ser Leu Leu Arg Gly Arg
       530                 535                 540
   ctc ccc acc ctc ctg cac ctg ggc aga ctg gct ctg tgg ggc ctg ggc     1680
   Leu Pro Thr Leu Leu His Leu Gly Arg Leu Ala Leu Trp Gly Leu Gly
   545                 550                 555                 560
   atg tgc tgc tac gtg ttc tca gcc cag cag ctc cag gca gca cag gtc     1728
   Met Cys Cys Tyr Val Phe Ser Ala Gln Gln Leu Gln Ala Ala Gln Val
                   565                 570                 575
   agc cct gat gac att tct ctt ggc ttc ctg gtg cgt gcc aaa ggt gtc     1776
   Ser Pro Asp Asp Ile Ser Leu Gly Phe Leu Val Arg Ala Lys Gly Val
               580                 585                 590
   gtg cca ggg agt acg gcg ccc ctg gaa ttc ctt cac atc act ttc cag     1824
   Val Pro Gly Ser Thr Ala Pro Leu Glu Phe Leu His Ile Thr Phe Gln
           595                 600                 605
   tgc ttc ttt gcc gcg ttc tac ctg gca ctc agt gct gat gtg cca cca     1872
   Cys Phe Phe Ala Ala Phe Tyr Leu Ala Leu Ser Ala Asp Val Pro Pro
       610                 615                 620
   gct ttg ctc aga cac ctc ttc aat tgt ggc agg cca ggc aac tca cca     1920
   Ala Leu Leu Arg His Leu Phe Asn Cys Gly Arg Pro Gly Asn Ser Pro
   625                 630                 635                 640
   atg gcc agg ctc ctg ccc acg atg tgc atc cag gcc tcg gag gga aag    1968
   Met Ala Arg Leu Leu Pro Thr Met Cys Ile Gln Ala Ser Glu Gly Lys
                   645                 650                 655
   gac agc agc gtg gca gct ttg ctg cag aag gcc gag ccg cac aac ctt    2016
   Asp Ser Ser Val Ala Ala Leu Leu Gln Lys Ala Glu Pro His Asn Leu
               660                 665                 670
   cag atc aca gca gcc ttc ctg gca ggg ctg ttg tcc cgg gag cac tgg    2064
   Gln Ile Thr Ala Ala Phe Leu Ala Gly Leu Leu Ser Arg Glu His Trp
           675                 680                 685
   ggc ctg ctg gct gag tgc cag aca tct gag aag gcc ctg ctc cgg cgc    2112
   Gly Leu Leu Ala Glu Cys Gln Thr Ser Glu Lys Ala Leu Leu Arg Arg
       690                 695                 700
   cag gcc tgt gcc cgc tgg tgt ctg gcc cgc agc ctc cgc aag cac ttc    2160
   Gln Ala Cys Ala Arg Trp Cys Leu Ala Arg Ser Leu Arg Lys His Phe
   705                 710                 715                 720
   cac tcc atc ccg cca gct gca ccg ggt gag gcc aag agc gtg cat gcc    2208
   His Ser Ile Pro Pro Ala Ala Pro Gly Glu Ala Lys Ser Val His Ala
                   725                 730                 735
   atg ccc ggg ttc atc tgg ctc atc cgg agc ctg tac gag atg cag gag    2256
   Met Pro Gly Phe Ile Trp Leu Ile Arg Ser Leu Tyr Glu Met Gln Glu
               740                 745                 750
   gag cgg ctg gct cgg aag gct gca cgt ggc ctg aat gtt ggg cac ctc    2304
   Glu Arg Leu Ala Arg Lys Ala Ala Arg Gly Leu Asn Val Gly His Leu
           755                 760                 765
   aag ttg aca ttt tgc agt gtg ggc ccc act gag tgt gct gcc ctg gcc    2352
   Lys Leu Thr Phe Cys Ser Val Gly Pro Thr Glu Cys Ala Ala Leu Ala
       770                 775                 780
   ttt gtg ctg cag cac ctt cgg cgg ccc gtg gcc ctg cag ctg gac tac    2400
   Phe Val Leu Gln His Leu Arg Arg Pro Val Ala Leu Gln Leu Asp Tyr
   785                 790                 795                 800
   aac tct gtg ggt gac att ggc gtg gag cag ctg ctg cct tgc ctt ggt    2448
   Asn Ser Val Gly Asp Ile Gly Val Glu Gln Leu Leu Pro Cys Leu Gly
                   805                 810                 815
   gtc tgc aag gct ctg tat ttg cgc gat aac aat atc tca gac cga ggc    2496
   Val Cys Lys Ala Leu Tyr Leu Arg Asp Asn Asn Ile Ser Asp Arg Gly
               820                 825                 830
   atc tgc aag ctc att gaa tgt gct ctt cac tgc gag caa ttg cag aag    2544
   Ile Cys Lys Leu Ile Glu Cys Ala Leu His Cys Glu Gln Leu Gln Lys
           835                 840                 845
   tta gct cta ttc aac aac aaa ttg act gac ggc tgt gca cac tcc atg    2592
   Leu Ala Leu Phe Asn Asn Lys Leu Thr Asp Gly Cys Ala His Ser Met
       850                 855                 860
   gct aag ctc ctt gca tgc agg cag aac ttc ttg gca ttg agg ctg ggg    2640
   Ala Lys Leu Leu Ala Cys Arg Gln Asn Phe Leu Ala Leu Arg Leu Gly
   865                 870                 875                 880
   aat aac tac atc act gcc gcg gga gcc caa gtg ctg gcc gag ggc ctc    2688
   Asn Asn Tyr Ile Thr Ala Ala Gly Ala Gln Val Leu Ala Glu Gly Leu
                   885                 890                 895
   cga ggc aac acc tcc ttg cag ttc ctg gga ttc tgg ggc aac aga gtg    2736
   Arg Gly Asn Thr Ser Leu Gln Phe Leu Gly Phe Trp Gly Asn Arg Val
               900                 905                 910
   ggt gac gag ggg gcc cag gcc ctg gct gaa gcc ttg ggt gat cac cag    2784
   Gly Asp Glu Gly Ala Gln Ala Leu Ala Glu Ala Leu Gly Asp His Gln
           915                 920                 925
   agc ttg agg tgg ctc agc ctg gtg ggg aac aac att ggc agt gtg ggt    2832
   Ser Leu Arg Trp Leu Ser Leu Val Gly Asn Asn Ile Gly Ser Val Gly
       930                 935                 940
   gcc caa gcc ttg gca ctg atg ctg gca aag aac gtc atg cta gaa gaa    2880
   Ala Gln Ala Leu Ala Leu Met Leu Ala Lys Asn Val Met Leu Glu Glu
   945                 950                 955                 960
   ctc tgc ctg gag gag aac cat ctc cag gat gaa ggt gta tgt tct ctc    2928
   Leu Cys Leu Glu Glu Asn His Leu Gln Asp Glu Gly Val Cys Ser Leu
                   965                 970                 975
   gca gaa gga ctg aag aaa aat tca agt ttg aaa atc ctg aag ttg tcc    2976
   Ala Glu Gly Leu Lys Lys Asn Ser Ser Leu Lys Ile Leu Lys Leu Ser
               980                 985                 990
   aat aac tgc atc acc tac cta ggg gca gaa gcc ctc ctg cag gcc ctt    3024
   Asn Asn Cys Ile Thr Tyr Leu Gly Ala Glu Ala Leu Leu Gln Ala Leu
           995                 1000                1005
   gaa agg aat gac acc atc ctg gaa gtc tgg ctc cga ggg aac act ttc    3072
   Glu Arg Asn Asp Thr Ile Leu Glu Val Trp Leu Arg Gly Asn Thr Phe
       1010                1015                1020
   tct cta gag gag gtt gac aag ctc ggc tgc agg gac acc aga ctc ttg    3120
   Ser Leu Glu Glu Val Asp Lys Leu Gly Cys Arg Asp Thr Arg Leu Leu
   1025                1030                1035                1040
   ctt tgaagtctcc gggaggatgt tcgtctcagt ttgtttgtga caggctgtga         3173
   Leu
   gtttgggccc cagaggctgg gtgacatgtg ttggcagcct cttcaaaatg agccctgtcc 3233
   tgcctaaggc tgaacttgtt ttctgggaac accataggtc acctttattc tggcagagga 3293
   gggagcatca gtgccctcca ggatagactt ttcccaagcc tacttttgcc attgacttct 3353
   tcccaagatt caatcccagg atgtacaagg acagcccccc tccatagtat gggactggcc 3413
   tctgctgatc ctcccaggct tccgtgtggg tcagtggggc ccatggatgt gcttgttaac 3473
   tgagtgcctt ttggtggaga ggcccggccc acataattca ggaagcagct ttccccatgt 3533
   ctcgactcat ccatccaggc cattccccgt ctctggttcc tcccctcctc ctggactcct 3593
   gcacacgctc cttcctctga ggctgaaatt cagaatatta gtgacctcag ctttgatatt 3653
   tcacttacag cacccccaac cctggcaccc agggtgggaa gggctacacc ttagcctgcc 3713
   ctcctttccg gtgtttaaga catttttgga aggggacacg tgacagccgt ttgttcccca 3773
   agacattcta ggtttgcaag aaaaatatga ccacactcca gctgggatca catgtggact 3833
   tttatttcca gtgaaatcag ttactcttca gttaagcctt tggaaacagc tcgactttaa 3893
   aaagctccaa atgcagcttt aaaaaattaa tctgggccag aatttcaaac ggcctcacta 3953
   ggcttctggt tgatgcctgt gaactgaact ctgacaacag acttctgaaa tagacccaca 4013
   agaggcagtt ccatttcatt tgtgccagaa tgctttagga tgtacagtta tggattgaaa 4073
   gtttacagga aaaaaaatta ggccgttcct tcaaagcaaa tgtcttcctg gattattcaa 4133
   aatgatgtat gttgaagcct ttgtaaattg tcagatgctg tgcaaatgtt attattttaa 4193
   acattatgat gtgtgaaaac tggttaatat ttataggtca ctttgtttta ctgtcttaag 4253
   tttatactct tatagacaac atggccgtga actttatgct gtaaataatc agaggggaat 4313
   aaactgttg 4322
<210> 2
   <211> 1041
   <212> PRT
   <213> Homo sapiens
<400> 2
   Met Glu Lys Arg Arg Gly Leu Thr Ile Glu Cys Trp Gly Pro Gln Ser
    1               5                  10                  15
   Pro Ser Leu Thr Leu Phe Ser Ser Pro Gly Cys Glu Met Cys Ser Gln
               20                  25                  30
   Glu Ala Phe Gln Ala Gln Arg Ser Gln Leu Val Glu Leu Leu Val Ser
           35                  40                  45
   Gly Ser Leu Glu Gly Phe Glu Ser Val Leu Asp Trp Leu Leu Ser Trp
       50                  55                  60
   Glu Val Leu Ser Trp Glu Asp Tyr Glu Gly Phe His Leu Leu Gly Gln
   65                  70                  75                  80
   Pro Leu Ser His Leu Ala Arg Arg Leu Leu Asp Thr Val Trp Asn Lys
                   85                  90                  95
   Gly Thr Trp Ala Cys Gln Lys Leu Ile Ala Ala Ala Gln Glu Ala Gln
               100                 105                 110
   Ala Asp Ser Gln Ser Pro Lys Leu His Gly Cys Trp Asp Pro His Ser
           115                 120                 125
   Leu His Pro Ala Arg Asp Leu Gln Ser His Arg Pro Ala Ile Val Arg
       130                 135                 140
   Arg Leu His Ser His Val Glu Asn Met Leu Asp Leu Ala Trp Glu Arg
   145                 150                 155                 160
   Gly Phe Val Ser Gln Tyr Glu Cys Asp Glu Ile Arg Leu Pro Ile Phe
                   165                 170                 175
   Thr Pro Ser Gln Arg Ala Arg Arg Leu Leu Asp Leu Ala Thr Val Lys
   180 185 190
   Ala Asn Gly Leu Ala Ala Phe Leu Leu Gln His Val Gln Glu Leu Pro
   195 200 205
   Val Pro Leu Ala Leu Pro Leu Glu Ala Ala Thr Cys Lys Lys Tyr Met
   210 215 220
   Ala Lys Leu Arg Thr Thr Val Ser Ala Gln Ser Arg Phe Leu Ser Thr
   225 230 235 240
   Tyr Asp Gly Ala Glu Thr Leu Cys Leu Glu Asp Ile Tyr Thr Glu Asn
   245 250 255
   Val Leu Glu Val Trp Ala Asp Val Gly Met Ala Gly Ser Pro Gln Lys
   260 265 270
   Ser Pro Ala Thr Leu Gly Leu Glu Glu Leu Phe Ser Thr Pro Gly His
   275 280 285
   Leu Asn Asp Asp Ala Asp Thr Val Leu Val Val Gly Glu Ala Gly Ser
   290 295 300
   Gly Lys Ser Thr Leu Leu Gln Arg Leu His Leu Leu Trp Ala Ala Gly
   305 310 315 320
   Gln Asp Phe Gln Glu Phe Leu Phe Val Phe Pro Phe Ser Cys Arg Gln
   325 330 335
   Leu Gln Cys Met Ala Lys Pro Leu Ser Val Arg Thr Leu Leu Phe Glu
   340 345 350
   His Cys Cys Trp Pro Asp Val Gly Gln Glu Asp Ile Phe Gln Leu Leu
   355 360 365
   Leu Asp His Pro Asp Arg Val Leu Leu Thr Phe Asp Gly Phe Asp Glu
   370 375 380
   Phe Lys Phe Arg Phe Thr Asp Arg Glu Arg His Cys Ser Pro Thr Asp
   385 390 395 400
   Pro Thr Ser Val Gln Thr Leu Leu Phe Asn Leu Leu Gln Gly Asn Leu
   405 410 415
   Leu Lys Asn Ala Arg Lys Val Val Thr Ser Arg Pro Ala Ala Val Ser
   420 425 430
   Ala Phe Leu Arg Lys Tyr Ile Arg Thr Glu Phe Asn Leu Lys Gly Phe
   435 440 445
   Ser Glu Gln Gly Ile Glu Leu Tyr Leu Arg Lys Arg His His Glu Pro
   450 455 460
   Gly Val Ala Asp Arg Leu Ile Arg Leu Leu Gln Glu Thr Ser Ala Leu
   465 470 475 480
   His Gly Leu Cys His Leu Pro Val Phe Ser Trp Met Val Ser Lys Cys
   485 490 495
   His Gln Glu Leu Leu Leu Gln Glu Gly Gly Ser Pro Lys Thr Thr Thr
               500                 505                 510
   Asp Met Tyr Leu Leu Ile Leu Gln His Phe Leu Leu His Ala Thr Pro
           515                 520                 525
   Pro Asp Ser Ala Ser Gln Gly Leu Gly Pro Ser Leu Leu Arg Gly Arg
       530                 535                 540
   Leu Pro Thr Leu Leu His Leu Gly Arg Leu Ala Leu Trp Gly Leu Gly
   545                 550                 555                 560
   Met Cys Cys Tyr Val Phe Ser Ala Gln Gln Leu Gln Ala Ala Gln Val
                   565                 570                 575
   Ser Pro Asp Asp Ile Ser Leu Gly Phe Leu Val Arg Ala Lys Gly Val
               580                 585                 590
   Val Pro Gly Ser Thr Ala Pro Leu Glu Phe Leu His Ile Thr Phe Gln
           595                 600                 605
   Cys Phe Phe Ala Ala Phe Tyr Leu Ala Leu Ser Ala Asp Val Pro Pro
       610                 615                 620
   Ala Leu Leu Arg His Leu Phe Asn Cys Gly Arg Pro Gly Asn Ser Pro
   625                 630                 635                 640
   Met Ala Arg Leu Leu Pro Thr Met Cys Ile Gln Ala Ser Glu Gly Lys
                   645                 650                 655
   Asp Ser Ser Val Ala Ala Leu Leu Gln Lys Ala Glu Pro His Asn Leu
               660                 665                 670
   Gln Ile Thr Ala Ala Phe Leu Ala Gly Leu Leu Ser Arg Glu His Trp
           675                 680                 685
   Gly Leu Leu Ala Glu Cys Gln Thr Ser Glu Lys Ala Leu Leu Arg Arg
       690                 695                 700
   Gln Ala Cys Ala Arg Trp Cys Leu Ala Arg Ser Leu Arg Lys His Phe
   705                 710                 715                 720
   His Ser Ile Pro Pro Ala Ala Pro Gly Glu Ala Lys Ser Val His Ala
                   725                 730                 735
   Met Pro Gly Phe Ile Trp Leu Ile Arg Ser Leu Tyr Glu Met Gln Glu
               740                 745                 750
   Glu Arg Leu Ala Arg Lys Ala Ala Arg Gly Leu Asn Val Gly His Leu
           755                 760                 765
   Lys Leu Thr Phe Cys Ser Val Gly Pro Thr Glu Cys Ala Ala Leu Ala
       770                 775                 780
   Phe Val Leu Gln His Leu Arg Arg Pro Val Ala Leu Gln Leu Asp Tyr
   785                 790                 795                 800
   Asn Ser Val Gly Asp Ile Gly Val Glu Gln Leu Leu Pro Cys Leu Gly
                   805                 810                 815
   Val Cys Lys Ala Leu Tyr Leu Arg Asp Asn Asn Ile Ser Asp Arg Gly
               820                 825                 830
   Ile Cys Lys Leu Ile Glu Cys Ala Leu His Cys Glu Gln Leu Gln Lys
   835 840 845
   Leu Ala Leu Phe Asn Asn Lys Leu Thr Asp Gly Cys Ala His Ser Met
   850 855 860
   Ala Lys Leu Leu Ala Cys Arg Gln Asn Phe Leu Ala Leu Arg Leu Gly
   865 870 875 880
   Asn Asn Tyr Ile Thr Ala Ala Gly Ala Gln Val Leu Ala Glu Gly Leu
   885 890 895
   Arg Gly Asn Thr Ser Leu Gln Phe Leu Gly Phe Trp Gly Asn Arg Val
   900 905 910
   Gly Asp Glu Gly Ala Gln Ala Leu Ala Glu Ala Leu Gly Asp His Gln
   915 920 925
   Ser Leu Arg Trp Leu Ser Leu Val Gly Asn Asn Ile Gly Ser Val Gly
   930 935 940
   Ala Gln Ala Leu Ala Leu Met Leu Ala Lys Asn Val Met Leu Glu Glu
   945 950 955 960
   Leu Cys Leu Glu Glu Asn His Leu Gln Asp Glu Gly Val Cys Ser Leu
   965 970 975
   Ala Glu Gly Leu Lys Lys Asn Ser Ser Leu Lys Ile Leu Lys Leu Ser
   980 985 990
   Asn Asn Cys Ile Thr Tyr Leu Gly Ala Glu Ala Leu Leu Gln Ala Leu
   995 1000 1005
   Glu Arg Asn Asp Thr Ile Leu Glu Val Trp Leu Arg Gly Asn Thr Phe
   1010 1015 1020
   Ser Leu Glu Glu Val Asp Lys Leu Gly Cys Arg Asp Thr Arg Leu Leu
   025 1030 1035 1040
   Leu
<210> 3
   <211> 37443
   <212> ADN
   <213> Homo sapiens
<220>
   <221> exon
   <222> (63)..(106)
<220>
   <221> exon
   <222> (3908)..(4406)
<220>
   <221> exon
   <222> (12307)..(12412)
<220>
   <221> exon
   <222> (15010)..(16825)
<220>
   <221> exon
   <222> (21017)..(21100)
<220>
   <221> exon
   <222> (21321)..(21404)
<220>
   <221> exon
   <222> (24355)..(24438)
<220>
   <221> exon
   <222> (27052)..(27135)
<220>
   <221> exon
   <222> (27730)..(27813)
<220>
   <221> exon
   <222> (29917)..(30000)
<220>
   <221> exon
   <222> (34244)..(34327)
<220>
   <221> exon
   <222> (36123)..(37443)
<400> 3
   tcaccatata actggtattt aaagccacaa gagcaggtgg gctcatctag ggatggagtg 60
   atatggagaa gagaaggggt ctaaccattg agtgctgggg cccccagtgt taggaaccag 120
   ccaagaagac agaaagagtg aaaatcagag agttggggtg tcctggagga aatgaagaaa 180
   atgccccaaa gaggaaggag ggaacaaata tgaccaatgc ccctggcaga gcaagcaggc 240
   tgagggctga ggattgagca atgggaggtc actggtgaca gtttcactgg agctggatgg 300
   ggaactagag ggaatgggag gggatgggag gacttgggga cagcagtaca ggcaacagac 360
   aagggggcct gctgtaaagg gagcagataa atgggattgg agccaaatga agaaggggag 420
   tgtcaagaga gtgctttact tttacaatgg agaattagag tgcattgtgc actggtgggg 480
   ggatttgatc tcttagggag agaacagtgt tagggaggga gaatgcagga tagctggggg 540
   agggtggggg gcttggcccc agcagagact caggacactt gggaagttga gcttccctgg 600
   gcttcccctc ctctcctgtc tgcaaggggt cagtgggctg agatttcagc acttaagcaa 660
   agcatttgct cttggcccca gagaaaccgg gctggctgtg gtctcaggaa ggaaggaggt 720
   gtccaggctc aggcctgggc ctgggtttca gggagggccc acgtgggtca ccccttgacc 780
   ctctctttca gcaaggaagt gatcctttct ctacatgggc ctcaccttgg ggaggacaat 840
   ggtgtctttg aagttgtagt aactgaagta gagatcaaaa ggcaatgcag atagactgac 900
   agatttcgcc tgaagagggg aagcccgacc aggtaataaa ggagtaagag gaaggatgtt 960
   aaggacaatt ttaggaaaca gataatgagt gaatattttt tctctctctt tcccaattta 1020
   aactgaagca ggagaaactg aagctagaca taatgattaa cttcccaagc tggtgagctt 1080
   cctgagctgg ttagtgagaa cagcactaag gccaggttct cctccccaga tgtttaagat 1140
   gagacaggac aatgcctgct cagagacagg gcctggctga attggccctc aggattctct 1200
   ctgctctgag gtttctggaa gaaggccagg gcagaggtgt ggtgatgtag ctgctgggag 1260
   gacagagctc cgagtcacgt ggcttgggcg ggcctcccct tcctggtgtc cacagaagcc 1320
   caacgtcact agctggggtg tgtatggctc acacgtaggc caggctgccc taggcttggt 1380
   gtgcaaggga ggggccccta cttacttgtg gcctgtcccc tcgtgaatgt gtctcatgtc 1440
   cccagtgggg tttttcagtg agggtcatgg tctccaggat gcacaaggct ttgtgccaga 1500
   attgcttgga attgcctagt tctggaaggc tggttggcca actctggcct ccggcttttc 1560
   ctttgggaat ttcccttgaa ggtggggttg gtagacagat ccaggctcac cagtcctgtg 1620
   ccactgggct tttggcattc tgcacaaggc ctacccgcag atgccatgcc tgctccccca 1680
   gcctaatggg ctttgatggg ggaagagggt ggttcagcct ctcacgatga ggaggaaaga 1740
   gcaagtgtcc tcctcggaca ttctccgggt aagaggagca ggcattgtcc cgtcccagct 1800
   tgatcctcag ccttctttca tccttggccg cgacatgctc ccaggcctgg ggtcagatgg 1860
   ggagtgctga ctctgtttct gggctgtttt ctggggagaa tgggtcggcg ggtttttttc 1920
   cccaggacct gggcagggtc aatggtgggg gccgctgtcg catccttggc tggtgtttcc 1980
   acagctgaga accactccag ggccaagccc agagcttatt ctaccctttt ttgtcctctc 2040
   ttcccctgtc ctcggccacc ccaccctctt ggctcctctg cttagatgtg ggcacaagga 2100
   ggagaactcc ttggcctgag agaactacct tagatcctgg cttccagtgg cctctgcagg 2160
   ggggtacacc ctctctccca agcagccaga cacacaagta acctcattgc ctcagtttcc 2220
   ccatctgacc agcacagggc cccctgtgcc ccagcagcgt tctgagagat tggagctttc 2280
   tccttttgct taccttggct accgtatgag gacggataca gagtgttccc cccaccccca 2340
   gcccagggga tatttgattc atgaacattc cctcagtgtc tttgtggggg acaatgctgt 2400
   gccaggctca gggatgccag gacgagtaag acccaggctc ccacgtggcc caggcaggga 2460
   gagagacaca taaacaacca tcaggaaaga ggtaaaatcc ccaggccact tggcatctgc 2520
   tcccttgagt gtctgggaat gtccctgatt tataaaaaga agctgacggc cctctttgtt 2580
   gtccatgcct acaccctttc actttcgttt cttcggggca ctgcagcagc ccttgtccac 2640
   agaccccatg acaatcgcag aactgaccat gctgagagat tttcttggct gctcagggac 2700
   cctgccaggg cttgaagctc ctggagggtc acttgccctc aaattcccag aacgcacagc 2760
   aggtcactga tgatagcagt ggcagcagtc tgtgcacggt ggtttcgagg gcgtgggagg 2820
   gaggtgaggg ccctagggca agtgtgtgtg ggaagtgttg atgggggaca aggcaccaga 2880
   acgctcggaa acaacttagt ttgcaccgta atttttcact tcgcctagga caggaccttt 2940
   agagcaatat tctgagtcta ccccttggag tagcagtgtg caaaacacac agcacgggct 3000
   tggggccccc gtggggaacc caaatgtaag agttagagac atgcattccg gagtcataca 3060
   tggctcgtgt tgaaatcctg actctgcctg tctagctgtg acacatcgta caaatcactt 3120
   agcttcttgg tgcctcagtg tcttcctctg tagaatgggt agatcatagg cactacttca 3180
   gagtggctgg gagggttcag tgaattcctg caggagagca cttagaatgg cacttggtgt 3240
   gtagtttatg cttaattaat attagccgtt actgaaactg ctgtagcctg aatccagcca 3300
   gcatgaaaga gcccctctca ccctgcttcg aagagaatga attccctgat tgtttggaag 3360
   atctctctct ctctctctgt cttttttttt tttttttgag aaacggtctt gctctcttgc 3420
   ccaggctgga gcgcaatggt gccatcttgg ctcactgcaa cctctgcctc ccgggttcaa 3480
   gtgattctcc tgtctcagcc tcctgagtag ctgggattac aggcgctcgc caccacgcct 3540
   ggctaatttt tgtattttta gtagagacag cgtttcaccg tgttggccgg gctggtctag 3600
   cgctcctgat ctcaagtgac cttgggagat ctcttgctcc taatattacc tcaagccttt 3660
   ttaaacgttt taagccggag accaagcatg gatatgggag ttaggggtct tgatttaatt 3720
   cttggttgct tcaaactctg tggaaccttg aggtgtttct tgccttctct gggtctcaat 3780
   tttcacatct atatggtggg gagcttggat tgggtaatgt ctgaggctag aaccatggcc 3840
   aactcgggtt ctgctggggc tgacttgccc tggccttccc tgaccaccct gcatctggct 3900
   tctggagaag tccctcactg accttgttct cctccccagg ttgtgaaatg tgctcgcagg 3960
   aggcttttca ggcacagagg agccagctgg tcgagctgct ggtctcaggg tccctggaag 4020
   gcttcgagag tgtcctggac tggctgctgt cctgggaggt cctctcctgg gaggactacg 4080
   agggcttcca cctcctgggc cagcctctct cccacttggc caggcgcctt ctggacaccg 4140
   tctggaataa gggtacttgg gcctgtcaga agctcatcgc ggctgcccaa gaagcccagg 4200
   ccgacagcca gtcccccaag ctgcatggct gctgggaccc ccactcgctc cacccagccc 4260
   gagacctgca gagtcaccgg ccagccattg tcaggaggct ccacagccat gtggagaaca 4320
   tgctggacct ggcatgggag cggggtttcg tcagccagta tgaatgtgat gaaatcaggt 4380
   tgccgatctt cacaccgtcc cagagggtga ggcactcctg gtgtgcatca cagagttctc 4440
   aggaaagggg tgcttagtca ccaagactga tttgtcctca tgaagtcagc ctgtggggta 4500
   acttggtccg tgggatttcc cctaaaaagg tagccaggca ggtaaaattt gctcttgact 4560
   cttggcagga aacatacaac tctttctttc ttcttttctt ttctttttct cactctgtta 4620
   ccctggctag aatgcagtgg cacaatcata gctcactgta gccttgaatt cctgcgctca 4680
   agtgatcttc tggccttaga gtagctggga ctacggctgc tgtaccacca tgaacagcta 4740
   attttttttt tttcttttag agatggggtg ttgctatgtt gcccaggctg gtctccagct 4800
   cctggcttta agcaatcctc ccgccttggc ctcccaaact gttgggattg caggcatgag 4860
   ccactttgcc tggccaacag aacacttctg ccgagaggaa gtgtgtggtg gccaggaact 4920
   cagattctgg agccagaatg gtgcaggctc aaggtcaacc ctgtgtgatc tcaggcttcc 4980
   ctatggagcc tctccagcct cagtctccct tgtttcagtt tcctcatcta caaaacaatg 5040
   ttaatagtca aatggtgcct atcctataag gctcttggga ggattcagtg agttaatttg 5100
   agtaatgctt aggatagtgt ctattaccac tggctgctat ttattatttc tgttatgagt 5160
   gatactctgt acttgtacac ttttatttct gtctgtttta aattaacagc acaacagacc 5220
   ataacactgc agtatattga atttatttta taattaacat agcatattat aaactaatat 5280
   agcttaaatg tttatgtagg atttctgaca tgaaattgca ttagatcata gatgttcaga 5340
   gttggtatat aacagcccct gagaatgtag taactcagca gagaccagaa ggtcagagaa 5400
   atgaccactg agtatttttg aaactctttt gttttcttcc aaatagtgat tcttagggct 5460
   cctgagaggc agatggaaca atcattaaca ttccacttta taaatcggga agttgagacc 5520
   aaggaaagta gtttgaataa gctcacagta gttaatgagg gggccagtgc tggaccaatt 5580
   ggccagcact ggtcattgac ttattcatcc atcattcatt tattcagcca gaatctatta 5640
   ggtgcttcat acatatttgc ttaaagtttg ttgtgttcat agagctttgc acacggtagg 5700
   tactccataa acatttgttg atgaaataag tgagttactg aatgaatgat tgaattagaa 5760
   tgacactgca gtgttaaaat gggctgggtt ggggaacatt ttagtttttg tttttgtctg 5820
   ttttccaaaa atgtatgtgt tgttcacatg agtctggata accctagatt gagattgatg 5880
   acataaataa atttgtcttc aaggctgcac taaagctggc tcacatggct aggtatttac 5940
   agagcagaag tggtgcagtc ctctctgatt agttgcacgt acagaagaca tattcgttat 6000
   tggactgacc ttagtttctc ttataatttg ttaggggaat tgaatcagcc catctgagaa 6060
   gttacaagat tgtgtcttgt catctttaaa agttcagcaa tgtgatgtgg tacagatggt 6120
   ctgaggggtt tggagaaggt agcctagatc cctagggccc agagaagaca ggatgtgaac 6180
   agaggaagta catggattgg tgaagaaaag aaatgggata actcatgggt caaagaagaa 6240
   atcatgatgg aaatcagaaa atattcagaa ccatacaata atgagaatat tatttatcaa 6300
   aatctattgg atgcagctaa agcaggacat agggggaaat ttacaacctt aggtgcctag 6360
   attaggaaag aaggaaggca tttgtttatt tatttgttta tttatttatt tgagatgggg 6420
   gtctcactgt gtcacccagg ctgctggagt gcagtagcac gatcataaat cactgaagtc 6480
   tcgaacttct gggctgaagt gatcctcccg cctcagcctt ccaagtaggt gggacacagg 6540
   ctagcaccac cataccaggc taattttttt tttgtagaca cagggtcttg ctatgttgag 6600
   gtctcaaact cctgggctca agtaatcctc ctccctcggc ttcccaaagt gctgggatta 6660
   caggcatgag ccactgcgcc catctaaggc tgaattttaa tgagctaaga attcatctta 6720
   agaaagggct aaatagacag caaaagcaaa cattgaaggt tgggactgag ctgagtgggt 6780
   agcagggatg ggagacaaca gatctgagga gagcaggaga ttttgaaagg attgcactgc 6840
   ctgaggttta agcctttaga atccagctct ctctgagctc cctttgagct ctgacattct 6900
   gtgactctga tttggtggcc ttcccttagt ggccttactg atttcatttg gatggtgctt 6960
   gtggtatatc caaccaacat gtcttcccaa atggcctttt aatttcctat aaagaagtag 7020
   ttgtcattga ttgcaggtta gggacagaaa atgctgtgga atgaaacaaa atgcaagtta 7080
   aagaactaaa ttccaaaaat acccattgct actattgact gagtgaattc ctactgtgtg 7140
   ccagacactg tacccagtcc attccctgta ttgttttatt taagcctcac aagggtatag 7200
   tgtgactaca ctgtttctta acaatgaaga aactgcccaa atcgcccatc tgggaagcgg 7260
   cccagctaga atttgaatcc aggcctgttt tcctccagag cttgtgctat tctctgtctg 7320
   tcataaaatg tgggggcttt gtgtggtaaa cttgctcagt tgggcatagc agttgttagg 7380
   aaacctgagg ctggtaacac cagctgtaat accagctgtc cgtctgactc atgcaactgt 7440
   taaagttgat agggctgagg tgtcagactg agctctgaat tgcctgattc ctataacaat 7500
   attaacttaa acatttttta aattgggaaa tgcaccatgc atacagaaga gtgtgtatat 7560
   ttcatatgta tagtgtaaac tgttcccatc acccaggtta aaaaacagga tgttgccagt 7620
   acctggggcc ttctttaact gcaactgcta gaggtaaaca ctggcttgac ttttgtgtaa 7680
   atcatctctt tgcctttctt taatgtttta gcatctttta aaataaatcc ccaaataatg 7740
   tattgttcta ttttgaaaaa ctgagtagca agccaaaaat agctgtgtaa agaaaggtca 7800
   cttaaattag gctgggtgca gtggctcaag cctttaatcc cagtactttg ggaggctgag 7860
   gcaggtggat cacaaggtca ggagatcgag accatcctgg ccaacatgga gaaaccccgt 7920
   ctctactaaa aatacaaaaa attagccaag aatagtggca tgtgcctgta gtcccagcta 7980
   ctcgggaggc tgaggcagga gaatcgcttg aacccgggag gcagatgttg cagtgagctg 8040
   agatcgcact gcttgaaccc gggaggcaga ggttgcagtg agccaagatc gcaccactgc 8100
   actctagcct gggtcacaga gcaagactct gtctcaaaaa aaaaaaaaaa aaaaagaaag 8160
   gttactattg ccttttctta gatgaaggtt cccaaggcag ggaaagctaa gtggagtctc 8220
   agggacttgg tctggctttt ccttccctgg gaatttataa ggacctcttc tgggaagtca 8280
   gtcggcaatg ccatgaatga gtctggggaa atattgggct cattgcaact ggagggtctg 8340
   gtaggactga tgtgaattag gtgctgtgtc cggaggaaaa tggccagagg aagtgggctg 8400
   ctttgtacag tcagtggtaa agttgccaaa ggctattata gctcacagga atgggccaag 8460
   gctaaacact cctgtggagt gaaatgaatg tcctcagctg actgaggcag cgggagttga 8520
   gaagaaacga tattagttca tggtgaagac aagtcaaata tagataaagg ttagggtcag 8580
   gcttgcctgg acatctagga gataactgcc ctcaacttgt ttgaatcttg agtcactgct 8640
   ccattttgtt tgaactggtg gccatctact tatagtatac agccatcaac ctgagatttc 8700
   cctacatggt cttcctgcct tggtctcctg tatcctgaat cctatggcct cttcttccct 8760
   ggtttactac attttgctag accgtatcct ccagtcaatt ccttagaatg aatgtatgaa 8820
   agttaaaatt tctgaggtct cacatgtctt aaagttccct catactggat tgatagtttg 8880
   gctgggtata aaattctggg ctggccatca ttttccttca gaattttgat tgcattattc 8940
   cattatcctc tcttttcaat attgcttcta agaattccaa aacctttttt tttttttctt 9000
   tttgagacag tgtctcactc tgtcacccag gctggaatgc agtagtgtga tctcagctca 9060
   ctgcaacctc cacctcctgg gtttaagcga ttcttcttcc tcagcctcct gagcagctgg 9120
   gattacaggc acccaccacc acacccttta gtagagatgg ggttttgcta tgttggccag 9180
   gctggtcttg aacttctgac tttaggtgat ctgcctactt cggcctccca aagtgctggg 9240
   attaaaggcg tgagccacca cacccagcct ccaaaaccat tttaaaactc tttctggaag 9300
   cttttaaaat tttcttttag tccccagaat tttaaaattt caattatgtg ccttggtgtt 9360
   cttccattat attagtcacc caagaggtac tttcaatctg gaaacttctc tatgttttgg 9420
   gaaatgttct tgattagttt acaggtgatt tcttcctctc cattttatct cttctctttt 9480
   catgaaacta ctattaattc aatgttagaa ttccttgact gatcatttaa ttttcttcta 9540
   ttttccatct ctgtgtcttt ttgctctact tttctatgat agtcacagct ctatctttaa 9600
   actcttgagt ttttcatttt tgatgtcatg attttaattt gcaagaggta ggtttgactg 9660
   attctttttt gtagtatctt actcttgttt tatggatgca acatcttctt tgacttaagg 9720
   atcataagat aggtgggttc tttgtttgtt tgtttgactg tttttcaccc tatgtaaact 9780
   ttttctacaa gtttctttcc ccttcccccc tttttggctt ctatctccca cattagatgc 9840
   tttctctggg ctcatgatac tctttggttt tctttctcaa gattgacagg taggacttta 9900
   aaacttgttg agcatgcggg tgaaacttgt ctaccatgaa tttcactgta gatattttgg 9960
   agattgacag tgtttatatc tttagatctc acctcctggg ttgatcaagt tatctgagta 10020
   caccacagac cttttgcctg gggataaacc agaaatctgt ttcagaaacc actttgattc 10080
   agtcttcctt gttttagtca tttccttcag ttccggaggt ccgtcatgct gatcattcca 10140
   gagcccttta cagatcctag ggtacacact gcatggtttt caactttctt gttttggggt 10200
   taagatttgg ctttcaggag tctcctcagt ccgttactat tcattcaatc agcaagtcct 10260
   tgagcacctg atttgtgcca gacattcttc taggtgttag ggatacctca gtgaacaaaa 10320
   cagacaaaaa tctttgtctt ggaaatacac acactccagt caggggagag ggacaataag 10380
   ccaaaggaag gaaattacag cgtgtgctag aaggtgataa gtgctgtaga aagtaagtaa 10440
   agtgggtttg ggagttgaga gtttgggaag gggataaatg atggcaattg taaatagagt 10500
   agtcagagtt ctcacttaga aggtgaaatt caagtaaaga cttgaaggag gacagggaat 10560
   tagccacatg gatggctagg ggaaggcttc caagctgaga ggacagccag agccaaggcc 10620
   cagaggcagg agcatacctg gtagttttag gaaacaggag gccaggatgc tgagtggagt 10680
   aagagggggc atgaaaggag aaacttgggt ccacgtggtt ctagacaggt atttttgtct 10740
   gttttgggcc ctgaaggtta ctattggact tggactctta ctctgaggaa atagggacgc 10800
   tattgggacg tttgtacagg agcaatgtga cctgagtttt gtttgtaaag gattagactc 10860
   tggctgtggc attaaggcta ggctgtgggg gcaggaacag aagcaggggg accagttttg 10920
   cagcctgtgc agctttccag ataagcaggg attgtggctt ggaggaggat ggtatagagg 10980
   aggtgacaag aaatgactct atgtctggta tgtagatatt ggccacagat ggcatttgag 11040
   cactagagac ctggctggtc cacatggagt ttccataagc acataataca catcagattt 11100
   caaagactta atatgaaaaa aaaaatttaa cgggccccgg gaattttttt cttttttttt 11160
   ttttttgaga cccagtcttg ctctgtcacc caggctggag tgcagtggtg tgatctcggc 11220
   tcactgcaac ctccgcctcc caggttcaag tgattctcct gcctcagcct cctgagtacc 11280
   tgggactaca ggcacctgcc accacgcctg gctaattttt tgtattttta gtagtgatgg 11340
   ggtttcacca tgttgtccag gctggtctgg aactccggac cttaggggat ctacccgcct 11400
   tggcctccca aattgctggg attacaggca tgagccacca tgctcagcca tatcttgcta 11460
   ttttctacat ggattacatg ttgaaatggt aatgttttgg ctattgtgga ttaaatagaa 11520
   tatatgatta aagttgattt catctatttc ttttaacttt aaaaaatatg tctgttagag 11580
   gatttgaaat tccacatgcg gcttgcattt gtgacctgca tttcatttct gtggaacagt 11640
   gccctttttg ggacatgctt tgaaggtgga gtcaacagga tttggcagat tacagacgag 11700
   aggcttcaag ggtgactcca agacttcggg gcagagcacc tggaagaaag gggttaatat 11760
   tagccaagat gaggaaggct gtcggtttgg caggtgcatg ggcaggttag gagtttagtt 11820
   ttgaatatgt tggaggtgtt tatgaaactt ttaagtggag atggaaaata ggcagttgga 11880
   tgtgcaagtc cagggttcag ggagacagtt caggctggag atgaagatgt gggagtctga 11940
   ggagagattg tattcaaata ttcaatccat gagacttgat gaaatcactt ctcttccaaa 12000
   tgatttacag cctgcagaat cattttccct atctttgtag gtttatgtct tcattttgtt 12060
   tcatttattt ttcagttatt cactgtttta gtgagttttg agtaggagcc agattggatg 12120
   catgcgttca attcaccatc caacactgta ttaactactt gaaactcatg tggttgttcg 12180
   gttgtttttt tgacctttta ttctggatgg aagagagatg cttatgaagt tgcagtaatc 12240
   agtaagcctt cccacattgc tccatcagcc ttcctggaag aataatgtct tctgcctttc 12300
   ctgtaggcaa gaaggctgct tgatcttgcc acggtgaaag cgaatggatt ggctgccttc 12360
   cttctacaac atgttcagga attaccagtc ccattggccc tgcctttgga aggtaggtgt 12420
   atgttctcag ttaatcagaa agggaagggc agtcagtgca gatccatggt taagagcaga 12480
   acacacctcg gttaacatcc catatgctgg cagtatagcc tccctatgac tcaatttcct 12540
   tgttttaagg ctagcaccac cccgtctcat tgggattttg ggagcattaa aaggacaaaa 12600
   gcgtgtaatg ttagctatta gctttcatta tctcccacac agtatactga caattgggct 12660
   accatatatt gagggctaac taaaggtgtt acttaccatc caaactctca ttatctgtac 12720
   cgaaaagata tggacacatg ttttgagtta gggctggtat ctcttgatct ctgaaattta 12780
   gcagctcaca atgggaaact caagaaccaa gtggatctag agactctggt atccctcagt 12840
   gcccagggtc accacccaaa ctcaggaaca ggaggggctt ggaccgcacc acttgaacat 12900
   accaggcatc ctgccaggtg ctttatggac aatgtctacc ctttgcaaca accctgagaa 12960
   gtaggtggtg tttttttcca ccttatagat gtggaaactg ggcagggagg ttaagtgacg 13020
   agggagggga agatgggtct gattgtaaat tgtccccacc tacactttct cttttcttgg 13080
   gagaagaaat gtcagttgta aagagagagt gcaagcctgg cactctttag ggcttgttcc 13140
   tacaccactg tagggaaagc tcattggcac tgaagccccc tgagctgtgt gtggtgctgg 13200
   cagatgggtc tatcaccctg gactgtgtcc tctgggcagc aagcaagcct gtgggcgggg 13260
   tggctggaag tctgtgcctg gcactcgcga gtgcaccgtc tcattgaaga acaggatcta 13320
   aacatcagtg cgccacagca gggtgcgcgg cacggagtgc aggccctggt ttggcccttg 13380
   gttgaggttt gctgttgaca tcatcaagca cagctagtca ctgtaagacc aggccagggt 13440
   gcaagattcc ccacacttct aaaggtgaca attggtgtat ttatttctct ataaaatgac 13500
   attttttttt tctggagaat tttagtatca ttggtgatga ctggaaaacc tgcatcagaa 13560
   atcaggtcgg aagaggaaga tatatatctg atatgtactg gagaggaaga tatctatctt 13620
   atggtctaag ttcagggatc ctggtatatt cagagggcag aaagctcagc aataatcatc 13680
   aactctggga acagaggtga cataaacaca gggcgtcccc tttgtgtgac tgcagatagt 13740
   catcagtgag ctcagagctc tatgaaaatt acttgctagt ttttgggttg aaaatagtgg 13800
   gccagtgttt ggttgggggc agtgaggctg tgatggcggg ggaccatgcc aagctcctac 13860
   cagcctggga cgctaaacca gcacttcccc atttcctgaa aggggaacta aactctgaca 13920
   caggaaatgg tttgcttgca ttactttcag gatgagaaag gaagagcact ggccttccaa 13980
   acacaccccg tgcatgaaaa ctctccctgc atggggtgca tggggaggat ggggaagtgg 14040
   aggcaggatc acagactctt gttcgagtgc tcagctgggg caccccggtg accccgaggc 14100
   cttcccttgc taggtccacc cagatcaatc aggatcatct ccccatctcg aagtttaact 14160
   ttatcacatc tcagagttcc ttttgccacg taaggtaaca tattcacagg ttctgagaat 14220
   ccggacatgg acatctttga gggtctattg ttgtgcctac tatatccatg aataataatg 14280
   ataataagca ccattttttg agagtttgcc atgtcagata ttcttttaaa ctgtatttta 14340
   tctcgctgcc tcctgaaaaa atccttccag gtgtatattg tccccatttt tacagatgag 14400
   agaactgagg cccagaaagg ctaaatggct tgcccaagtg tatggtggac ccaggttttc 14460
   aaactcaggt gtgtctggct tcagagactg ggctcctgag cccttaagcc ctttgttccc 14520
   ctttagaaaa agtcacctga ggctgagtgg tgaagggatt tatccaaagc cacccggcca 14580
   ctatggcagg acagatatca gaatacaggt cttccgatcc cagcccagag ccccttcccg 14640
   tcatctagaa ctcctcctgg tgtcagtaat gataacggca gtcactgatg tcttttgagc 14700
   acttactttg tgttgagcac ttacactgtg ctaagcactt gacataggtc atcttagttg 14760
   atccgtgtaa aactctgtga ggtagtgacc aacatttctc ccaccttaca gaggtggaaa 14820
   ctgagggtta ggaagtttcc ttgactgtcc tcaaagtgca cagcttgtga atggaggagc 14880
   caggatgggc gcccgctggc tctcctatcc cttcagttat gtcagcgtcc cccgcagcag 14940
   cccattgtct ggttaggtcc cgtcttcacc atggtgccac cttcatctgc ctcttcttct 15000
   gccttccagc tgccacatgc aagaagtata tggccaagct gaggaccacg gtgtctgctc 15060
   agtctcgctt cctcagtacc tatgatggag cagagacgct ctgcctggag gacatataca 15120
   cagagaatgt cctggaggtc tgggcagatg tgggcatggc tggatccccg cagaagagcc 15180
   cagccaccct gggcctggag gagctcttca gcacccctgg ccacctcaat gacgatgcgg 15240
   acactgtgct ggtggtgggt gaggcgggca gtggcaagag cacgctcctg cagcggctgc 15300
   acttgctgtg ggctgcaggg caagacttcc aggaatttct ctttgtcttc ccattcagct 15360
   gccggcagct gcagtgcatg gccaaaccac tctctgtgcg gactctactc tttgagcact 15420
   gctgttggcc tgatgttggt caagaagaca tcttccagtt actccttgac caccctgacc 15480
   gtgtcctgtt aacctttgat ggctttgacg agttcaagtt caggttcacg gatcgtgaac 15540
   gccactgctc cccgaccgac cccacctctg tccagaccct gctcttcaac cttctgcagg 15600
   gcaacctgct gaagaatgcc cgcaaggtgg tgaccagccg tccggccgct gtgtcggcgt 15660
   tcctcaggaa gtacatccgc accgagttca acctcaaggg cttctctgaa cagggcatcg 15720
   agctgtacct gaggaagcgt catcatgagc ccggggtggc ggaccgcctc atccgcctgc 15780
   tccaagagac ctcagccctg cacggtttgt gccacctgcc tgtcttctca tggatggtgt 15840
   ccaaatgcca ccaggaactg ttgctgcagg agggggggtc cccaaagacc actacagata 15900
   tgtacctgct gattctgcag cattttctgc tgcatgccac ccccccagac tcagcttccc 15960
   aaggtctggg acccagtctt cttcggggcc gcctccccac cctcctgcac ctgggcagac 16020
   tggctctgtg gggcctgggc atgtgctgct acgtgttctc agcccagcag ctccaggcag 16080
   cacaggtcag ccctgatgac atttctcttg gcttcctggt gcgtgccaaa ggtgtcgtgc 16140
   cagggagtac ggcgcccctg gaattccttc acatcacttt ccagtgcttc tttgccgcgt 16200
   tctacctggc actcagtgct gatgtgccac cagctttgct cagacacctc ttcaattgtg 16260
   gcaggccagg caactcacca atggccaggc tcctgcccac gatgtgcatc caggcctcgg 16320
   agggaaagga cagcagcgtg gcagctttgc tgcagaaggc cgagccgcac aaccttcaga 16380
   tcacagcagc cttcctggca gggctgttgt cccgggagca ctggggcctg ctggctgagt 16440
   gccagacatc tgagaaggcc ctgctctggc gccaggcctg tgcccgctgg tgtctggccc 16500
   gcagcctccg caagcacttc cactccatcc cgccagctgc accgggtgag gccaagagcg 16560
   tgcatgccat gcccgggttc atctggctca tccggagcct gtacgagatg caggaggagc 16620
   ggctggctcg gaaggctgca cgtggcctga atgttgggca cctcaagttg acattttgca 16680
   gtgtgggccc cactgagtgt gctgccctgg cctttgtgct gcagcacctt cggcggcccg 16740
   tggccctgca gctggactac aactctgtgg gtgacattgg cgtggagcag ctgctgcctt 16800
   gccttggtgt ctgcaaggct ctgtagtgag tgttactggg cattgctgtt caggtatggg 16860
   ggagcaccat caaggctaag tgtgggagca ccgagctggg ctctagaagt ctgggcccag 16920
   cttcgcctct gccaccctgc tttgcaacac tgcccagatc ccttcccttc tgggccttaa 16980
   tttcaatatg tgatgatgac agccacactt tattgactgg cctatgtgct gggtctggtg 17040
   ctatgctttc cggaatgacc tcatctaatc tctacaacca ccctgggggg taggcaggaa 17100
   tgttattatc tccattatcc ttgacttgag gctcagagaa gtgaagtaac ttgtccagga 17160
   aatggcagag ctggggttca caaattgcat cattctgatt acaggttttc tgcctcccac 17220
   cagtctatgg atacacttca gaggctccct gaaaaccttg aggtcacttg cagaaagttt 17280
   tgtgtagtat gtgtccgtat caggaacaac accaaatcag aggtgacttg tgccccatca 17340
   gagactttaa caccccaacc agatgggaat ttcaggaccc aagaaataga aagtggctgc 17400
   agggttacaa ctactgttgg attcctgagg tagcacagtg tccaaacagg atttcagcac 17460
   tacccgtatt gcttagagcc ccagccaaag atgtgaggtt ttgccctttg gagaatctgt 17520
   gcccctgaac tcgggggcct ctttccacat cttgggggca ggcaagggca gagggtgtgc 17580
   ctaggcctgc ggatcagcat gcgacagatt ccccaacatc cttccagctt gaaaggggat 17640
   tgccctgctt ctatttagaa cctataggaa agcagaagtt ctagattgaa gttaaaattg 17700
   attcccagcc tccaggggct ttgggctaca cctggatgac cttaattgac cctaagcatg 17760
   ggacaaacca cttcctgaga gtattaggat ggtatacatc ttctctgggg gcaaagcaac 17820
   aagatttatt tttcatcatg gaccaaacac atggataccc actagaaact gtgtagtgaa 17880
   ttttgttaac cctgacatag ggaccatggt ctttaggtta aagcataata acaacataat 17940
   acataacata tatagcgaat atatatatgt attatatgca atgaatgtaa atatgattat 18000
   acccatcatg gtcttggagg aaacagatga cacacttaaa atgggtgttt tgaggagagt 18060
   ttgaaaaaca gattgtttac aagccatggg caggagttag gaagagtgag agggttggtg 18120
   caggggcctg gggttagtaa cagctggggg agggtagact tgaaggggga aggggaggga 18180
   gactaattag ctggggggaa ggtatggaga cggctgcctg agcttctgca aagtggaaga 18240
   atactgcttg gccctaactc ctcaccccaa ctcttgctcg tggccagcgc cttccaccag 18300
   ctggacccat cagggaggcc gagtgggctg tctgctggag tagtccccag gcatcagcct 18360
   cccaggagcc agggacgggt agagaagggg gagagtggat ctggccaggc aaatggaaaa 18420
   cagccagcac caaactctat ttccctagga gggaggatca tgatactttg agtgggaatt 18480
   tggaaacctg tctgttggag caatttccct gatagaaata agaatgtgca ttttcctggg 18540
   tagtagactc agtttttacc ccaagaggcc aggcatcact ggcctgtgtg atcctcatag 18600
   gccagtccat ctctggaatt cttgaatgga tcatccatcc ttgattaggg atgtccccgt 18660
   gattaccagg gtgtgcagaa gggctctggg aaacctgtgg gtctgtctct gtgttcagag 18720
   aaaggtgagg gtggcctggt tctagctcat ggtgctcaga ctgtggtgtg taaaggcact 18780
   cgtggcaatg cagattcctg ggcctgcctc tagtgattcc cattcagtag gtttggggtg 18840
   gggcccagga aatctatatt tttcacagac acccctggtg attctgatac aagtggtctc 18900
   gccctgggag aactactggt ctgcagcaac cagcttggtt ttccattagc aattactgtc 18960
   cttgagcgag ttttactgct cttcacctta cacacactaa aactgccaag gccgtagggg 19020
   aggggaagca accatgaggt tgctgtgagt gcactgtgtg tgtgtgtgtg tgtgtgtgtg 19080
   tgtgtgtgtg tgtatgagag agagagagag attgagaaag agaggaaggg aggaaggggg 19140
   agggcacagg ctcctctccc acagtgccaa cctgcctctc tcccacttga agcgtttcca 19200
   tgccaactga aatcctcagc ctctaggaaa ccctatatac acagtgcccc tatataggtt 19260
   tctttagact ctggctctct cagactctag agtgatggct ttaaaagttt tatgttaccc 19320
   acagagagag agcacgcacc accatgtaaa catggaacct aagtttcaca aaatgacttc 19380
   gctttatgaa ctctgagaca ctctgctctc ttctgttctg ttctatttcc attttagaaa 19440
   tgctgctcag gaccttcaaa atgatttgca tgacctgcaa cctgcagtct gaaaaatcac 19500
   tgcactacag aagtggccat aagaggccct gagggagaag ctgcacaatg tcatggttaa 19560
   gagtggggtt tggagccaag ccgcctaggc tcaaagcctt tatgtgccgt acaaccttgg 19620
   caaagtcact tcgcttgtct gtgcctcagt ttctttctca cgaatgctca taataatggt 19680
   tcccatttca ctggcttgtt gtgaggatga aatagtgtta ttattgagaa gtggtaaggg 19740
   tagtgatcag tgctagcgat catgattcta ggtgactttt actgtgtacc gggtgctcac 19800
   aaggctttat gtgcacagcc tggtgaggct gataatacta ttgttccctc tttttttttt 19860
   ttggaaacgg agtctcgttc tgttgcccag gctgggggta cagtggcaca atctcggctc 19920
   atgcaatctc tgcctcccgg gttcacgcca ttctcctgcc tcagcctccc aagtagctgg 19980
   gactacaggc gcctgccacc acgcccggct aatttttttg tatttttggt agcgacaggg 20040
   tttcactgtg ttaaccagga tggtctcgat ctcctgacct cgtgatccgc ccgcctcggc 20100
   ctcccaaagt gctgggatta caggcgtgag ccaccgtgcc cggcctgttc cctcttttat 20160
   agatgaagag accagcaaat aactagtaag tcgctgatca ggatcacaat atccagctga 20220
   ggcactccag agcctgagct gttaaccatt cagtcagggc ctcccaagtt tgcctaaaga 20280
   taaagaatca tgtgcacagt tgttaaaata tacagattcc tgggccccac cccgcagata 20340
   cttgattgcc agctccaggg tatgggcctg agaatctgtc ttttagggaa gctttcagat 20400
   gatgttgtga tcaggtgagt tttgggaatg gtgccccaag aggagtggca gacagggctt 20460
   gctcggcagg gactagcctg ttggagtggt gccattgggg ttaaggactg ggcagcaggg 20520
   cctcactaac cacagcctat atgcctgttt ctgaagtttt ggccactctc atccagctgg 20580
   tctactgtct gctgacctag atgatggtaa attgtcccca ggggtagcct gtctagttca 20640
   ggctgcacct ttcgcatata tcagctcctt tccaccatca tcccctttgt gaggctgctg 20700
   tgattatcat gttccttttg cagagatgga aacattgcct caaattagct ctgtcatttc 20760
   ctaaggattc cagggttctt tagtaggggg tctggatcct acgtcctggg ccatccccat 20820
   catagtgcac cacgtcacct ccctggccag ggaccgtggg gtctccactt ttttggggtg 20880
   ctccatctat gcagggtttc ctggaagcac agatgctggc acttcaggga tgaatgaaag 20940
   tctttttggg ggatttgtag atttttttct tgtcttacta gctccatttt caaatgtatt 21000
   tattttgtct ctttagtttg cgcgataaca atatctcaga ccgaggcatc tgcaagctca 21060
   ttgaatgtgc tcttcactgc gagcaattgc agaagttagc gtaagtcagc ctgggctgtg 21120
   gacaatgggc tccaagtgcc ctggtctcac cccaggtcgt gcagcctggg aagctgtgag 21180
   tgatgggctg gggcaggggc tgtttgcatg atggggggtg caggtgattc ctgcccagag 21240
   gggaagggca accctgggat ttggtgctca ctgtccaatg tgctttgctt ctgtgtctcc 21300
   tctcttctgg aactgaacag tctattcaac aacaaattga ctgacggctg tgcacactcc 21360
   atggctaagc tccttgcatg caggcagaac ttcttggcat tgaggtgagc ccaggttttc 21420
   cttattccct ggaaactatt ttttgcccca ttcctgagtc agtctgatct ggtcttggcc 21480
   tggcactgcc cacactggct cctgacctcc tgattgaatg cagggacagt gtctcatttt 21540
   aagcaggggt tctctaatgc tgtgatctcc ccagtaaact ctggactagc tctgctgagg 21600
   acttcctgtc ttttgacctt tagcccgtag ggcaagaaag cttttctagg cccctttcct 21660
   tttctgtgtc taagagtgtc acagctttct ggggttactg agttccacga tgcatgttga 21720
   gctcgtcctg gtgggggagg catacacagt tacttgccac cccagctgtg gcagcgagtt 21780
   gctgcaacac tcccaggagg tcctttcacc actcagagca tgcaaggttt gcagtccatc 21840
   tggttctgca tttctgctac tccagtgtct cccagtttca acaggagtct ctctctctcc 21900
   tacctgatgc ctttaaattg cccctctagc tggccgctgg gttggcctgg cttctctctc 21960
   cttctctctc tctcagatat tcttgcctcc tgtgatttgt gaggcagtaa aaaaagacaa 22020
   agtaaagaat tgcttccatc tattctttta cctcttgggc tgggtttgtg gatgggagcc 22080
   gccattttaa aatggcgggc cacatagctc agtctcggca agggctactg agatcagaac 22140
   cacaggtgcc aatttgtaca aaggactcag tcctgctacc actgcctgat ccctcagact 22200
   cacaagcctg gaataggctg tggccagacc tggctggccc atccctgaga agggtgctag 22260
   tttcagaaat ggaggctgag tttgtggcca acacagtagt cctccggtat gtgcaggaga 22320
   gatgttctaa gaccccagtg gatgcctgaa accatggaga gtatcaagcc ctacacatac 22380
   catgcttttc ccaataccta cacacctgca ataaagtgta gtttataaat taggctcagt 22440
   aagagagtaa tagcaactca taataaaata gaacaattat aacaatcaat atactataat 22500
   aacactatgt gaatgtggac tctctccatc tccctcaaaa tatcttcttg tactgtactc 22560
   acccttcttc ttgggaagat gtgtggtggt aaaatgcctg tgtgatggga ggaagtgagg 22620
   tggatgacgc atgcagcact gtgctctagc gctgggctgc tgttgacctg accacacttc 22680
   agaaggagaa tcatctgctc ccagagatcc ctaatctttg agcaacaatg aggtcggcag 22740
   ctggatgtca ggagcagacg atcttgatga ttaccaaatg ggagcgtata gagcgtggat 22800
   gcgctggacg gggggctgat tcacgtcctg ggtgggatgg agctggatgg cacgtgatca 22860
   gaatagcatg caatttaaaa tgtatgaatt gtttatctct agaattttcc atttaatatt 22920
   tttggactgc agttgatttc agataactga aaccatagaa ggcgaagctg cggataagca 22980
   gggggcaggg attaccgtat atcattgtaa tagagagcac aggctctgga gccagactgc 23040
   ccgaggtttg aaccctcatt agctgcgtga cctcaggtca gcccaatgtc tgtgtgcctc 23100
   cgtttcccct tctgtagaat ggaggtaata accctggcta cctcacaggc tgtagtgatg 23160
   agcaagcaag ttaatccaca tgaagggctg caccgtctgg caggggcttt atatagtaag 23220
   cgagtggctg aaagatgatg ggtaaatcac acaagcactc agcttgtttc tccttatgtg 23280
   agtccggtcc tccaagcagg gattcaatgt gccacccatt tattggggaa aagtcctaaa 23340
   aggggaagtg gggaagggag ctgggggagg ctgggaggtg tgtccctgag tgaaggagag 23400
   agggaaggaa ggaaggttga gactgggcac cttggacttc agtgcagtcc taagacatct 23460
   tggcaaggct gatgaggagt tcttgaacca aattcaccag gcaggggagc ctgatgtctc 23520
   aggcaggggc tggcaagtgc agatgcgagg atgttagatt ttggagcaca gcagctgggg 23580
   cccttggcta cctccaagga gctgaggctg gagacctgaa aggcgagttc tcctagctgc 23640
   cacacccctt ctccaaggat acaataatat ctgccttata ggattgttgt gagctgagtg 23700
   gcttgacgtt ccttgaaaga atgaaagcgt atagttatcc caggaagcct agggttgcag 23760
   gtgagagctc tggggcttct ccgaagctct ccgaggtgtc tggattcagt tgcagcagga 23820
   gccttccttg ctgggatctt cccccacccc tagccttggc cctccctctc tccttccttt 23880
   ctggaaggct cagtgggccc cacccctccc tccagccacc tggacctgcc cagcgctctt 23940
   gtgcaacagg taaagcctac ctgtagcaac aacagatctg ggaaggctgc agagggcacg 24000
   atggggtctg gatcgagggc ggctgagacc agagggaaag gtgtgaccct gagtcaccct 24060
   cgctgtcccg gggaaaccac ctcccaggac agctgcctac tgtggctcct gcctggaatt 24120
   gtcacactgc tgtgcaaaca gcgtcccgct gcccctttcc ctttgctggg ggaaaatgaa 24180
   gttgtgggag ccgctgagta aactagacct agcagcgagg gcacctgatg tggctgctgc 24240
   ctcccgggca ggtcttcaat gctttcttcc tgtgtttccc tggccagggc acagacggcc 24300
   ctccttttct gcctgccgct gtgttctctc agcctcctct gtcttccctt ccaggctggg 24360
   gaataactac atcactgccg cgggagccca agtgctggcc gaggggctcc gaggcaacac 24420
   ctccttgcag ttcctggggt aggttggatt ccaggaagag ggacctgcat ggaggggctt 24480
   gggacttttg aggatttagg ggcaggtgaa actcttcagc caggaggccc cagaggcagc 24540
   ccagctccag tggggaggac aagccaggga gagagtgggc ggcccttgac tgccaccttc 24600
   atacttggtc tatgcctgac aaacaggaag tttgggatgt tggggctagg ggaggacagt 24660
   gcccacgagc tggtgacagg aagccctctg atcctcaggg ggcgctaggg ctgtacttta 24720
   gctgcatatt aaaaccacct ggaagcttct aaacactatt gccaggcctc ccaccccaga 24780
   ctgatgaaat gcaaatatct aggtgcaagg cccaggtatc aggagtttta aaaagcttcc 24840
   caggggatgt acagccaggg gtgaggaccc ctgacctaag aaagagaagg aaatggggaa 24900
   ggataggaag gcacccagga taagaggggc tgtgctaggt ccctcggagc tcttgctccc 24960
   tgtaggacca tgctagggcc tgccagggag gggagtaccc caacctgcag ccccagggtg 25020
   ggcttcctct gtttgctagg cacccaggct tgcacctgtg ctgtttccag cagcctctct 25080
   cctatcctgt catgccctag tgtgaactgg agtccatttg acaagaactg ggagttttag 25140
   aacctgggac tgtaggaaga gagaataacc ttagggccta ggtgttccag cccatttcac 25200
   agggaggcaa gttgccccca agctcagttt tttgttttgt tttgttttgt ttgagatgta 25260
   gtctcactct gttgcccagg ctagagtgca gtggcacgat cttggctcac tgcaacctcc 25320
   gcctccttgg ttcaagcgat tcacctgcct cagcttctca agtagctggg attataggca 25380
   cccaccacca cgcccagcta atttttgtat ttttagtaga gacagggttt caccatgttg 25440
   gcccggctgg tcttgaactc ctgatctcag atgatccgcc cgcctcggcc tcccaaagtg 25500
   ctgggattac aggtgtgagc caccgcaccc ggcccccaag ctcagtttga gccacaaatg 25560
   ggactatgtt gctctagaaa tcaacatctt ttccacactg cattagtagc aacagagtct 25620
   agaacaaagg aggccacagc cccactgaac tctcttctgc ttgaggtcac atctgccaca 25680
   tcaggggtat ttacctcttt caacacatat ttattagggc acctgtctgg gccaggcgtt 25740
   gtgctaaaac ccccaaacgc tgtcatatga tacaaagtgt tctgtaactt gcttggtttt 25800
   tttttttgtt tgtttgtttg ttttgttttg tttttgttgt tgtttttttt tgcttcgcca 25860
   tatattatag gaattttttt aggtcattat gacctcttta tttacttaat tatctattta 25920
   tttattttac taatatttac agaaagggtc tcactctgtc acccaggctg gagtgcagtg 25980
   gttgcaatca tagctcattg tagccttgaa ctcctgagct caagtgatct tcctacctcg 26040
   gcctcctgag tagctgggac tacaggcaca agccaccatg cctggccgat atttttatgt 26100
   tttgtagaga cggggtctca ctatgttgcc caggctggtc tcaaactcct gggctcaggt 26160
   gatcctccct cctttgcctc ccaaagtatt gggattacac aagtgagcca ccttgctcag 26220
   cctgacctca tttttcaaag agctgcagag tgttacataa tgtatttaac tggtcacttt 26280
   ttgatgacta ttaagttgtt ttcaggtttt ttgttattac agtgtcatat ccctggggca 26340
   cagagcagtg ctggcacata gccagagctc aatcgataca tacctaatga atgaaagtac 26400
   agtggacatc ctaattcagc cattctttgc taacttgtgt acatacctgt ccagggtagg 26460
   tccctagaat acagtcaata agtcagaagg tgtgagttgg gatctacctt ttggaaaggg 26520
   atgttttcaa actacagtga gtcagaggag gatggcccag aagctggggg agttgaagct 26580
   gatggcgtga aggaattagg ggtgttagga agaagcagga gataaagagc tagcttgcag 26640
   aagaagtgtt agacttgtta tgggcaggta ctggagggta gctaaggact tgtgggtggc 26700
   agttaccagg aagcgtatct gaactaagtg tcagaaaaag tgtcacaact gtaaattact 26760
   cttgtcagtg agttcctgtc cttaagggtt agggctgggt agccctctac tattctctaa 26820
   gtctgtaatg taaagccact gaaaactctt gggttaagtt tggccatccc acccaaaaga 26880
   tggaggcagg tccactttgc tgggaccagg agccccagtg aggccactct gggattgagt 26940
   ggtcctgccc ctctggctgg gactgcagag ggaggaggac tgttagttca tgtctagaac 27000
   acatatcagg tactcactga cactgtctgt tgactctttt ggccttttca gattctgggg 27060
   caacagagtg ggtgacgagg gggcccaggc cctggctgaa gccttgggtg atcaccagag 27120
   cttgaggtgg ctcaggtaag cttcagagtc tatcctgcag ttttcttggg gagatcaggt 27180
   gaagagggag gagctggggc cagttctgaa ggtctttgaa ctttatttct accccacaat 27240
   gttaggcaat ggagtaagga aaaaagacca ttggatttca agagaggaca cttgagtctt 27300
   tctgggtgac ttggaaatgt cccttgtcct ctcagggttt tgatacagta tctgtaaatt 27360
   gaagatattg ggctggatca ggtacatttt atcttaaggg ccaattccaa tccattggta 27420
   gtgggtgccc agtgcaccac attaaaaaga attctaaggc tgcacctggg cttaaagaag 27480
   agcactataa tcaattagtg atgtctaaaa aagctaaaaa aaaaaaaaaa gagcactgca 27540
   ttcaattagt gatgtctaaa aagggtagaa aaaaaaaaaa aaagaaaaaa gaaagagcac 27600
   cgcaatcaat tagtgatgtc tgaaatggag cagaccagga gagcaccacg aattttgccc 27660
   tccataggtt agctcatctc tgaggtcttt ccctgctctg acatactttt gttccatgat 27720
   tacctccagc ctggtgggga acaacattgg cagtgtgggt gcccaagcct tggcactgat 27780
   gctggcaaag aacgtcatgc tagaagaact ctggtgagtt tgggggattc tctgctctgg 27840
   ggaagtggat cacaatctct gttgatcccc tggcctcatc cataggagcg gttgtgtgga 27900
   cagacaaagg tggatgattg agtgattgac tgattgattg attgtgtttg tctttatatg 27960
   tactgagtgg tatgaagctt atagagcctg gtatgtacat gctaattttt ttatttaata 28020
   aaatatatgg gtttgctggt ttggtgactg cctccacatg gcataagtgt taagagcaca 28080
   gactctgtaa tcaagcaggc cgtgatctta ggcaagttaa ataacaattt cagaatctca 28140
   agtttcatgt ctgtaaaatg agggtaagaa tacttccaac cataaaggat ttttgcaaga 28200
   attagataaa gtagtgcctg tgaagacctt aatatagtgc ctggcatatt tgtaagtgct 28260
   ccataaatgt taaattagaa taatggcagg gttactacta ctattactgc tgctgctgct 28320
   gctgctgctg ctacaactac tatagtactg tgactactac tactaataaa gttttgttat 28380
   tttaaagtga ttttgagttc ctaggagcac tgggtattca agtcttaggt cattttggaa 28440
   ggtgtaatgg agttttgata gttgaaagag gaaccatgaa tcatgcttat actgttgacc 28500
   tgaagcagat tctaagtttc tcatccttta gatgccacta gtatagtttt ctgacatgtt 28560
   ctgggcagct tcagattatg tcagggagat aaaatactga atgtttgatt ttcccgggaa 28620
   gcagaaaggc actgcaacat atgggcattg ccataaacag attttatgga tggaccttgg 28680
   ctgttgcagg gcttactagc tctactcaag tatgattgat tctatcctga ctggattttg 28740
   ccacttggaa tttcttagta gaggagaacc ttgttatgag agcatcagtt atgattactg 28800
   ttaaaagaaa aactttaggc aaattaaatt tagcagaact ggtttgaaca tacagcaatt 28860
   tatgaattgg gcagcattca gaactgggag tgctccaccc agcaaggtag gcaagcagta 28920
   tctatagaca ggaaaaggaa gtgatgtaca aaacagcttg attggttgca gctgggcatt 28980
   tgccttatat gggcatggtg tgatgaggca ttttctttat atggatatag actgatcagc 29040
   tggtagactg tgactgactg aagcctggct gctgtgattg gctaagactt agctgtttgt 29100
   tataaggata tgttgttagg ttgcagtttg ctacatagga actcaaagta cagaggcagt 29160
   ctcaggccaa atttagttta actatatgtt aagctgcagg tgacagaata cctccatcta 29220
   tagaggttta aacaaggaaa gggtttattt tttcctgtat aggcagctgg atgtaggcag 29280
   tgtagggttt gtacagtggc tacaagaggc caggaggggt ctcagctctg tctcattctc 29340
   ttcctgttcc atcatcctta gcctgtaact tcattcacat ggttggttgt ctcatgatca 29400
   caggatggct gctccaggtg cagcactact tctgtattcc cggattcgat ctatataccc 29460
   aggaaagcca tctgggttct ctcctttaaa aagcattcct ggaagcccca cctgtcgact 29520
   tccccttatg tatcaaccat gtgtatgtca cttgaccaac ccacttgtat gttgtttgac 29580
   cagccctggc tgcaatggag agtgggaaat acagtttttt caccaagtgc atggctgtcc 29640
   aaatgaaatg agacttccat taataaggaa gaaaggaaag atggagatca ggaagctggg 29700
   ggatcaggga acttattaca ttgagagccc ttggagtgaa ttctcttgca aatatgtccc 29760
   tggaattgag aatccccaca acgtctttat ctgttctttc tttatccatg agtttgggtt 29820
   ttcagatgtt ggatttccta tatggggggc atgtgagttc atcatcttcc ataatcaatg 29880
   ttgtatcaac tggattttct ctcttcttct caccagcctg gaggagaacc atctccagga 29940
   tgaaggtgta tgttctctcg cagaaggact gaagaaaaat tcaagtttga aaatcctgaa 30000
   gtaaggaacc cataagcagg aaacaggaca ataattgctg gcctttggaa ggggcatttc 30060
   tgattaagat ctgggccgct ctccgctggg ctaactcatg tgaggtggcc tggtagaaca 30120
   gcttgccttg gtctaggtgg acaaggattc cagtgcaagt tgtttatctg ggaggtggtc 30180
   ccagtaaatg ctgataggag agtggtgaag tgagatgggg aagtgaaggt aaccaataaa 30240
   ggggagttat caagccagtt atcaatgagg gaaattggag ctcagtactc tggggcactc 30300
   ctggagccag tgcagaacac acatggtcac ctacccaacc aatgggcaag aaagccatgg 30360
   catttatcca ccaaccctct gtccttccta tgttgatgtg cgctcatggg gcactgattc 30420
   tccagcactt ccagctcacc ctcacccagc tgaacatgct tctggggtca ggagaatggc 30480
   ctcaggcaga gagtggcagg tcttctctgc aagcagtggc tggggaggtg atgtgatggg 30540
   gagtactgtg gcctcctcca gtggctgact cagtggcttg ggacttgtgc cacaaagaga 30600
   tggacagctc aggtgaacat gaacccacct agtgaccatc atgggtttgt cagggtgctc 30660
   tctgaggctg atgccaaaat tcttatttca agtagacctc aggaacccca tcagatggct 30720
   ccttttgctg gaggaaagtg gcatctgcct aggcaaatgt ggtcctagga aaacgcttgc 30780
   ctttagagac agacagacag acagctgcct ctgtgagtgc cagctttgct gccaggctgc 30840
   tacccactct ggcgacactc atttgtgttg ctttcacaag ctaggaagtt tccaaatatt 30900
   tggagaaaac acttccacta attatttggg tggaaatggg ctgggaagtt ggggtgaagc 30960
   ccggatgtgt ctgagccaga tgccagcttt gcactgaggg tcggcctttg ggaataccaa 31020
   gcccattatc aaccaggtgt ggatatggca ggtttgtctt ccctccttgt cacagcctta 31080
   ctccacttga ctcccatgga tgccaggcaa tgaggctggg gttggtccca tgccaccctg 31140
   tcatcagcct tatttttcag catcctaaac tatatcatcc cccacaaaaa ttgaacttct 31200
   gatatatctt ttataaaaaa gagaaatgcc tacatctttc ttttccagga ttagtttctg 31260
   ccaagagttg gttgagagcc caggcttgct gggtgcagtg gctcacacct gtaatcccag 31320
   cactttggga ggctgaggcg ggtggatcac ctgaggtggg gagttccata ccagcctgac 31380
   caacatggag aaaccccatc tctactaaaa atacaaaatt agccgggcgt ggtggcatac 31440
   acctgtaatc ccatctactc aggaagctga ggcaggagaa tcacttgaac ctgggaggtg 31500
   gaggttgcca tgagccaaga tcacaccatt gcaccctaga ctggacaaga gagaaacttc 31560
   catctcaaaa aaaaaaaaaa ggatgagaaa aataataatt taaaaaaaag agtccaggct 31620
   ctggaaccag acagcctggg tcttacccct gctccaccat taccagccag ttcttcttgg 31680
   atgagtgcct cagttgcctc aagtgtaaat ggagataatg gctggacctt cattataggc 31740
   catgagcatt cactgagaga atgtagctaa caaaagtgag ttgtaggttg gagcaaaagt 31800
   aattgtggtt tcagaccatg aactttaaat tattataact aggctaaaat acatctttat 31860
   taatcaaaat aggaaccatt aaaatcaaca catttttgcc aataagaaat aagtttgttt 31920
   attcctgtag cataaaaatt catgcttcgg gattcaacaa actcttggaa agcattttct 31980
   gcatcctcct ggttgtggaa gcatttttcc tgcagaaagt tgtcaagatt cttgaagaaa 32040
   tggtagtcag ttggctagag gtcaggtaaa tatggcggat gaggcaaaac ttcatagtcc 32100
   aattcattca acttttgaag ctttggttgt gtgacatgca gtccggttgt tgtcgcggag 32160
   aattggaccc tttctgttga cgaatgccgg ttgcaggtgt tgcagttttc agtgcatctc 32220
   attgacttgc cgagcatact tctcatatgt aatggtttcg cagggattca gaaagctgta 32280
   ggggatcaga ctagcagcag accaccagtg accatgacct tttttttttg gtgcgaattt 32340
   gcctttggga agtgctttgg agcttcttct cggtccaacc actgagctag tcattgccag 32400
   ttgtataaaa tccacttttc atcgcacgtc acaatcagat caagaaatgg ttcgctgttg 32460
   ttgtgtagaa taagagaaga tgacacttca aaatgacgat tttcttggtt ttcactcagc 32520
   tcatgaggca cacacttatc gaggtttttc acctttccaa tttgcttcaa atgctgaatg 32580
   accatggaat ggtcgatgtt gagttctcaa gtagttgtaa gaaaatcagc tttgatgatt 32640
   gctctcaatt ggtcattgtc agcttctgat ggcctgccag tacactcctc atcttcaagg 32700
   ctcttatctc cttcgcaaaa cttcttgaac caccactgca ctatacgtta gttagcagtt 32760
   cctgggccaa atgcattgct gatgttgtga gttgtctccg ctgctttaca acccattttg 32820
   aattcaaata agaaaattgc ttgaatttgc tttttgtcta acatcatttt catagtctaa 32880
   aataaatata aaataaacag aaagtattaa gtcattagca aaaaatcata aagtgagaat 32940
   tgtgcattaa aatgatgtat agcataacca catttattta agaatgtatt ccaatatcaa 33000
   atggcaaatt tcaacaatgc aaaaactgca attacttttg caccaatcta atagaagttc 33060
   aataaatact ggcaattaca attggcattg ccttagggtc aacttgtaag acattcctga 33120
   aattgtggga aagggggagg acctggagtg gacattattg gaaggcaaag ctgtaaccaa 33180
   aagagcaacc tgggaaacac atgactcctc tgttgctgtc cctggcccta tcctgtctcc 33240
   cctccctgtt gtcagctacc tcatatgttc tctaatctct gtctctgtgc cctcaaagac 33300
   ccccctgaaa atagaaatat tactgctcat tggttatttt ctatcaatta agtactgtat 33360
   tagtccgttt tcatgctgat gataaagata tacccaagac tgggcacttt atgaaagaaa 33420
   gagttttatt gaacttacag ttccacgtgg ctggggaggt ctcacaatca tggctgaagg 33480
   tgaaaggcac atctcacatg gcagcagaca ggagaagagg gcttgttcag ggaaactccc 33540
   ctttttaaaa ccatcagata tcatgaaact tatttactgt aatgagaaca ggatgggatt 33600
   caattacctt ccactgggtc gctcccacaa cacgtgggaa ttcaagagat ttgggtgggg 33660
   acacagccaa accatatcaa gtactgtgca agtgttttag gcatgcagag agtggtgggt 33720
   cttcccagca agcagagtgt ggggaggtaa tgggggactg gtggctgact taatggccca 33780
   ggacccatgc cacaaggaga tggatggtgg atgtgaatag gagcctgctt acacccatca 33840
   caatttagat tcttatgctc gatggcacgg gtactctttt aggcccattt taccaatgag 33900
   gagattggga ctaatttgct cgagatcaaa aaagaagtgg tgtaggtggg atttaaaccc 33960
   aggatgtcta gcactaaaat gcaggtactt aaccactatc ctaagggagt ggctacttaa 34020
   tttgataaac tcatctagtg aatggaagag agacggttac atttcactga tggtactgag 34080
   cctttgttga tgagctcatt gggaatctca gacatgagca ggatgtgtct aagggacagg 34140
   tgggcttcag tagactggct aactcctgca gtctctttaa ctggacagtt tcaagaggaa 34200
   aaccaagaat ccttgaagct caccattgta tcttcttttc caggttgtcc aataactgca 34260
   tcacctacct aggggcagaa gccctcctgc aggcccttga aaggaatgac accatcctgg 34320
   aagtctggta aggcccctgg gcaggcctgt tttagctctc cgaacctcag tttttctatc 34380
   tgtaaaatgg ggtgacggga gagaggaatg gcagaatttt gaggatccct tctgattctg 34440
   acattcagtg agaatgattc tgcatgtgaa ggatctgatt ctctgtctaa gaaagaagtc 34500
   tttacctctt taagtaggga gcaatgattt catttttaaa ccttgactat ttattcagca 34560
   acttctctgc tctatgagat agtgtaggaa tggggatgtg gttgaagaat gaaaagaaaa 34620
   gtcagctccc gccctcctag aaattgcatc tgccttcaca ggtcaaggat attggatcag 34680
   accttctgcg gttctgaatg gagattacac aggttaggag caggttgcac agtgtttcca 34740
   attctctata attaaagcca tagactttca tgtattgaaa aaagcaagaa ttgcattctt 34800
   gacagattct ttcattgcct taaaaagaat gactagcctt gggagtctgg gcagctgggt 34860
   ccagtgttgt agactttctc tctgctgagc cacagcttca aagatttgtc cttcttgttt 34920
   ccagggatct atttctcaga caataagtaa aggctttccc tggcctaatg tgctgtaagt 34980
   gaatgctact atatatgttc caggcactgg gctagagact aatatttaaa agccaggaaa 35040
   tttcctatag aaaatctata tctcagggtt ttctcaaaag agctgggaac tctggatgcc 35100
   cattcatgat tccagtagtt aaccagagta caagaagggc tgagtcttct cagatgggca 35160
   aacccactct ggctgactgc agatccacca agcctattgt cttagaccag gaccctttgg 35220
   caactcattc ccataagcct gtgacccttg ctttaaatat gcaggccttg tcttctctca 35280
   aaaagcacat caaggctgca gcgaatgcag atatcaaatg atgaagttaa aaacaaaagc 35340
   tttgctgggc gtggcagctc acacctgtaa tcctagcact ttgggaggct gaggcaggag 35400
   gatcacttta ggccagaggt tcaacaccag accttgtctc tcaaaaaata aaaaattcag 35460
   ctgggtgcgg tgtagttcct agccacttgg gaggctggga tggaaggatc ccttgaaccc 35520
   aggagttcaa ggctgcagtg ggccatgatt gcatcactgc acaggcgaca gaattagatc 35580
   ccatctctta aaaaaataaa aaatttaaaa gtgacttcaa aaatctatgc tgtgatggag 35640
   agatttttcc ttctgtatga ttgtgatagc tctgtggcct atgacgtcat caggttctgg 35700
   gcaaagtgta ggttttctgt ttctttgttt ttgaaaccat tgcacagtcc taagaaacat 35760
   cacattctgg gtcctgggca ccagccaaca tgaggtgagg gcaccagggt ttgctcattg 35820
   cattcttgac agattctctt attgccttaa aaagaatcac tggccttggg gagtctgtgg 35880
   ctggctgggt gcagtgttgt ggactctctc tgcagagtca tggagccttg ttcagaatgc 35940
   ttcctgagct gccctggttg gccaagggta aaaacagccc tgacttccct gcaagaaaca 36000
   ctgcagctgg gccagagagt cagcccatcc caggcatggg tttaaaaagt ggaggctttt 36060
   gtttgaaagc cctgctctaa ttttgtcctc actcaaacct ctgttcactt gatctgcttt 36120
   aggctccgag ggaacacttt ctctctagag gaggttgaca agctcggctg cagggacacc 36180
   agactcttgc tttgaagtct ccgggaggat gttcgtctca gtttgtttgt gagcaggctg 36240
   tgagtttggg ccccagaggc tgggtgacat gtgttggcag cctcttcaaa atgagccctg 36300
   tcctgcctaa ggctgaactt gttttctggg aacaccatag gtcaccttta ttctggcaga 36360
   ggagggagca tcagtgccct ccaggataga cttttcccaa gcctactttt gccattgact 36420
   tcttcccaag attcaatccc aggatgtaca aggacagccc ctcctccata gtatgggact 36480
   ggcctctgct gatcctccca ggcttccgtg tgggtcagtg gggcccatgg atgtgcttgt 36540
   taactgagtg ccttttggtg gagaggcccg gcctctcaca aaagacccct taccactgct 36600
   ctgatgaaga ggagtacaca gaacacataa ttcaggaagc agctttcccc atgtctcgac 36660
   tcatccatcc aggccattcc ccgtctctgg ttcctcccct cctcctggac tcctgcacac 36720
   gctccttcct ctgaggctga aattcagaat attagtgacc tcagctttga tatttcactt 36780
   acagcacccc caaccctggc acccagggtg ggaagggcta caccttagcc tgccctcctt 36840
   tccggtgttt aagacatttt tggaagggga cacgtgacag ccgtttgttc cccaagacat 36900
   tctaggtttg caagaaaaat atgaccacac tccagctggg atcacatgtg gacttttatt 36960
   tccagtgaaa tcagttactc ttcagttaag cctttggaaa cagctcgact ttaaaaagct 37020
   ccaaatgcag ctttaaaaaa ttaatctggg ccagaatttc aaacggcctc actaggcttc 37080
   tggttgatgc ctgtgaactg aactctgaca acagacttct gaaatagacc cacaagaggc 37140
   agttccattt catttgtgcc agaatgcttt aggatgtaca gttatggatt gaaagtttac 37200
   aggaaaaaaa attaggccgt tccttcaaag caaatgtctt cctggattat tcaaaatgat 37260
   gtatgttgaa gcctttgtaa attgtcagat gctgtgcaaa tgttattatt ttaaacatta 37320
   tgatgtgtga aaactggtta atatttatag gtcactttgt tttactgtct taagtttata 37380
   ctcttataga caacatggcc gtgaacttta tgctgtaaat aatcagaggg gaataaactg 37440
   ttg                                                               37443
<210> 4
   <211> 1315
   <212> ADN
   <213> Homo sapiens
<220>
   <221> CDS
   <222> (117)..(1118)
<400> 4
   cgatcagaag caggtcacac agcctgtttc ctgttttcaa acggggaact tagaaagtgg 60
   cagcccctcg gcttgtcgcc ggagctgaga accaagagct cgaaggggcc atatga cac 119
                                                                 His
                                                                  1
   tcc tcc cgg acc cct gga cac aca cag ccc tgg aga ctg gag cct tgg   167
   Ser Ser Arg Thr Pro Gly His Thr Gln Pro Trp Arg Leu Glu Pro Trp
               5                   10                  15
   agc atg gca agt cca gag cac cct ggg agc cct ggc tgc atg gga ccc   215
   Ser Met Ala Ser Pro Glu His Pro Gly Ser Pro Gly Cys Met Gly Pro
           20                  25                  30
   ata acc cag tgc acg gca agg acc cag cag gaa gca cca gcc act ggc   263
   Ile Thr Gln Cys Thr Ala Arg Thr Gln Gln Glu Ala Pro Ala Thr Gly
       35                  40                  45
   ccc gac ctc ccg cac cca gga cct gac ggg cac tta gac aca cac agt   311
   Pro Asp Leu Pro His Pro Gly Pro Asp Gly His Leu Asp Thr His Ser
    50                 55                  60                  65
   ggc ctg agc tcc aac tcc agc atg acc acg cgg gag ctt cag cag tac   359
   Gly Leu Ser Ser Asn Ser Ser Met Thr Thr Arg Glu Leu Gln Gln Tyr
                   70                  75                  80
   tgg cag aac cag aaa tgc cgc tgg aag cac gtc aaa ctg ctc ttt gag   407
   Trp Gln Asn Gln Lys Cys Arg Trp Lys His Val Lys Leu Leu Phe Glu
               85                  90                  95
   att gct tca gct cgc atc gag gag aga aaa gtc tct aag ttt gtg gtg   455
   Ile Ala Ser Ala Arg Ile Glu Glu Arg Lys Val Ser Lys Phe Val Val
           100                 105                 110
   tac caa atc atc gtc atc cag act ggg agc ttt gac aac aac aag gcc   503
   Tyr Gln Ile Ile Val Ile Gln Thr Gly Ser Phe Asp Asn Asn Lys Ala
       115                 120                 125
   gtc ctg gaa cgg cgc tat tcc gac ttc gcg aag ctc cag aaa gcg ctg   551
   Val Leu Glu Arg Arg Tyr Ser Asp Phe Ala Lys Leu Gln Lys Ala Leu
   130                 135                 140                 145
   ctg aag acg ttc agg gag gag atc gaa gac gtg gag ttt ccc agg aag   599
   Leu Lys Thr Phe Arg Glu Glu Ile Glu Asp Val Glu Phe Pro Arg Lys
                   150                 155                 160
   cac ctg act ggg aac ttc gct gag gag atg atc tgt gag cgt cgg cgc   647
   His Leu Thr Gly Asn Phe Ala Glu Glu Met Ile Cys Glu Arg Arg Arg
               165                 170                 175
   gcc ctg cag gag tac ctg ggc ctg ctc tac gcc atc cgc tgc gtg cgc   695
   Ala Leu Gln Glu Tyr Leu Gly Leu Leu Tyr Ala Ile Arg Cys Val Arg
           180                 185                 190
   cgc tcc cgg gag ttc ctg gac ttc ctc acg cgg ccg gag ctg cgc gag   743
   Arg Ser Arg Glu Phe Leu Asp Phe Leu Thr Arg Pro Glu Leu Arg Glu
       195                 200                 205
   gct ttc ggc tgc ctg cgg gcc ggc cag tac ccg cgc gcc ctg gag ctg    791
   Ala Phe Gly Cys Leu Arg Ala Gly Gln Tyr Pro Arg Ala Leu Glu Leu
   210                 215                 220                 225
   ctg ctg cgc gtg ctg ccg ctg cag gag aag ctc acc gcc cac tgc cct    839
   Leu Leu Arg Val Leu Pro Leu Gln Glu Lys Leu Thr Ala His Cys Pro
                   230                 235                 240
   gcg gcc gcc gtc ccg gcc ctg tgc gcc gtg ctg ctg tgc cac cgc gac    887
   Ala Ala Ala Val Pro Ala Leu Cys Ala Val Leu Leu Cys His Arg Asp
               245                 250                 255
   ctc gac cgc ccc gcc gag gcc ttc gcg gcc gga gag agg gcc ctg cag    935
   Leu Asp Arg Pro Ala Glu Ala Phe Ala Ala Gly Glu Arg Ala Leu Gln
           260                 265                 270
   cgc ctg cag gcc cgg gag ggc cat cgc tac tat gcg cct ctg ctg gac    983
   Arg Leu Gln Ala Arg Glu Gly His Arg Tyr Tyr Ala Pro Leu Leu Asp
       275                 280                 285
   gcc atg gtc cgc ctg gcc tac gcg ctg ggc aag gac ttc gtg act ctg   1031
   Ala Met Val Arg Leu Ala Tyr Ala Leu Gly Lys Asp Phe Val Thr Leu
   290                 295                 300                 305
   cag gag agg ctg gag gag agc cag ctc cgg agg ccc acg ccc cga ggc   1079
   Gln Glu Arg Leu Glu Glu Ser Gln Leu Arg Arg Pro Thr Pro Arg Gly
                   310                 315                 320
   atc acc ctg aag gag ctc act gtg cga gaa tac ctg cac tgagccggcc    1128
   Ile Thr Leu Lys Glu Leu Thr Val Arg Glu Tyr Leu His
               325                 330
   tgggaccccg cagggacgct ggagatttgg ggtcaccatg gctcacagtg ggctgtttgg 1188
   ggttcttttt ttttattttt ccttttcttt tttgttattt gagacagtct tgctctgtca 1248
   cccagactga agtgcagtgg ctcaattatg tctcactgca gcctcaaact cctgggcaca 1308
   agcaatc                                                           1315
<210> 5
   <211> 334
   <212> PRT
   <213> Homo sapiens
<400> 5
   His Ser Ser Arg Thr Pro Gly His Thr Gln Pro Trp Arg Leu Glu Pro
   1 5 10 15
   Trp Ser Met Ala Ser Pro Glu His Pro Gly Ser Pro Gly Cys Met Gly
   20 25 30
   Pro Ile Thr Gln Cys Thr Ala Arg Thr Gln Gln Glu Ala Pro Ala Thr
   35 40 45
   Gly Pro Asp Leu Pro His Pro Gly Pro Asp Gly His Leu Asp Thr His
   50 55 60
   Ser Gly Leu Ser Ser Asn Ser Ser Met Thr Thr Arg Glu Leu Gln Gln
   65 70 75 80
   Tyr Trp Gln Asn Gln Lys Cys Arg Trp Lys His Val Lys Leu Leu Phe
   85 90 95
   Glu Ile Ala Ser Ala Arg Ile Glu Glu Arg Lys Val Ser Lys Phe Val
   100 105 110
   Val Tyr Gln Ile Ile Val Ile Gln Thr Gly Ser Phe Asp Asn Asn Lys
   115 120 125
   Ala Val Leu Glu Arg Arg Tyr Ser Asp Phe Ala Lys Leu Gln Lys Ala
   130 135 140
   Leu Leu Lys Thr Phe Arg Glu Glu Ile Glu Asp Val Glu Phe Pro Arg
   145 150 155 160
   Lys His Leu Thr Gly Asn Phe Ala Glu Glu Met Ile Cys Glu Arg Arg
   165 170 175
   Arg Ala Leu Gln Glu Tyr Leu Gly Leu Leu Tyr Ala Ile Arg Cys Val
   180 185 190
   Arg Arg Ser Arg Glu Phe Leu Asp Phe Leu Thr Arg Pro Glu Leu Arg
   195 200 205
   Glu Ala Phe Gly Cys Leu Arg Ala Gly Gln Tyr Pro Arg Ala Leu Glu
   210 215 220
   Leu Leu Leu Arg Val Leu Pro Leu Gln Glu Lys Leu Thr Ala His Cys
   225 230 235 240
   Pro Ala Ala Ala Val Pro Ala Leu Cys Ala Val Leu Leu Cys His Arg
   245 250 255
   Asp Leu Asp Arg Pro Ala Glu Ala Phe Ala Ala Gly Glu Arg Ala Leu
   260 265 270
   Gln Arg Leu Gln Ala Arg Glu Gly His Arg Tyr Tyr Ala Pro Leu Leu
   275 280 285
   Asp Ala Met Val Arg Leu Ala Tyr Ala Leu Gly Lys Asp Phe Val Thr
   290 295 300
   Leu Gln Glu Arg Leu Glu Glu Ser Gln Leu Arg Arg Pro Thr Pro Arg
   305 310 315 320
   Gly Ile Thr Leu Lys Glu Leu Thr Val Arg Glu Tyr Leu His
   325 330
<210> 6
   <211> 8135
   <212> ADN
   <213> Homo sapiens
<220>
   <22.1> exon
   <222> (1)..(161)
<220>
   <221> exon
   <222> (3812)..(3950)
<220>
   <221> exon
   <222> (5426)..(5577)
<220>
   <221> exon
   <222> (7273)..(8235)
<400> 6
   cgatcagaag caggtcacac agcctgtttc ctgttttcaa acggggaact tagaaagtgg 60
   cagcccctcg gcttgtcgcc ggagctgaga accaagagct cgaaggggcc atatgacact 120
   cctcccggac ccctggacac acacagccct ggagactgga ggtcagtatt tgatcccaag 180
   ctcagctgtc ctctgcctgc tgtggcctga gtccccttct cctggggccc tgcctggcac 240
   ctgctggggg cagggtggga gggggaagag ttagtgacag ccgctgtgtc tggagctctc 300
   cttagcacac tgaggcagag gaagggacag ctcctggacc ttccatcacc tccattcctt 360
   ttgaaatgct aggcgcttgt acaacccatc ttgggcctgg agaataagtc accacacctg 420
   tgtttctcaa aagaacagtg tcagggaacc cctgcctcag cacagcctta gaggactcat 480
   ggaaaatgca gaatccaggc ctgttcaatg gcaccttcct atgttagcag ccaggaaacc 540
   tgctcttgga caagcccctg ggatcccacc cccaccccac caggggattc ttacacacac 600
   tgggttggga gcccctggct ttggcaaggc ttctcaggtg agcgtccagt tgttggaggg 660
   tacccaccct ttccccaaga gaggcagcca cacatccaac atcctgggat ctctgtctcc 720
   cagcgtgggc catgtgcttt atttcacccc ctagaggctc atcccccatg aaaagtcctc 780
   cgcaggccct cagaaagata gtgtggcctc tgtgtgccca gcagaagaag gactggactt 840
   ggcagtcagc tcttggagag ggggtggtta ggacacctgg ggacaggagg aggagaatga 900
   ctgtctgtgc acacacggct ggaaggtaca ggaggctggg aagctgctct gtcccctggg 960
   ccaactacag gcccccaggc caacagcaac aacactttta gtattttgtt ataaagtcaa 1020
   gaaatctttg ctacagaggg tgaggagagg gaaggaaagg gccatggaac cgtctatgtg 1080
   gctatcccca gagagctttt agagtgacag gattgctttc ccatttcaca gatgaggaaa 1140
   ctgaggcctg gagagggatg ggaagctacc caaggcccca tggatacacc agtgcacaac 1200
   tctttccttc cccctcctct ttaaatgggt gattcccaat gaaacctgta agagacaacc 1260
   ataagggagc tgactgtggc tgctgaattt gattttattc taaggcctgg ttttataatc 1320
   agctttctca gtctttactg gagtgtcaag ccgaggcatc atttctaggg tcttacaggg 1380
   tctctgggcc aatagtgccc tgcttctgac ctggagccag ctgcctggtc atgaaagcag 1440
   atctgcaaag gctggggccc ctgaggccaa ggccactcgc catcacccat tttacagaag 1500
   tgctgagcat aggagtgccc tgggccccca agaatcccag ccaccaagaa tcacgtaaac 1560
   catccactgt ctcacttagg caccagtcag aatgtaggga acccacccct agtcatccat 1620
   catcttatca acaggacggg gcttgtagcc acatttatca ggtagggaaa ctgaagccta 1680
   gagatattaa agcacttgct taaggacaca cggttggtca ggatggaagg cgatgtctcc 1740
   tgactccctg acaggcacaa gagacaagcg agaggtgccc gtgacggcat gctcaagaac 1800
   gtgcagccct gggccagcca ggcccctgct ccgtgcctct gtttgcccat ctgtaaaagg 1860
   tgaggttgga tcgagggtcc ctgagggccg cccactggat ggctgtgcag agccaaacgg 1920
   agaaggcccc agggttcctt tcacccgaca cagcaagcac ttccccctga agtgcaggct 1980
   ccaggcccca gctgacctcc cctctcccag gccagcggct ctcacccctg gagcaaggga 2040
   caggcgctgg ctgtgctcag ggacatgcat gactcccgcc cccatctgtg ctcagggggt 2100
   gccagggagg cactggctct atctttctct aggccgtagt cagcccaggg gttcagacca 2160
   agagcccaga atccaacaga tcagagttca agtcccagct ctacctctat gttccactgg 2220
   cagcttcctc aggtcatttg caccttcctt gtcttgaatt tccatgccta accagtatac 2280
   cagctactcc ctccagccga tctaatgttt taattgtccc tttctctaag ttgtctcaaa 2340
   catttgtaat tctattccaa tccaccttaa tttagtcatt tatttcacaa atatttctgg 2400
   aaacatctag cacttaacag acactaaaag cgggggtact acacagtccc tgggatggac 2460
   agggccctga gctgaggctt cagagtctgc ctgactgaat cctcacccca gccttgtgaa 2520
   cgtgggttct gttattatcc ccaatttata ggaaacagaa gcacagagaa gttgagtcac 2580
   ttgccagcta ccaggtcatc ccttccactt atccgggtca cagacagagt tattatgtaa 2640
   accagatccc agctgcctgt tctccctccc tgagtaaggt ggagagaatt ctgaagtcag 2700
   cccagcctgg gtctgtatcc tgcccaccac tcaccagctc ctcatctttg gcaactctaa 2760
   gtctcagttc ccttatcata aaagggagat gtaaacagtc ctgagtgcag acagtgttca 2820
   ggttagtgca agagtgtgtg ctgggtgtga agtgcacagc cagcacgtca caagcactgg 2880
   agacaaattc agctttgctt gttgcgcaca ctcaccagct gcgtgacttt agacctcagt 2940
   tttctcatct gttatgtggt ggtaatgata gacttttgtg agcattaaac tagattaggg 3000
   gctatggaga acctagatgg gtatgaagtg ggtataataa gctatcagtt aattttgctg 3060
   atagatagat tattgattga ttgatcgata gaagattcat accagtatct acctgctctg 3120
   aacactgacc tttctttttt tctttttgag atggtcttgt tctgtcaccc agactggagt 3180
   gcagtggcat catcatagct cactgcagcc tcagtctctt gggcttaagg gatcctcctg 3240
   tctcagcctc ccaagtagct gggaccacag gcgtgcatcc tggataattt ttttttattt 3300
   tttctagaga cggggtctca ctacattggc caggctggtc tcaaattcct gggctcaagt 3360
   gatccttcta acccagcctc ccaaagcgct gggattacag gcatgagtgg ccatgttcaa 3420
   cttgaacact gagacttcat tcgcatgtgt aacataaaac tgagtatcta gacaagccag 3480
   catctttctt tcaagtaatc actaaagcca atacttttac ttgaaatcat ctcatttaaa 3540
   actctgagca atacgtaagg atcacctcaa taacatatgg atcatcgcaa taggtgaagg 3600
   gtcttctctg ccttggagta acctgcccag caaaggggca gacccagatt tgggatctgg 3660
   cagctgggag agtggggaag gttgagccgt ggggcccttg tcattccctc tgcctgccag 3720
   gagggggcat gacacagctc ctaggcaccc caggagccac cgggaacccc aactggagtg 3780
   ggtcctcact gttctctttt tcctctggca gccttggagc atggcaagtc cagagcaccc 3840
   tgggagccct ggctgcatgg gacccataac ccagtgcacg gcaaggaccc agcaggaagc 3900
   accagccact ggccccgacc tcccgcaccc aggacctgac gggcacttag gtgggcttga 3960
   ggcttgagac tcggtctggg ggagaggtct gaagacattc aaagtacaaa tgtgggtcac 4020
   tttgggggat gcagcaagag gcccgggcag ctcttgtaac ttgggttatc ccaaaacaga 4080
   cactgagaca cagatctagt gcaagctgtt tatccgggag acggtcctag gagtcatggc 4140
   aggggagtgg gaatggaagg aaagggcaag aggccagggc aggacatcag tgaacagata 4200
   ggcacggtag gtggctgaag ctcaacccca gcgggggtct tctgggagac cctggaacat 4260
   atctctgggt tgtcctatcc taggggtgag gaagccgggc tgttatctac cagtcctgcc 4320
   ctgcatagga gaagggacgc tcctgggcct gctgctatgg ccctagaaag ccctcaggga 4380
   agccagtggc atgttctgga aaagtgggtg ccaagagggc acggtccagc ctggggcatg 4440
   gacagcatct gctgtagtgc catctcctgg aacagatctt ttcttacagt ccttcgagat 4500
   gccctattca atacctgctc tgttcctggc cctatgcagg gcactggaga aacagaaaca 4560
   ggaagaaatc aaacactgca ctagtcctga ggtttggtag agaaacagat cagtgagaaa 4620
   cagttacacg tgccacgaga aataaataaa taaaatgaaa aacctgtagg aacaaggtgg 4680
   gaagctctta ctctaatgcc aaggggcatt tgcagtgatg tgggggctgg gtcttgaagg 4740
   gtagactgga aaagggctgg gacccatgcc ctttgcaata aaatgcacaa ttatttgtgc 4800
   ttcttaagaa cctcagagtg gcgcagggct caagtggggt ttaagaaaca ctgtgttcgt 4860
   tttccaggcg tggaaataga gggttggatg caaggcagag cagtgcacgt ccgagaagag 4920
   cccggcatgt gggcagttag atgagaaggt taggaagggc cagcccgctg aggctggaac 4980
   ataacatcct cctcactgcc tcccctgccc actgatgtgt gctcaaggag tcgtggcaac 5040
   agtcacgaag tcagggctgc agggagcaca gaaacacaca agccaccgtc tctgcttgtc 5100
   cagagcaggg atttcaccat ggccaatcta cagaccagaa gtggacgatg caaagtgccc 5160
   gcaccgcatt ccaaagctgt gaaaccactt gggggtgatg ggctatttgg gattgtcggt 5220
   ggtagggtgg attctgccag gctgggcaca gaggtctgtc tgatgcccca attgggccta 5280
   taaatggcgg ggtgggagag agggatattc aatactcttc aggagttctg atatgccatc 5340
   tcagatagac ccagccatct ccccaagccc atgcctcgga agtgcactga cagggtgcag 5400
   atccttaagg gtgttgtcct tccagacaca cacagtggcc tgagctccaa ctccagcatg 5460
   accacgcggg agcttcagca gtactggcag aaccagaaat gccgctggaa gcacgtcaaa 5520
   ctgctctttg agatcgcttc agctcgcatc gaggagagaa aagtctctaa gtttgtggta 5580
   agcagagatt gggaaatggt ggagcctctt tcactctgct tccttcctgg ccctgaataa 5640
   gtcttgtaga gcctcaggtt tcccaactat gaaatgggtc aacacactaa ctcacagctt 5700
   tcttctggag aaaatggcca aagagcaaga tttcaggctc agcacctgct agggtctgtg 5760
   aggattcgaa ccatataagt catatttctt ggtcccaaga aggaaatagc ccagtttaat 5820
   cccatcttat caggtgtcag tcacctgtgt cctttcttca ccaattttgc catatcactg 5880
   tatctgttct aattattatt acttattttt ttctttaaat tggatcactt tttaaaaaca 5940
   tgaagcacat ttatttcaaa gagaaatacc ttaaatggaa aaccaatatc acatggcaca 6000
   aagcaaaagt aacatactag aaaagtcgat acaaggaaag tcaatacaag gaaagctatg 6060
   tgctgttatt aaattctagc tggttactgt ggcttcggga aagccctgtg cctgggagct 6120
   gctcctctcc ctgttagaat ggaattttag cttgtgttaa gggatgttaa agactgccta 6180
   agagccacac ttcatccttc tccttcactt acctgggacc gggataaata acatagctac 6240
   cactgaatgc caatggcatg ccgggcacag ctccatgtgg tttcagtgca ttaactcatt 6300
   taatcctcac tgggtgaggt aggcactatg cctatccttg ttttatgaat gagaaaagtg 6360
   agactcggag aggttaaatt actcatctaa aaccacacag ctagaccatg gtagggctat 6420
   aattacaacc catgcaatct ggctctggag tcagatgcat gggttataat tgcccttaat 6480
   atataattgc ccgtaatcag gattctcttg aaagatgatt gaaaaggatt gatttcttta 6540
   ccatataacg gcatcaccag tgtacctaaa tgatgttata ttgtacgtaa aactaattcc 6600
   caagtgtgaa acatttggaa aacacagcat ctcagttcag aaaacagagg cccagtttta 6660
   gcaagtaaag ccaagaggga ccccagcagc ctgcagggca ggaccctctg ccctttctcc 6720
   tcccagatgt ccccaccttg ctgtgttgtt gttccagggt tgactcagct gatgccaata 6780
   gcaatttaaa acagaattgg gccaggtgca gtggctcatg cctgtaatcc cagcactttg 6840
   ggaggcccag gtaggaggat cgcttgagcc caggagttgg agaccagcct gggcaacaca 6900
   gccagacccc atcttttaaa aagaatcaaa aaatctgcca ggtagtgggt gtgcctgtag 6960
   tcccagctac tcaggaggct caggtgggca ggtcaattga gcccataagt tcaaggttgc 7020
   agtgaggtat gatcgcatca ctgtactcca gcctgggtaa cagtgcgaga ccctgtctct 7080
   aaaaataaat aaataaataa ataaataaat aaataaacaa acaaacaaac aaacaaacaa 7140
   tcaattgcat ataaggatcg cccgttttca gggcatgctt tacaccggcc tggttaactt 7200
   tactctgggt gtgctccgtc cgccgcagcc cccgccggga ggtggccaca gctctctctg 7260
   gttgcgccct aggtgtacca aatcatcgtc atccagactg ggagctttga caacaacaag 7320
   gccgtcctgg aacggcgcta ttccgacttc gcgaagctcc agaaagcgct gctgaagacg 7380
   ttcagggagg agatcgaaga cgtggagttt cccaggaagc acctgactgg gaacttcgct 7440
   gaggagatga tctgtgagcg tcggcgcgcc ctgcaggagt acctgggcct gctctacgcc 7500
   atccgctgcg tgcgccgctc ccgggagttc ctggacttcc tcacgcggcc ggagctgcgc 7560
   gaggctttcg gctgcctgcg ggccggccag tacccgcgcg ccctggagct gctgctgcgc 7620
   gtgctgccgc tgcaggagaa gctcaccgcc cactgccctg cggccgccgt cccggccctg 7680
   tgcgccgtgc tgctgtgcca ccgcgacctc gaccgccccg ccgaggcctt cgcggccgga 7740
   gagagggccc tgcagcgcct gcaggcccgg gagggccatc gctactatgc gcctctgctg 7800
   gacgccatgg tccgcctggc ctacgcgctg ggcaaggact tcgtgactct gcaggagagg 7860
   ctggaggaga gccagctccg gaggcccacg ccccgaggca tcaccctgaa ggagctcact 7920
   gtgcgagaat acctgcactg agccggcctg ggaccccgca gggacgctgg agatttgggg 7980
   tcaccatggc tcacagtggg ctgtttgggg ttcttttttt ttatttttcc ttttcttttt 8040
   tgttatttga gacagtcttg ctctgtcacc cagactgaag tgcagtggct caattatgtc 8100
   tcactgcagc ctcaaactcc tgggcacaag caatc                            8135
<210> 7
   <211> 16
   <212> ADN
   <213> Homo sapiens
<400> 7
   ctgggtgcga ttgctc                                                   16
<210> 8
   <211> 16
   <212> ADN
   <213> Homo sapiens
<400> 8
   ccaggcccca tgacag                                                   16
<210> 9
   <211> 25
   <212> ADN
   <213> Homo sapiens
<400> 9
   tggtcccggc ccaatcccaa tgctt                                         25
<210> 10
   <211> 28
   <212> ADN
   <213> Homo sapiens
<400> 10
   ttcctcatgt ataaattggg tgtggcca 28
<210> 11
   <211> 25
   <212> ADN
   <213> Homo sapiens
<400> 11
   acagagtgag gaccccatct ctatc 25
<210> 12
   <211> 25
   <212> ADN
   <213> Homo sapiens
<400> 12
   tccaactgct gggattacag gcaca 25
<210> 13
   <211> 22
   <212> ADN
   <213> Homo sapiens
<400> 13
   agtccccgag accagggcaa ac 22
<210> 14
   <211> 23
   <212> ADN
   <213> Homo sapiens
<400> 14
   tccatttctg cagtacacat gca 23
<210> 15
   <211> 20
   <212> ADN
   <213> Homo sapiens
<400> 15
   ctctccccat agaaggcatc 20
<210> 16
   <211> 20
   <212> ADN
   <213> Homo sapiens
<400> 16
   ggatagagac gttctcttaa 20
<210> 17
   <211> 20
   <212> ADN
   <213> Homo sapiens
<400> 17
   caggctgaat gacagaacaa 20
<210> 18
   <211> 20
   <212> ADN
   <213> Homo sapiens
<400> 18
   attgaaaaca actccgtcca 20
<210> 19
   <211> 25
   <212> ADN
   <213> Homo sapiens
<400> 19
   atactcactt ttagacagtt caggg 25
<210> 20
   <211> 21
   <212> ADN
   <213> Homo sapiens
<400> 20
   ggctcagttc ctaaccagtt c 21
<210> 21
   <211> 20
   <212> ADN
   <213> Homo sapiens
<400> 21
   agtcagtctg tccagaggtg 20
<210> 22
   <211> 20
   <212> ADN
   <213> Homo sapiens
<400> 22
   tgaatcttac atcccatccc 20
<210> 23
   <211> 17
   <212> ADN
   <213> Homo sapiens
<400> 23
   gatcttccca aagcgcc 17
<210> 24
<211> 17
   <212> ADN
   <213> Homo sapiens
<400> 24
   tcccgtcagc caagcta 17
<210> 25
   <211> 20
   <212> ADN
   <213> Homo sapiens
<400> 25
   aagcttgtat ctttctcagg 20
<210> 26
   <211> 20
   <212> ADN
   <213> Homo sapiens
<400> 26
   atctaccttg gctgtcattg 20
<210> 27
   <211> 20
   <212> ADN
   <213> Homo sapiens
<400> 27
   cctccataat catgtgagcc 20
<210> 28
   <211> 20
   <212> ADN
   <213> Homo sapiens
<400> 28
   aatctcccca actcaagacc 20
<210> 29
   <211> 20
   <212> ADN
   <213> Homo sapiens
<400> 29
   ggatgcctgc tctaaatacc 20
<210> 30
   <211> 19
   <212> ADN
   <213> Homo sapiens
<400> 30
   cccaggggtc aaacttaat 19
<210> 31
   <211> 21
   <212> ADN
   <213> Homo sapiens
<400> 31
   ggtttgaaag tatctccagg g 21
<210> 32
   <211> 21
   <212> ADN
   <213> Homo sapiens
<400> 32
   ggtttgaaag tatctccagg g 21
<210> 33
   <211> 20
   <212> ADN
   <213> Homo sapiens
<400> 33
   gtgcatgtgt tcgtatcaac 20
<210> 34
   <211> 20
   <212> ADN
   <213> Homo sapiens
<400> 34
   tcatctccaa aggagtttct 20
<210> 35
   <211> 18
   <212> ADN
   <213> Homo sapiens
<400> 35
   aaagccaacc ttgcttca 18
<210> 36
   <211> 20
   <212> ADN
   <213> Homo sapiens
<400> 36
   tcttggaaac aggtaagtgc 20
<210> 37
   <211> 18
   <212> ADN
   <213> Homo sapiens
<400> 37
   attgccctca agaacagc 18
<210> 38
   <211> 17
   <212> ADN
   <213> Homo sapiens
<400> 38
   gtgctatgcc atcccag 17
<210> 39
   <211> 20
   <212> ADN
   <213> Homo sapiens,
<400> 39
   ccacaccagc gtttttctaa 20
<210> 40
   <211> 24
   <212> ADN
   <213> Homo sapiens
<400> 40
   cacactttac acacacctat accc 24
<210> 41
   <211> 22
   <212> ADN
   <213> Homo sapiens
<400> 41
   aagccatatt aggtctgtcc at 22
<210> 42
   <211> 19
   <212> ADN
   <213> Homo sapiens
<400> 42
   gcttgggtta aatgcgtgt 19
<210> 43
   <211> 20
   <212> ADN
   <213> Homo sapiens
<400> 43
   agcagtttgg gtaaacattg 20
<210> 44
   <211> 20
   <212> ADN
   <213> Homo sapiens
<400> 44
   aaatatgcct tctggaggtg 20
<210> 45
   <211> 20
   <212> ADN
   <213> Homo sapiens
<400> 45
   ggaggatcag gggagtttat 20
<210> 46
   <211> 24
   <212> ADN
   <213> Homo sapiens
<400> 46
   caaagtaaat gaatgtctac tgcc 24
<210> 47
   <211> 23
   <212> ADN
   <213> Homo sapiens
<400> 47
   ccaactctgt agtttcaaag agc 23
<210> 48
   <211> 20
   <212> ADN
   <213> Homo sapiens
<400> 48
   tcacagccta cttgcttggt 20
<210> 49
   <211> 25
   <212> ADN
   <213> Homo sapiens
<400> 49
   gacagcctca aatgaaatat aacac 25
<210> 50
   <211> 25
   <212> ADN
   <213> Homo sapiens
<400> 50
   gctctcagct agggtagttg tttat 25
<210> 51
   <211> 25
   <212> ADN
   <213> Homo sapiens
<400> 51
   atttttaagg aatgtaaagn acaca 25
<210> 52
   <211> 20
   <212> ADN
   <213> Homo sapiens
<400> 52
   gaccaggagt cagtaaaagg 20
<210> 53
   <211> 20
   <212> ADN
   <213> Homo sapiens
<400> 53
   gtccaaaaca ccaccctcta 20
<210> 54
   <211> 24
   <212> ADN
   <213> Homo sapiens
<400> 54
   gaagtagatc agtcatcttg ctgc 24
<210> 55
   <211> 19
   <212> ADN
   <213> Homo sapiens
<400> 55
   tcctctgggg gattcactc 19
<210> 56
   <211> 20
   <212> ADN
   <213> Homo sapiens
<400> 56
   gggacatcac caagcacaag 20
<210> 57
   <211> 25
   <212> ADN
   <213> Homo sapiens
<400> 57
   caggaaaata aatctaacac acata 25
<210> 58
   <211> 20
   <212> ADN
   <213> Homo sapiens
<400> 58
   cctgtgggca ctgataaata 20
<210> 59
   <211> 19
   <212> ADN
   <213> Homo sapiens
<400> 59
   cccagccccc atctcaccg 19
<210> 60
   <211> 19
   <212> ADN
   <213> Homo sapiens
<400> 60
   cccagccccc atctcacca 19
<210> 61
   <211> 19
   <212> ADN
   <213> Homo sapiens
<400> 61
   ctgcggagga ggctgctgg 19
<210> 62
   <211> 19
   <212> ADN
   <213> Homo sapiens
<400> 62
   tcactcccac caccctttc 19
<210> 63
   <211> 20
   <212> ADN
   <213> Homo sapiens
<400> 63
   agaagtttag tgtggcgtgg 20
<210> 64
   <211> 17
   <212> ADN
   <213> Homo sapiens
<400> 64
   gccatctccc caagccc 17
<210> 65
   <211> 18
   <212> ADN
   <213> Homo sapiens
<400> 65
   tcgatgcgag ctgaagcg 18
<210> 66
   <211> 18
   <212> ADN
   <213> Homo sapiens
<400> 66
   tcgatgcgag ctgaagca 18
<210> 67
   <211> 20
   <212> ADN
   <213> Homo sapiens
<400> 67
   tgaatgttaa agggctctgg 20
<210> 68
   <211> 19
   <212> ADN
   <213> Homo sapiens
<400> 68
   ttggttctca gctccggcg 19
<210> 69
   <211> 19
   <212> ADN
   <213> Homo sapiens
<400> 69
   ttggttctca gctccggca 19
<210> 70
   <211> 19
   <212> ADN
   <213> Homo sapiens
<400> 70
   agaaaccggg ctggctgtg 19
<210> 71
   <211> 21
   <212> ADN
   <213> Homo sapiens
<400> 71
   gcattgcctt ttgatctcta c 21
<210> 72
   <211> 18
   <212> ADN
   <213> Homo sapiens
<400> 72
   tgggctcttc tgcgggga 18
<210> 73
   <211> 18
   <212> ADN
   <213> Homo sapiens
<400> 73
   tgggctcttc tgcggggg 18
<210> 74
   <211> 20
   <212> ADN
   <213> Homo sapiens
<400> 74
   tgcctcttct tctgccttcc 20
<210> 75
   <211> 22
   <212> ADN
   <213> Homo sapiens
<400> 75
   cgagctgtac ctgaggaagc gt 22
<210> 76
   <211> 24
   <212> ADN
   <213> Homo sapiens
<400> 76
   cctgagctgt acctgaggaa gcgc 24
<210> 77
   <211> 20
   <212> ADN
   <213> Homo sapiens
<400> 77
   catcatgagc ccggggtggc 20
<210> 78
   <211> 23
   <212> ADN
   <213> Homo sapiens
<400> 78
   tttctcttgg cttcctggtg cgt 23
<210> 79
   <211> 25
   <212> ADN
   <213> Homo sapiens
<400> 79
   accttctctt ggcttcctgg tgcgg 25
<210> 80
   <211> 26
   <212> ADN
   <213> Homo sapiens
<400> 80
   gccaaaggtg tcgtgccagg gctcca 26
<210> 81
   <211> 20
   <212> ADN
   <213> Homo sapiens
<400> 81
   atctgagaag gccctgctct 20
<210> 82
   <211> 20
   <212> ADN
   <213> Homo sapiens
<400> 82
   atctgagaag gccctgctcc 20
<210> 83
   <211> 19
   <212> ADN
   <213> Homo sapiens
<400> 83
   cccacactta gccttgatg 19
<210> 84
   <211> 19
   <212> ADN
   <213> Homo sapiens
<400> 84
   atgagttagc ccagcggag 19
<210> 85
   <211> 19
   <212> ADN
   <213> Homo sapiens
<400> 85
   attgagagcc cttggagtg 19
<210> 86
   <211> 19
   <212> ADN
   <213> Homo sapiens
<400> 86
   tgatttcgta agacaagtg 19
<210> 87
   <211> 20
   <212> ADN
   <213> Homo sapiens
<400> 87
   agcaaattct aggagttatg 20
<210> 88
   <211> 19
   <212> ADN
   <213> Homo sapiens
<400> 88
   agctgagatg tccggatcg 19
<210> 89
   <211> 18
   <212> ADN
   <213> Homo sapiens
<400> 89
   agctgagatt ccggatca 18
<210> 90
   <211> 20
   <212> ADN
   <213> Homo sapiens
<400> 90
   gtcctcttaa cttcccttcc                                                    20

## Description

La présente invention concerne des gènes impliqués dans les maladies inflammatoires et/ou immunes et certains cancers, en particulier les maladies inflammatoires cryptogénétiques de l'intestin, ainsi que les protéines codées par ces gènes. Des méthodes de diagnostics de maladies inflammatoires sont également des objets de la présente invention.

Les maladies inflammatoires cryptogénétiques de l'intestin (MICI) sont des maladies caractérisées par une inflammation du tube digestif dont la cause est inconnue. Selon la localisation et les caractéristiques de l'inflammation on distingue deux entités nosologiques différentes: la rectocolite hémorragique (RCH) et la maladie de Crohn (MC). La RCH a été décrite par S Wilkes en 1865 tandis que le premier cas d'iléite régionale a été rapportée par Crohn en 1932. En réalité, il est possible que ces deux maladies soient beaucoup plus anciennes.

Les MICI sont des maladies chroniques qui évoluent tout au long de la vie et qui touchent environ 1 à 2 personnes sur 1000 habitants dans les pays occidentaux, ce qui représente entre 60.000 et 100.000 malades en France. Il s'agit de maladies apparaissant chez le sujet jeune (le pic d'incidence est dans la troisième décennie), évoluant par poussées entrecoupées de rémissions, avec des complications fréquentes telles que la dénutrition, le retard de croissance chez l'enfant, la déminéralisation osseuse et à terme la dégénérescence maligne vers le cancer du colon. Il n'existe pas de traitement spécifique. Les thérapeutiques habituelles font appel aux anti-inflammatoires, aux immunosuppresseurs et à la chirurgie. Tous ces moyens thérapeutiques sont eux-mêmes source d'une morbidité iatrogène importante. Pour toutes ces raisons les MICI apparaissent comme un important problème de santé publique.

L'étiologie des MICI est actuellement inconnue. Des facteurs d'environnement sont impliqués dans la survenue de la maladie comme en témoignent l'augmentation séculaire d'incidence de la maladie et la concordance incomplète chez les jumeaux monozygotes. Les seuls facteurs de risque environnementaux actuellement reconnus sont 1) le tabac dont le rôle est néfaste dans la MC et bénéfique dans la RCH et 2) l'appendicectomie qui a un rôle protecteur pour la RCH.

Une prédisposition génétique est depuis longtemps suspectée devant l'existence d'agrégations ethniques et familiales de ces maladies. En effet, les MICI sont plus fréquentes dans la population caucasienne et en particulier la population juive d'Europe centrale. Les formes familiales représentent de 6 à 20% des cas de MICI. Elles sont particulièrement fréquentes lorsque le début de la maladie est précoce. Cependant, ce sont les études chez les jumeaux qui ont permis de confirmer le caractère génétique de ces maladies. En effet, le taux de concordance entre jumeaux pour ces maladies est plus important chez les jumeaux monozygotes que chez les jumeaux dizygotes plaidant fortement pour une composante héréditaire aux MICI, en particulier à la MC. Selon toute vraisemblance, les MICI sont des maladies génétiques complexes faisant intervenir plusieurs gènes différents, en interaction entre eux et avec des facteurs d'environnement. Les MICI peuvent donc être classées dans le cadre des maladies multifactorielles.

Deux grandes stratégies ont été développées afin de mettre en évidence les gènes de susceptibilité aux MICI. La première repose sur l'analyse de gènes candidats pour des raisons physiopathologiques. Ainsi de nombreux gènes ont été proposés comme potentiellement importants pour les MICI. Il s'agit souvent de gènes ayant un rôle dans l'inflammation et la réponse immune. On peut citer les gènes HLA, TAP, TNF, MICA, le récepteur T du lymphocyte, ICAM1, l'interleukine 1, CCR5, etc. D'autres gènes participent à des fonctions diverses tels que GA12, la motiline, MRAMP, HMLH1, etc. En réalité, aucun des différents gènes candidats étudiés n'a actuellement fait la preuve définitive de son rôle dans la survenue des MICI,

Le récent développement de cartes du génome humain utilisant des marqueurs génétiques hautement polymorphes a permis aux généticiens de développer une approche non ciblée sur l'ensemble du génome. Cette démarche, appelée aussi génétique inverse ou clonage positionnel, ne fait aucune hypothèse sur les gènes impliqués dans la maladie et tente de découvrir ceux-ci à travers un criblage systématique du génome. La méthode la plus utilisée pour les maladies génétiques complexes repose sur l'étude de l'identité par la descendance des malades d'une même famille. Cette valeur est calculée pour un grand nombre (300-400) de marqueurs de polymorphisme répartis régulièrement (tous les 10cM) sur le génome. En cas d'excès d'identité entre malades, le(s) marqueur(s) testé(s) indique(nt) une région supposée contenir un gène de susceptibilité à la maladie. Dans le cas des maladies génétiques complexes, le modèle sous-jacent à la prédisposition génétique (nombre de gènes et importance respective de chacun d'entre eux) étant inconnu, les méthodes statistiques à utiliser devront être adaptées.

La présente invention concerne la mise en évidence de la séquence nucléique de gènes impliqués dans les MICI, et d'autres maladies inflammatoires, ainsi que l'utilisation de ces séquences nucléiques.

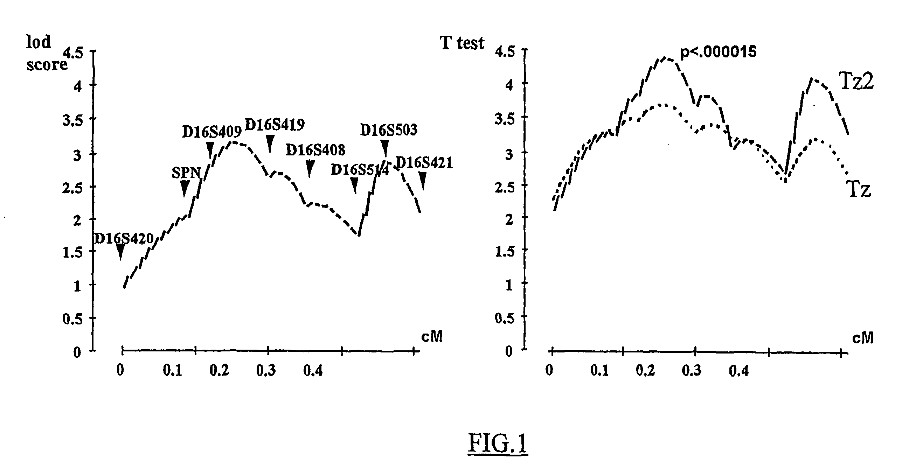

Dans le cadre de la présente invention, des travaux préliminaires des inventeurs ont déjà permis de localiser un gène de susceptibilité à la MC. En effet, les inventeurs (Hugot et al., 1996) ont montré qu'un gène de susceptibilité à la MC était localisé dans la région péricentromérique du chromosome 16 (figure 1). Il s'agissait du premier gène de susceptibilité à une maladie génétique complexe localisé par clonage positionnel et satisfaisant aux critères stricts proposés dans la littérature (Lander et Kruglyak, 1995). Ce gène a été nommé IBD1 (pour Inflammatory Bowel Disease 1). Depuis, d'autres localisations ont été proposées par d'autres auteurs en particulier sur les chromosomes 12, 1, 3, 6 et 7 (Satsangi et al., 1996 ; Cho et al., 1998). Bien que localisés, aucun de ces gènes de susceptibilité aux MICI n'a actuellement pu être identifié.

Certains auteurs n'ont pu répliquer cette localisation (Rioux et al., 1998). Ceci n'est cependant pas surprenant dans le cas de maladies génétiques complexes où une hétérogénéité génétique est probable.

Il est intéressant de noter que selon la même approche de clonage positionnel, des localisations ont aussi été proposées sur le chromosome 16 pour plusieurs maladies immunes et inflammatoires telles que la spondylarthrite ankylosante, le syndrome de Blau, le psoriasis, etc. (Becker et al., 1998 ; Tromp et al., 1996). Toutes ces maladies pourraient alors partager un même gène (ou un même groupe de gènes) localisé sur le chromosome 16.

Le maximum des tests de liaison génétique est situé pratiquement toujours à la même position, au niveau de D16S409 ou D16S411 séparés seulement de 2cM. Ce résultat est en opposition avec la taille importante (habituellement supérieure à 20cM) de l'intervalle de confiance attribuable à la localisation génétique selon une démarche utilisant des analyses de liaison non paramétriques.

La comparaison des tests statistiques utilisés dans les travaux des inventeurs montre que les tests basés sur l'identité par descendance complète (Tz2) sont meilleurs que les tests basé sur la moyenne de l'identité par descendance (Tz) (fig. 1). Une telle différence peut être expliquée par un effet récessif de IBD1.

Plusieurs gènes connus dans la région péricentromérique du chromosome 16, tels que le récepteur à l'interleukine 4, CD19, CD43, CD11, apparaissent comme de bons candidats potentiels pour la MC. Des résultats préliminaires ne plaident cependant pas en faveur de l'implication de ces gènes dans la MC.

En particulier, la présente invention fournit la séquence non seulement du gène IBD1, mais également la séquence partielle d'un autre gène, appelé IBD1prox en raison de sa localisation à proximité d'IBD, et mis en évidence comme rapporté dans les exemples ci-après. Ces gènes dont la séquence d'ADNc correspond respectivement à SEQ ID N° 1 et SEQ ID N° 4 sont donc potentiellement impliqués dans de nombreuses maladies inflammatoires et/ou immunes ainsi que dans des cancers.

La séquence peptidique exprimée par les gènes IBD1 et IBD1prox est représentée par SEQ ID N° 2 et SEQ ID N° 5 respectivement; la séquence génomique de ces gènes est représentée par SEQ ID N° 3 et SEQ ID N° 6 respectivement.

Ainsi, la présente invention a pour objet un acide nucléique purifié ou isolé, caractérisé en ce qu'il comprend une séquence nucléique choisie dans le groupe de séquences suivantes :
a) SEQ ID N° 1 et SEQ ID N° 3,
b) la séquence complémentaire ou la séquence de l'ARN correspondant à une séquence telle que définie en a).

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADNs. Ainsi, les séquences nucléiques selon l'invention englobent également les PNA (Peptid Nucleic Acid), ou analogues.

Il doit être compris que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par copie, leur environnement ayant été au moins partiellement modifié. On entend ainsi également désigner les acides nucléiques obtenus par synthèse chimique.

On entend également désigner par séquence nucléique variante tout ARN ou ADNc résultant d'une mutation et/ou variation d'un site d'épissage de la séquence nucléique génomique dont l'ADNc a pour séquence SEQ ID N° 1.

L'invention concerne de préférence un acide nucléique purifié ou isolé selon la présente invention, caractérisé en ce qu'il comprend ou est constitué de la séquence SEQ ID N° 1, de ses séquences complémentaires ou des séquences de l'ARN correspondant à SEQ ID N° 1.

Les amorces ou sondes, caractérisées en ce qu'elles comprennent une séquence d'un acide nucléique selon l'invention, font également partie de l'invention.

Ainsi, la présente invention concerne également les amorces ou les sondes selon l'invention qui peuvent permettre en particulier de mettre en évidence ou de discriminer les séquences nucléiques variantes, ou d'identifier la séquence génomique des gènes dont l'ADNc est représenté par SEQ ID N° 1 en utilisant notamment une méthode d'amplification telle que la méthode PCR, ou une méthode apparentée.

L'invention concerne également l'utilisation d'une séquence d'acide nucléique selon l'invention comme sonde ou amorce, pour la détection, l'identification, le dosage ou l'amplification de séquence d'acide nucléique.

Selon l'invention, les polynucléotides pouvant être utilisés comme sonde ou comme amorce dans des procédés de détection, d'identification, de dosage ou d'amplification de séquence nucléique, présentent une taille minimale de 15 bases, de préférence de 20 bases, ou mieux de 25 à 30 bases.

Les sondes et amorces selon l'invention peuvent être marquées directement ou indirectement par un composé radioactif ou non radioactif par des méthodes bien connues de l'homme du métier, afin d'obtenir un signal détectable et/ou quantifiable.

Les séquences de polynucléotides selon l'invention non marquées peuvent être utilisées directement comme sonde ou amorce.

Les séquences sont généralement marquées pour obtenir des séquences utilisables pour de nombreuses applications. Le marquage des amorces ou des sondes selon l'invention est réalisé par des éléments radioactifs ou par des molécules non radioactives.

Parmi les isotopes radioactifs utilisés, on peut citer le ³²P, le ³³P, le ³⁵S, le ³H ou le ¹²⁵I. Les entités non radioactives sont sélectionnées parmi les ligands tels la biotine, l'avidine, la streptavidine, la dioxygénine, les haptènes, les colorants, les agents luminescents tels que les agents radioluminescents, chémoluminescents, bioluminescents, fluorescents, phosphorescents.

Les polynucléotides selon l'invention peuvent ainsi être utilisés comme amorce et/ou sonde dans des procédés mettant en oeuvre notamment la technique de PCR (amplification en chaîne par polymérase) (Rolfs et al., 1991). Cette technique nécessite le choix de paires d'amorces oligonucléotidiques encadrant le fragment qui doit être amplifié. On peut, par exemple, se référer à la technique décrite dans le brevet américain U.S. N° 4,683,202. Les fragments amplifiés peuvent être identifiés, par exemple après une électrophorèse en gel d'agarose ou de polyacrylamide, ou après une technique chromatographique comme la filtration sur gel ou la chromatographie échangeuse d'ions, puis séquencés. La spécificité de l'amplification peut être contrôlée en utilisant comme amorces les séquences nucléotidiques de polynucléotides de l'invention et comme matrices, des plasmides contenant ces séquences ou encore les produits d'amplification dérivés. Les fragments nucléotidiques amplifiés peuvent être utilisés comme réactifs dans des réactions d'hybridation afin de mettre en évidence la présence, dans un échantillon biologique, d'un acide nucléique cible de séquence complémentaire à celle desdits fragments nucléotidiques amplifiés.

L'invention vise également les acides nucléiques susceptibles d'être obtenus par amplification à l'aide d'amorces selon l'invention.

D'autres techniques d'amplification de l'acide nucléique cible peuvent être avantageusement employées comme alternative à la PCR (PCR-like) à l'aide de couple d'amorces de séquences nucléotidiques selon l'invention. Par PCR-like on entend désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues. En général il s'agit de l'amplification de l'ADN par une polymérase ; lorsque l'échantillon d'origine est un ARN il convient préalablement d'effectuer une transcription reverse. Il existe actuellement de très nombreux procédés permettant cette amplification, comme par exemple la technique SDA (Strand Displacement Amplification) ou technique d'amplification à déplacement de brin (Walker et al., 1992), la technique TAS (Transcription-based Amplification System) décrite par Kwoh et al. (1989), la technique 3SR (Self-Sustained Sequence Replication) décrite par Guatelli et al. (1990), la technique NASBA (Nucleic Acid Sequence Based Amplification) décrite par Kievitis et al. (1991), la technique TMA (Transcription Mediated Amplification), la technique LCR (Ligase Chain Reaction) décrite par Landegren et al. (1988), la technique de RCR (Repair Chain Reaction) décrite par Segev (1992), la technique CPR (Cycling Probe Reaction) décrite par Duck et al. (1990), la technique d'amplification à la Q-béta-réplicase décrite par Miele et al. (1983). Certaines de ces techniques ont depuis été perfectionnées.

Dans le cas où le polynucléotide cible à détecter est un ARNm, on utilise avantageusement, préalablement à la mise en oeuvre d'une réaction d'amplification à l'aide des amorces selon l'invention ou à la mise en oeuvre d'un procédé de détection à l'aide des sondes de l'invention, une enzyme de type transcriptase inverse afin d'obtenir un ADNc à partir de l'ARNm contenu dans l'échantillon biologique. L'ADNc obtenu servira alors de cible pour les amorces ou les sondes mises en oeuvre dans le procédé d'amplification ou de détection selon l'invention.

La technique d'hybridation de sondes peut être réalisée de manières diverses (Matthews et al., 1988). La méthode la plus générale consiste à immobiliser l'acide nucléique extrait des cellules de différents tissus ou de cellules en culture sur un support (tels que la nitrocellulose, le nylon, le polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

Selon un autre mode de mise en oeuvre des sondes nucléiques selon l'invention, ces dernières peuvent être utilisées comme sondes de capture. Dans ce cas, une sonde, dite « sonde de capture », est immobilisée sur un support et sert à capturer par hybridation spécifique l'acide nucléique cible obtenu à partir de l'échantillon biologique à tester et l'acide nucléique cible est ensuite détecté grâce à une seconde sonde, dite « sonde de détection », marquée par un élément facilement détectable.

Parmi les fragments d'acides nucléiques intéressants, il faut ainsi citer en particulier les oligonucléotides anti-sens, c'est-à-dire dont la structure assure, par hybridation avec la séquence cible, une inhibition de l'expression du produit correspondant. Il faut également citer les oligonucléotides sens qui, par interaction avec des protéines impliquées dans la régulation de l'expression du produit correspondant, induiront soit une inhibition, soit une activation de cette expression.

Dans les deux cas (sens et anti-sens), les oligonucléotides de l'invention peuvent être utilisés *in vitro* et *in vivo.*

La présente invention concerne également un polypeptide isolé caractérisé en ce qu'il comprend un polypeptide correspondant à SEQ ID N° 2.

Par « polypeptide », on entend, au sens de la présente invention, désigner des protéines ou des peptides.

La présente invention concerne également les vecteurs de clonage et/ou d'expression comprenant un acide nucléique ou codant pour un polypeptide selon l'invention. Un tel vecteur peut également contenir les éléments nécessaires à l'expression et éventuellement à la sécretion du polypeptide dans une cellule hôte. Une telle cellule hôte est également un objet de l'invention.

Les vecteurs caractérisés en ce qu'ils comportent une séquence de promoteur et/ou de régulateur selon l'invention, font également partie de l'invention.

Lesdits vecteurs comportent de préférence un promoteur, des signaux d'initiation et de terminaison de la traduction, ainsi que des régions appropriées de régulation de la transcription. Ils doivent pouvoir être maintenus de façon stable dans la cellule et peuvent éventuellement posséder des signaux particuliers spécifiant la sécrétion de la protéine traduite.

Ces différents signaux de contrôle sont choisis en fonction de l'hôte cellulaire utilisé. A cet effet, les séquences d'acide nucléique selon l'invention peuvent être insérées dans des vecteurs à réplication autonome au sein de l'hôte choisi, ou des vecteurs intégratifs de l'hôte choisi.

Parmi les systèmes à réplication autonome, on utilise de préférence en fonction de la cellule hôte, des systèmes de type plasmidique ou viral, les vecteurs viraux pouvant notamment être des adénovirus (Perricaudet et al., 1992), des rétrovirus, des lentivirus, des poxvirus ou des virus herpétiques (Epstein et al., 1992). L'homme du métier connaît les technologies utilisables pour chacun de ces systèmes.

Lorsque l'on souhaite l'intégration de la séquence dans les chromosomes de la cellule hôte, on peut utiliser par exemple des systèmes de type plasmidique ou viral ; de tels virus sont, par exemple, les rétrovirus (Temin, 1986), ou les AAV (Carter, 1993).

Parmi les vecteurs non viraux, on préfère les polynucléotides nus tels que l'ADN nu ou l'ARN nu selon la technique développée par la société VICAL, les chromosomes artificiels de bactérie (BAC, bacterial artificial chromosome), les chromosomes artificiels de levure (YAC, yeast artificial chromosome) pour l'expression dans la levure, les chromosomes artificiels de souris (MAC, mouse artificial chromosome) pour l'expression dans les cellules murines et de manière préférée les chromosomes artificiels d'homme (HAC, human artificial chromosome) pour l'expression dans les cellules humaines.

De tels vecteurs sont préparés selon les méthodes couramment utilisées par l'homme du métier, et les clones en résultant peuvent être introduits dans un hôte approprié par des méthodes standard, telles que par exemple la lipofection, l'électroporation, le choc thermique, la transformation après perméabilisation chimique de la membrane, la fusion cellulaire.

L'invention comprend en outre les cellules hôtes, notamment les cellules eucaryotes et procaryotes, transformées par les vecteurs selon l'invention ainsi que les animaux transgéniques, de préférence les mammifères, excepté l'Homme, comprenant une desdites cellules transformées selon l'invention. Ces animaux peuvent être utilisés en temps que modèles, pour l'étude de l'étiologie de maladies inflammatoires et/ou immunes, et en particulier des maladies inflammatoires du tube digestif, ou pour l'étude de cancers.

Parmi les cellules utilisables aux sens de la présente invention, on peut citer les cellules bactériennes (Olins et Lee, 1993), mais aussi les cellules de levure (Buckholz, 1993), de même que les cellules animales, en particulier les cultures de cellules de mammifères (Edwards et Aruffo, 1993), et notamment les cellules d'ovaire de hamster chinois (CHO). On peut citer également les cellules d'insectes dans lesquelles on peut utiliser des procédés mettant par exemple en oeuvre des baculovirus (Luckow, 1993). Un hôte cellulaire préféré pour l'expression des protéines de l'invention est constitué par les cellules COS.

Parmi les mammifères selon l'invention, on préfère des animaux tels que les rongeurs, en particulier les souris, les rats ou les lapins, exprimant un polypeptide selon l'invention.

Parmi les mammifères selon l'invention, on préfère également des animaux tels que les souris, les rats ou les lapins, caractérisés en ce que le gène codant pour la protéine de séquence SEQ ID N° 2 ou dont la séquence est codée par le gène homologue chez ces animaux, n'est pas fonctionnel, est invalidé ou présente au moins une mutation.

Ces animaux transgéniques sont obtenus par exemple par recombinaison homologue sur cellules souches embryonnaires, transfert de ces cellules souches à des embryons, sélection des chimères affectées au niveau des lignées reproductrices, et croissance desdites chimères.

Les animaux transgéniques selon l'invention peuvent ainsi surexprimer le gène codant pour la protéine selon l'invention, ou leur gène homologue, ou exprimer ledit gène dans lequel est introduite une mutation. Ces animaux transgéniques, en particulier des souris, sont obtenus par exemple par transfection de copie de ce gène sous contrôle d'un promoteur fort de nature ubiquitaire, ou sélectif d'un type de tissu, ou après transcription virale.

Alternativement, les animaux transgéniques selon l'invention peuvent être rendus déficients pour le gène codant pour l'un des polypeptides de séquence SEQ ID N° 2 ou ses gènes homologues, par inactivation à l'aide du système LOXP/CRE recombinase (Rohlmann et al., 1996) ou de tout autre système d'inactivation de l'expression de ce gène.

Les cellules et mammifères selon l'invention sont utilisables dans une méthode de production d'un polypeptide selon l'invention, comme décrit ci-dessous, et peuvent également servir à titre de modèle d'analyse.

Les cellules ou mammifères transformés tels que décrits précédemment peuvent aussi être utilisés à titre de modèles afin d'étudier les interactions entre les polypeptides selon l'invention, et les composés chimiques ou protéiques, impliqués directement ou indirectement dans les activités des polypeptides selon l'invention, ceci afin d'étudier les différents mécanismes et interactions mis en jeu.

Ils peuvent en particulier être utilisés pour la sélection de produits interagissant avec les polypeptides selon l'invention, notamment la protéine de séquence SEQ ID N° 2 ou ses variants selon l'invention, à titre de cofacteur, ou d'inhibiteur, notamment compétitif, ou encore ayant une activité agoniste ou antagoniste de l'activité des polypeptides selon l'invention. De préférence, on utilise lesdites cellules transformées ou animaux transgéniques à titre de modèle notamment pour la sélection de produits permettant de lutter contre les pathologies liées à une expression anormale de ce gène.

L'invention concerne également l'utilisation d'une cellule, d'un mammifère ou d'un polypeptide selon l'invention pour le criblage de composés chimiques ou biochimiques pouvant interagir directement ou indirectement avec les polypeptides selon l'invention, et/ou capable de moduler l'expression ou l'activité de ces polypeptides.

De la même façon, l'invention concerne aussi un procédé de criblage de composés capables d'interagir *in vitro* ou *in vivo* avec un acide nucléique selon l'invention, en utilisant un acide nucléique une cellule ou un mammifère selon l'invention, et en détectant la formation d'un complexe entre les composés candidats et l'acide nucléique selon l'invention.

Les composés ainsi sélectionnés sont également objets de l'invention.

L'invention concerne aussi l'utilisation d'une séquence d'acide nucléique selon l'invention pour la synthèse de polypeptides recombinants.

La méthode de production d'un polypeptide de l'invention sous forme recombinante, elle-même comprise dans la présente invention, se caractérise en ce que l'on cultive les cellules transformées, notamment les cellules ou mammifères de la présente invention, dans des conditions permettant l'expression d'un polypeptide recombinant codé par une séquence d'acide nucléique selon l'invention, et que l'on récupère ledit polypeptide recombinant.

Les polypeptides recombinants, caractérisés en ce qu'ils sont susceptibles d'être obtenus par ladite méthode de production, font également partie de l'invention.

Les polypeptides recombinants obtenus comme indiqué ci-dessus, peuvent aussi bien se présenter sous forme glycosylée que non glycosylée et peuvent présenter ou non la structure tertiaire naturelle.

Les séquences des polypeptides recombinants peuvent être également modifiées afin d'améliorer leur solubilité, en particulier dans les solvants aqueux.

De telles modifications sont connues de l'homme du métier comme par exemple la délétion de domaines hydrophobes ou la substitution d'acides aminés hydrophobes par des acides aminés hydrophiles.

Ces polypeptides peuvent être produits à partir des séquences d'acide nucléique définies ci-dessus, selon les techniques de production de polypeptides recombinants connues de l'homme du métier. Dans ce cas, la séquence d'acide nucléique utilisée est placée sous le contrôle de signaux permettant son expression dans un hôte cellulaire.

Un système efficace de production d'un polypeptide recombinant nécessite de disposer d'un vecteur et d'une cellule hôte selon l'invention.

Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

Les procédés utilisés pour la purification d'un polypeptide recombinant sont connus de l'homme du métier. Le polypeptide recombinant peut être purifié à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps monoclonaux ou polyclonaux spécifiques, etc...

Les polypeptides selon la présente invention peuvent aussi être obtenus par synthèse chimique en utilisant l'une des nombreuses synthèses peptidiques connues, par exemple les techniques mettant en oeuvre des phases solides (voir notamment Stewart et al., 1954) ou des techniques utilisant des phases solides partielles, par condensation de fragments ou par une synthèse en solution classique.

Les polypeptides obtenus par synthèse chimique et pouvant comporter des acides aminés non naturels correspondants sont également compris dans l'invention.

Les anticorps mono- ou polyclonaux ou leurs fragments, anticorps chimériques ou immunoconjugués, caractérisés en ce qu'ils sont capables de reconnaître spécifiquement un polypeptide selon l'invention, font partie de l'invention.

Des anticorps polyclonaux spécifiques peuvent être obtenus à partir d'un sérum d'un animal immunisé contre les polypeptides selon l'invention, notamment produit par recombinaison génétique ou par synthèse peptidique, selon les modes opératoires usuels.

On note notamment l'intérêt d'anticorps reconnaissant de façon spécifique certains polypeptides, variants, ou leurs fragments immunogènes, selon l'invention.

Les anticorps mono- ou polyclonaux ou leurs fragments, anticorps chimériques ou immunoconjugués, caractérisés en ce qu'ils sont capables de reconnaître spécifiquement les polypeptides de séquence SEQ ID N° 2 sont particulièrement préférés.

Les anticorps monoclonaux spécifiques peuvent être obtenus selon la méthode classique de culture d'hybridomes décrite par Köhler et Milstein (1975).

Les anticorps selon l'invention sont, par exemple, des anticorps chimériques, des anticorps humanisés, des fragments Fab ou F(ab')₂. Ils peuvent également se présenter sous forme d'immunoconjugués ou d'anticorps marqués afin d'obtenir un signal détectable et/ou quantifiable.

L'invention concerne également des méthodes pour la détection et/ou la purification d'un polypeptide selon l'invention, caractérisées en ce qu'elles mettent en oeuvre un anticorps selon l'invention.

L'invention comprend en outre des polypeptides purifiés, caractérisés en ce qu'ils sont obtenus par une méthode selon l'invention.

Par ailleurs, outre leur utilisation pour la purification des polypeptides, les anticorps de l'invention, en particulier les anticorps monoclonaux, peuvent également être utilisés pour la détection de ces polypeptides dans un échantillon biologique.

Ils constituent ainsi un moyen d'analyse immunocytochimique ou immuno-histochimique de l'expression des polypeptides selon l'invention, notamment les polypeptides de séquence SEQ ID N° 2 ou l'un de ses variants, sur des coupes de tissus spécifiques, par exemple par immunofluorescence, marquage à l'or, immuno-conjugués enzymatiques.

Ils peuvent permettre notamment de mettre en évidence une expression anormale de ces polypeptides dans les tissus ou prélèvements biologiques.

Plus généralement, les anticorps de l'invention peuvent être avantageusement mis en oeuvre dans toute situation où l'expression d'un polypeptide selon l'invention, normal ou muté, doit être observée.

Ainsi, un procédé de détection d'un polypeptide selon l'invention dans un échantillon biologique, comprenant les étapes de mise en contact de l'échantillon biologique avec un anticorps selon l'invention et de mise en évidence du complexe antigène-anticorps formé est également un objet de l'invention, ainsi qu'une trousse permettant de mettre en oeuvre un tel procédé. Une telle trousse contient en particulier :
a) un anticorps monoclonal ou polyclonal selon l'invention ;
b) éventuellement des réactifs pour la constitution d'un milieu propice à la réaction immunologique ;
c) les réactifs permettant la détection du complexe antigène-anticorps produit lors de la réaction immunologique.

Les anticorps selon l'invention peuvent également être utilisés dans le traitement d'une maladie inflammatoire et/ou immune, ou d'un cancer, chez l'homme, lorsque l'on observe une expression anormale du gène IBD1. Une expression anormale signifie une surexpression ou l'expression d'une protéine mutée.

Ces anticorps peuvent être obtenus directement à partir de sérum humain, ou à partir d'animaux immunisés avec des polypeptides selon l'invention, puis « humanisés », et peuvent être utilisés tels quels ou dans la préparation d'un médicament destiné au traitement des maladies précitées.

Font également partie de l'invention, les méthodes de détermination d'une variabilité allélique, d'une mutation, d'une délétion, d'une perte d'hétérozygotie ou de toute anomalie génétique du gène selon l'invention, caractérisées en ce qu'elles mettent en oeuvre une séquence d'acide nucléique, un polypeptide ou un anticorps selon l'invention.

L'invention fournit en effet la séquence du gène IBD1 impliqué dans des maladies inflammatoires et/ou immunes, et en particulier les MICI. Un des enseignements de l'invention est de préciser les mutations dans ces séquences nucléiques ou polypeptidiques, qui sont liées à un phénotype correspondant à une des ces maladies inflammatoires et/ou immunes.

On peut détecter ces mutations directement par analyse de l'acide nucléique et des séquences selon l'invention (ADN génomique, ARN, ou ADNc), mais également par l'intermédiaire des polypeptides selon l'invention. En particulier, l'utilisation d'un anticorps selon l'invention qui reconnaît un épitope portant une mutation permet de discriminer entre une protéine « saine » et une protéine « associée à une pathologie».

Ainsi, l'étude du gène IBD1 dans diverses maladies inflammatoires et/ou immunes humaines montre ainsi qu'il existe des variants de séquence de ce gène dans la maladie de Crohn, la rectocolite hémorragique et le syndrome de Blau, comme démontré par les exemples. Ces variations de séquence aboutissent à des variations importantes de la séquence protéique déduite. En effet, elles sont soit localisées sur des sites très conservés de la protéine dans des domaines fonctionnels importants, soit elles aboutissent à la synthèse d'une protéine tronquée. Il est donc extrêmement probable que ces altérations entraînent une modification de la fonction de la protéine et aient donc un effet causal dans la survenue de ces maladies.

La variété des maladies où sont observées ces mutations suggère que le gène IBD1 est potentiellement important dans de nombreuses maladies inflammatoires et/ou immunes. Ce résultat est à rapprocher du fait que la région péricentromérique du chromosome 16 a été décrite comme contenant des gènes de susceptibilité à diverses maladies humaines telles que la spondylarthrite ankylosante ou le rhumatisme psoriasique. On peut donc considérer qu'IBD1 a un rôle important dans un grand nombre de maladies inflammatoires et/ou immunes.

En particulier, on peut associer IBD1 aux maladies inflammatoires granulomateuses. En effet, le Syndrome de Blau et la MC sont des maladies faisant partie de cette famille. On espère donc trouver des variations dans le gène IBD1 pour les autres maladies de la même famille (sarcoïdose, maladie de Behçet...).

De plus, l'implication de IBD1 dans les voies cellulaires aboutissant à l'apoptose soulève la question de son éventuel rôle carcinogène. En effet, il est attendu qu'une dysrégulation de IBD1 puisse aboutir à une prédisposition cancéreuse. Cette hypothèse est renforcée par le fait qu'il existe une prédisposition au cancer du colon dans les maladies inflammatoires de l'intestin. IBD1 pourrait en partie expliquer cette susceptibilité au cancer et définir de nouvelles voies de carcinogenèse.

La description précise des mutations observables dans le gène IBD1 permet ainsi de poser les bases d'un diagnostic moléculaire des maladies inflammatoires et immunes où son rôle est démontré. Une telle démarche, basée sur la recherche de mutations dans le gène, permettra de contribuer au diagnostic de ces maladies et éventuellement de réduire l'importance de certains examens complémentaires invasifs ou coûteux. L'invention pose les bases d'un tel diagnostic moléculaire basé sur la recherche de mutations dans IBD1.

Le diagnostic moléculaire des maladies inflammatoires devrait aussi permettre d'améliorer la classification nosologique de ces maladies et de mieux définir des sous-groupes de malades particuliers par leur caractéristiques cliniques, l'évolutivité de la maladie ou la réponse à certains traitements. A titre d'exemple, le démembrement des mutations existantes pourrait ainsi permettre de classer les colites actuellement indéterminées qui représentent plus de 10% des maladies inflammatoires de l'intestin. Une telle démarche permettra de proposer une prise en charge précoce adaptée à chaque patient. D'une manière générale, une telle démarche permet d'espérer pouvoir définir à terme une prise en charge individualisée de la maladie, en fonction du terrain génétique de chaque malade, incluant des mesures curatives et préventives.

En particulier, on préfère une méthode de diagnostic et/ou d'évaluation pronostique d'une maladie inflammatoire ou d'un cancer caractérisée en ce qu'on détermine à partir d'un prélèvement biologique d'un patient la présence d'au moins une mutation et/ou une altération d'expression du gène correspondant à SEQ ID N° 1 par l'analyse de tout ou partie d'une séquence nucléique correspondant audit gène. On peut aussi étudier le gène SEQ ID N° 3.

Cette méthode de diagnostic et/ou d'évaluation pronostique peut être utilisée de façon préventive (étude d'une prédisposition à ces maladies inflammatoires ou au cancer), ou afin de servir à l'établissement et/ou la confirmation d'un état clinique chez un patient.

De préférence, la maladie inflammatoire est une maladie inflammatoire du tube digestif, et le cancer est un cancer du tube digestif (intestin grêle ou colon).

L'enseignement de l'invention permet en effet de connaître les mutations présentant un déséquilibre de liaison avec les maladies inflammatoires du tube digestif et qui sont donc associées à de telles maladies.

L'analyse peut être effectuée par séquence de tout ou partie du gène, ou par d'autres méthodes connues de l'homme du métier. On peut en particulier utiliser des méthodes basées sur la PCR, par exemple la PCR-SSCP qui permet de détecter des mutations ponctuelles.

On peut également effectuer l'analyse par fixation d'une sonde selon l'invention correspondant à l'une des séquences SEQ ID N° 1 ou 3, sur une puce à ADN et l'hybridation sur ces microplaques. Une puce à ADN contenant une séquence selon l'invention est également un des objets de l'invention.

De même, une puce à protéines contenant une séquence d'acides aminés selon l'invention est aussi un objet de l'invention. Une telle puce à protéines permet l'étude des interactions entre les polypeptides selon l'invention et d'autres protéines ou des composés chimiques, et peut ainsi être utile pour le criblage de composés interagissant avec les polypeptides selon l'invention. On peut également utiliser les puces à protéines selon l'invention pour détecter la présence d'anticorps dirigés contre les polypetides selon l'invention dans le sérum de patients. On peut aussi mettre en oeuvre une puce à protéines contenant un anticorps selon l'invention.

L'homme du métier sait également mettre en oeuvre des techniques. permettant l'étude de l'altération de l'expression d'un gène, par exemple par l'étude de l'ARNm (en particulier par Northem Blot ou par des expériences de RT-PCR, avec des sondes ou des amorces selon l'invention), ou de la protéine exprimée, en particulier par Western Blot, en utilisant des anticorps selon l'invention.

Le gène testé est de préférence le gène de séquence SEQ ID N° 1, la maladie inflammatoire pour laquelle on cherche à prédire la susceptibilité étant une maladie du tube digestif, en particulier la maladie de Crohn, ou la rectocolite hémorragique. Si l'on cherche à détecter un cancer, il s'agit de préférence du cancer du colon.

L'invention se rapporte également à des procédés d'obtention d'un allèle du gène IBD1, associé à un phénotype détectable, comprenant les étapes suivantes :
a) obtenir un échantillon d'acide nucléique d'un individu exprimant ledit phénotype détectable ;
b) mettre en contact ledit échantillon d'acide nucléique avec un agent capable de détecter spécifiquement un acide nucléique codant pour la protéine IBD1;
c) isoler ledit acide nucléique codant pour la protéine IBD1.

Un tel procédé peut être suivi d'une étape de séquence de tout ou partie de l'acide nucléique codant pour la protéine IBD1, ce qui permet de prédire la susceptibilité à une maladie inflammatoire ou d'un cancer.

L'agent capable de détecter spécifiquement un acide nucléique codant pour la protéine IBD1 est avantageusement une sonde d'oligonucléotides selon l'invention, qui peut être formée d'ADN, d'ARN, de PNA, modifiés ou non. Les modifications peuvent inclure un marquage radioactif ou fluorescent, ou être dues à des modifications dans les liaisons entre les bases (phosphorothioates, ou méthylphosphonates par exemple). L'homme du métier connaît les protocoles permettant d'isoler une séquence spécifique d'ADN. L'étape b) du procédé ci-dessus décrit peut également être une étape d'amplification telle que décrite précédemment.

L'invention se rapporte également à un procédé de détection et/ou de dosage d'un acide nucléique selon l'invention dans un échantillon biologique, comprenant les étapes suivantes de mise en contact d'une sonde selon l'invention avec un échantillon biologique et de détection et/ou dosage de l'hybride formé entre ledit polynucléotide et l'acide nucléique de l'échantillon biologique.

L'homme du métier sait mettre en oeuvre un tel procédé, et peut en particulier utiliser une trousse de réactifs comprenant :
a) un polynucléotide selon l'invention, utilisé en tant que sonde ;
b) les réactifs nécessaires à la mise en oeuvre d'une réaction d'hybridation entre ladite sonde et l'acide nucléique de l'échantillon biologique ;
c) les réactifs nécessaires à la détection et/ou le dosage de l'hybride formé entre ladite sonde et l'acide nucléique de l'échantillon biologique ;
qui est également un objet de l'invention.

Une telle trousse peut également contenir des contrôles positifs ou négatifs afin d'assurer la qualité des résultats obtenus.

Toutefois, afin de détecter et/ou doser un acide nucléique selon l'invention, l'homme du métier peut également effectuer une étape d'amplification à l'aide d'amorces choisies parmi les séquences selon l'invention.

Enfin, l'invention concerne également les composés choisis parmi un acide nucléique, un polypeptide, un vecteur, une cellule, ou un anticorps selon l'invention, ou les composés obtenus par les procédés de criblage selon l'invention, à titre de médicament, en particulier pour la prévention et/ou le traitement d'une maladie inflammatoire et/ou immune ou d'un cancer, associé à la présence d'au moins une mutation du gène correspondant à SEQ ID N° 1 de préférence une maladie inflammatoire du tube digestif, en particulier la maladie de Crohn ou la rectocolite hémorragique.

Les exemples qui suivent permettent de mieux comprendre les avantages de l'invention et ne doivent pas être considérés comme limitant la portée de l'invention.

### DESCRIPTION DES FIGURES

Figure 1 : tests de liaison génétique non paramétrique pour la maladie de Crohn dans la région péricentromérique du chromosome 16 (d'après Hugot et al., 1996). Analyse de liaison multipoint basé sur l'identité par descendance pour les marqueurs de la région péricentromérique du chromosome 16. Les distances génétiques entre marqueurs ont été estimées grâce au programme CRIMAP. Le lod score (MAPMAKER/SIBS) est indiqué sur la figure de gauche. Deux tests de pseudo vraisemblance ont été développés et rapportés sur la figure de droite. Le premier (Tz) est analogue au test des moyennes. Le deuxième (Tz2) est analogue au test de la proportion des paires d'affectés partageant deux allèles.

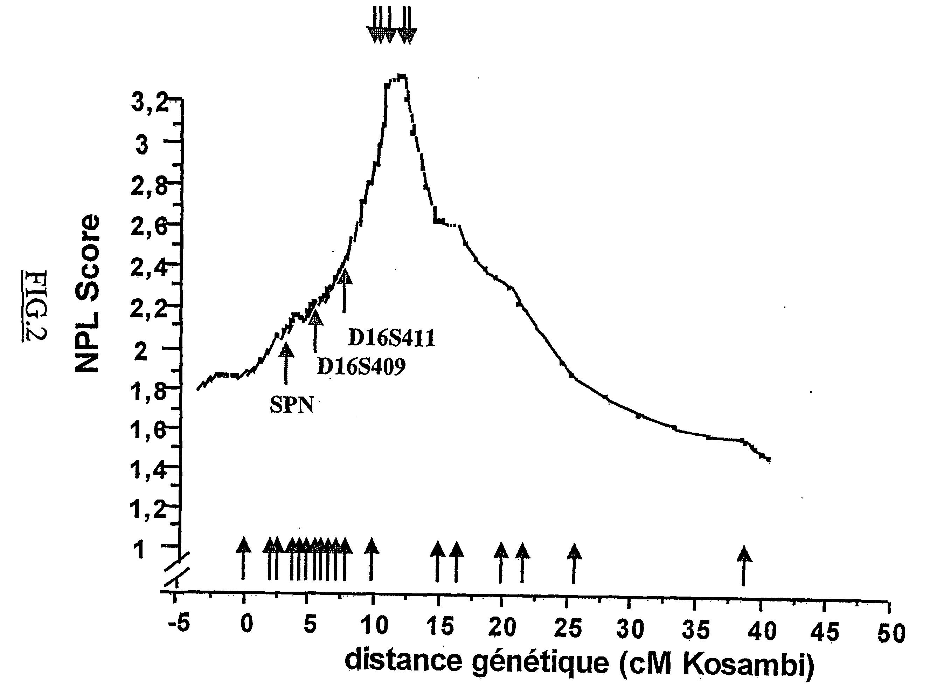

Figure 2 : analyse de liaison génétique multipoint non paramétrique. 78 familles avec plusieurs apparentés atteints de Maladie de Crohn ont été génotypées pour 26 marqueurs de polymorphisme dans la région péricentromérique du chromosome 16. La localisation de chaque marqueur est symbolisée par une flèche. L'ordre des marqueurs et la distance les séparant dérive de l'analyse des données expérimentales avec le logiciel Crimap. Les flèches sous la courbe indiquent les marqueurs SPN, D16S409 et D16S411 utilisés dans la première étude publiée (Hugot et al., 1996).Les flèches situées en haut de la figure correspondent aux marqueurs D16S3136, D16S541, D16S3117, D16S416 et D16S770 localisés au maximum du test de liaison génétique. Les données de typage ont été analysées à l'aide du programme d'analyse multipoint non paramétrique du logiciel Genehunter version 1.3. Le maximum du NPL Score est de 3,33 (p=0,0004).

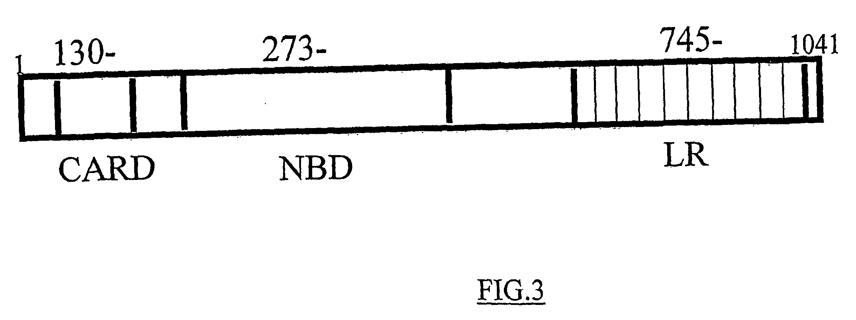

Figure 3 : représentation schématique de la protéine codée par IBD1. La protéine codée par IBD1 est représentée horizontalement. Les différents domaines qui la composent sont indiqués sur la figure avec le numéro de référence des acides aminés correspondant au début et à la fin de chaque domaine. La protéine est constituée d'un domaine CARD, d'un domaine liant les nucléotides (NBD) et de motifs riches en leucines (LRR).

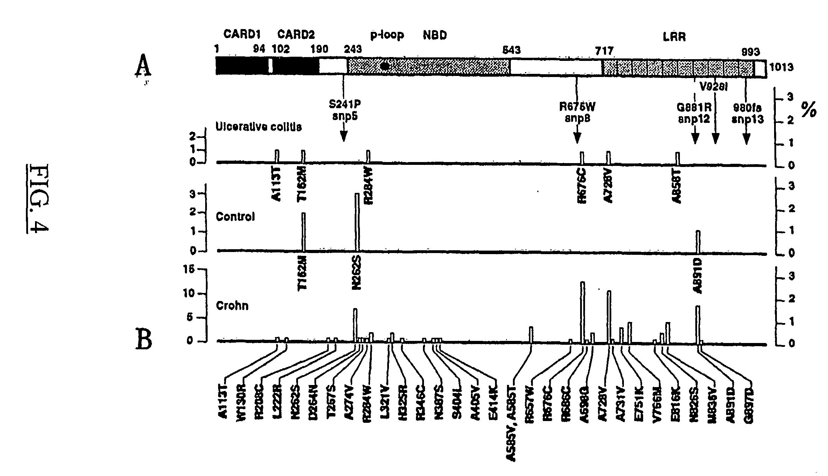

Figure 4 : représentation schématique de la protéine IBD1/NOD2 dans trois variants associés à MC.
   A : Le produit de traduction déduit de la séquence d'ADNc du gène candidat IBD1 est identique à celui de NOD2 (Ogura et al., 2000). Le polypeptide contient 2 domaines CARD (CAspase Recruitment Domains), un domaine de liaison aux nucléotides (NBD) et 10 répétitions de 27 acides aminés, des motifs riches en leucine (LRR). La séquence consensus du site du motif A (boucle P) liant l'ATP/GTP du NBD est indiquée par un cercle noir. Les changements de séquences codés par les trois principaux variants associés à MC sont SNP 8 (R675W), SNP 12 (G881R) et SNP 13 (déplacement de cadre 980). Le déplacement de cadre change un codon leucine en un codon proline à la position 980 qui est immédiatement suivi par un codon stop.
   B : Variants faux sens rares de NOD2 chez 457 patients MC, 159 patients RCH et 103 individus non apparentés, non atteints. Les positions des variants faux sens rares sont indiquées pour les trois groupes. L'échelle à gauche indique le nombre de chaque variant identifié dans les groupes faisant l'objet de recherche et celle à droite mesure la fréquence de la mutation. Les fréquences allèliques du polymorphisme V928I n'étaient pas significativement différentes( 0,92 : 0,08) dans les trois groupes et les génotypes correspondants étaient en équilibre Hardy-Weinberg.

### EXEMPLES

### Exemple 1 : localisation fine de IBD1

La première étape vers l'identification du gène IBD1 a été de réduire la taille de la région génétique d'intérêt, initialement centrée sur le marqueur D16S411 situé entre D16S409 et D16S419 (Hugot et al., 1996 et fig. 1). Un groupe de marqueurs proches (carte génétique à haute résolution) a été utilisé pour mieux préciser la région génétique et a permis de compléter les analyses de liaison génétique et de rechercher un déséquilibre de liaison génétique avec la maladie.

L'étude a porté sur 78 familles comportant au moins 2 apparentés atteints de MC, qui correspondaient à 119 paires d'affectés. Les familles comportant des malades atteints de RCH ont été exclues de l'étude.

Vingt-six marqueurs génétiques de polymorphisme de type microsatellites ont été étudiés. Ces marqueurs formaient ensemble une carte à haute résolution avec une distance moyenne entre marqueurs de l'ordre de 1cM dans la région génétique d'intérêt. Les caractéristiques des marqueurs étudiés sont rapportés sur le tableau 1.

**Tableau 1.**

| Marqueurs polymorphes de type microsatellite utilisés pour la localisation fine de IBD1 | | |
|---|---|---|
| Nom du marqueur de polymorphisme | Distance cumulée (cM) | Amorces PCR |
| D16S3120 | 0 | SEQ ID N° 7 |
| (AFM326vc5) | | SEQ ID N° 8 |
| D16S298 | 2,9 | SEQ ID N°9 |
| (AFMa189wg5) | | SEQ ID N° 10 |
| D16S299 | 3,4 | SEQ ID N° 11 |
| | | SEQ ID N° 12 |
| SPN | 3,9 | SEQ ID N°13 |
| | | SEQ ID N°14 |
| D16S383 | 4,3 | SEQ ID N° 15 |
| | | SEQ ID N° 16 |
| D16S753 | 4,9 | SEQ ID N° 17 |
| (GGAA3G05) | | SEQ ID N° 18 |
| D16S3044 | 5,8 | SEQ ID N° 19 |
| (AFMa222za9) | | SEQ ID N° 20 |
| D16S409 | 5,8 | SEQ ID N° 21 |
| (AFM161xa1) | | SEQ ID N° 22 |
| D16S3105 | 6,1 | SEQ ID N° 23 |
| (AFMb341zc5) | | SEQ ID N° 24 |
| D165261 | 6,8 | SEQ ID N° 25 |
| (MFD24) | | SEQ ID N° 26 |
| D16S540 | 6,9 | SEQ ID N° 27 |
| (GATA7B02) | | SEQ ID N° 28 |
| D16S3080 | 7 | SEQ ID N° 29 |
| (AFMb068zb9) | | SEQ ID N° 30 |
| D16S517 | 7 | SEQ ID N° 31 |
| (AFMa132we9) | | SEQ ID N° 32 |
| D16S411 | 8 | SEQ ID N° 33 |
| (AFM186xa3) | | SEQ ID N° 34 |
| D16S3035 | 10,4 | SEQ ID N° 35 |
| (AFMa189wg5) | | SEQ ID N° 36 |
| D16S3136 | 10,4 | SEQ ID N° 37 |
| (AFMa061xe5) | | SEQ ID N° 38 |
| D16S541 | 11,4 | SEQ ID N°39 |
| (GATA7E02) | | SEQ ID N° 40 |
| D16S3117 | 11,5 | SEQ ID N° 41 |
| (AFM288wb1) | | SEQ ID N° 42 |
| D16S416 | 12,4 | SEQ ID N° 43 |
| (AFM210yg3) | | SEQ ID N° 44 |
| D16S770 | 13,2 | SEQ ID N° 45 |
| (GGAA20G02) | | SEQ ID N° 46 |
| D16S2623 | 15 | SEQ ID N° 47 |
| (GATA81B12) | | SEQ ID N° 48 |
| D16S390 | 16,5 | SEQ ID N° 49 |
| | | SEQ ID N° 50 |
| D16S419 | 20,4 | SEQ ID N° 51 |
| (AFM225zf2) | | SEQ ID N° 52 |
| D16S771 | 21,8 | SEQ ID N° 53 |
| (GGAA23C09) | | SEQ ID N° 54 |
| D16S408 | 25,6 | SEQ ID N°55 |
| (AFM137xf8) | | SEQ ID N° 56 |
| D16S508 | 38,4 | SEQ ID N° 57 |
| (AFPM304xf1) | | SEQ ID N° 58 |

Chaque marqueur est répertorié selon la nomenclature internationale et le plus souvent par le nom proposé par le laboratoire d'origine. Les marqueurs apparaissent selon leur ordre sur le chromosome (de 16p vers 16q). La distance génétique entre les marqueurs (en centiMorgan Kosambi, calculée par le programme Crimap à partir des données expérimentales) est indiquée dans la deuxième colonne. Le premier marqueur polymorphe est pris arbitrairement comme point de référence. Les oligonucléotides ayant servi à la réaction de polymérisation en chaîne (PCR) sont indiqués dans la troisième colonne.

Le génotypage de ces marqueurs microsatellites a reposé sur la technologie des séquenceurs automatiques utilisant des amorces fluorescentes. Brièvement, après amplification, les produits de réaction de polymérisation en chaîne (PCR) fluorescents ont été déposés sur un gel de polyacrylamide sur séquenceur automatique selon les recommandations du constructeur (Perkin Elmer). La taille des allèles pour chaque sujet a été déduite grâce au logiciels Genescan^{R} et Genotyper^{R}. Les données ont ensuite été conservées sur une base informatique intégrée contenant les données généalogiques, phénotypiques et génétiques. Elles ont alors été utilisées pour les analyses de liaison génétique.

Plusieurs contrôles qualité ont été réalisés tout au long de la procédure de génotypage:
- double lecture indépendante des données de génotypage,
- utilisation d'un ADN standard servant de contrôle interne pour chaque migration électrophorétique,
- contrôle de la gamme de taille de chaque allèle observé,
- recherche d'erreurs de transmission mendélienne,
- calcul de la distance génétique entre marqueurs (programme CRIMAP) et comparaison de celle-ci avec les données de la littérature,
- nouveau typage des marqueurs pour lesquels il était observé une recombinaison entre marqueurs proches.

Les données de génotypage ont été analysées par des méthodes de liaison génétique multipoint non paramétrique (Programme GENEHUNTER version 1.3). L'informativité du système de marqueurs était supérieure à 80% pour la région étudiée. Le maximum du test (NPL= 3,33; P = 0,0004) a été obtenu pour les marqueurs D16S541, D16S3117, D16S770 et D16S416 (figure 2).

Les données de typage pour ces 26 marqueurs de polymorphisme ont aussi été analysées à la recherche d'un déséquilibre de transmission. Deux groupes de 108 et 76 familles avec un ou plusieurs malades atteints de MC ont été étudiés. Le test statistique de déséquilibre de transmission a été décrit par Spielman et al. (1993). Il n'a été pris en compte dans ce travail qu'un seul malade par famille et la valeur de p a été corrigée par le nombre d'allèles testés pour chaque marqueur étudié.

Un déséquilibre de transmission a été observé pour les allèles 4 et 5 (taille 205, resp. 207 paires de bases) du marqueur D16S3136 (p=0,05, resp. p=0,01).

Ces résultats suggestifs d'une association entre le marqueur D16S3136 et la MC ont conduit à construire une cartographie physique de la région génétique centrée sur D16S3136 et à établir la séquence d'un segment d'ADN génomique de grande taille (BAC) contenant ce site polymorphe. Il a alors été possible d'identifier et d'analyser un plus grand nombre de marqueurs de polymorphisme dans le voisinage de D16S3136 ainsi que de définir et d'étudier les séquences transcrites présentes dans la région.

### Exemple 2 : cartographie physique de la région IBD1

Un contig de fragments d'ADN génomique, centré sur les marqueurs D16S3136, D16S3117, D16S770 et D16S416, a été généré à partir des banques d'ADN génomique humain de la fondation Jean Dausset/CEPH. Les segments d'ADN chromosomique ont été identifiés à partir de certains marqueurs de polymorphisme utilisés dans la cartographie génétique fine (D16S411, D16S416, D16S541, D16S770, D16S2623, D16S3035, D16S3117 et D16S3136). Pour chaque marqueur, une banque de chromosomes artificiels de bactéries (BAC) a été criblée par PCR à la recherche de clones contenant la séquence du marqueur. Selon que les séquences testées étaient ou non présentes sur les clones de BAC il a été alors possible d'organiser les clones entre eux à l'aide du logiciel Segmap version 3.35.

On a pu établir, pour les BACs, une organisation continue (contig) couvrant la région génétique d'intérêt, selon une méthode connue de l'homme du métier (Rouquier et al., 1994 ; Kim et al., 1996 ; Asakawa et al., 1997). Pour ce faire, les extrémités des BACs identifiés ont été séquencées et ces nouvelles données de séquence ont alors servi à cribler itérativement les banques de BACs. A chaque criblage, le contig de BAC a alors progressé d'un pas jusqu'à l'obtention d'un continuum de clones chevauchants. La taille de chaque BAC participant au contig a été déduite de son profil de migration sur gel d'agarose en champ pulsé.

On a ainsi construit un contig de BAC contenant 101 BACs et s'étendant sur une distance globale de plus de 2,5 Mb avec une redondance moyenne de 5,5 BAC à chaque point du contig. La taille moyenne des BAC est de 136kb.

### Exemple 3 : séquençage du BAC hb87b10

Le BAC de ce contig contenant le marqueur de polymorphisme D16S3136 (appelé hb87b10), dont la taille était de 163761 bp a été séquence selon la méthode dite du "coup de fusil". En bref, l'ADN du BAC a été fragmenté par sonication. Les fragments d'ADN ainsi générés ont été soumis à une électrophorèse en gel d'agarose et ceux dont la taille était supérieure à 1,5 kb ont été élus pour être analysés. Ces fragments ont ensuite été clonés dans le phage m13 lui même introduit dans des bactéries rendues compétentes par électroporation. Après culture, l'ADN des clones a été récupéré et séquencé par des méthodes de séquençage automatique à l'aide d'amorces fluorescentes du vecteur m13 sur séquenceur automatique.

1526 séquences différentes d'une taille moyenne de 600 bp ont été générées, qui ont été organisées entre elles grâce au logiciel Polyphredphrap^{R} aboutissant à un contig de séquence couvrant l'ensemble du BAC. La séquence ainsi générée avait une redondance moyenne de 5,5 équivalents génomiques. Les rares (n=5) intervalles de séquence non représentés dans la banque de clones m13 ont été comblés en générant des amorces de PCR spécifiques, de part et d'autre de ces intervalles, et en analysant le produit de PCR dérivé de l'ADN génomique d'un sujet sain.

Des homologies de séquence avec des séquences disponibles dans les bases de données génétiques publiques (Genbank) ont été recherchées. Aucun gène connu n'a pu être identifié dans cet intervalle de 163 kb. Plusieurs EST ont été positionnés suggérant que des gènes inconnus étaient contenus dans cette séquence. Ces EST issus des bases de données génétiques publiques (Genbank, GDB, Unigene, dbEST) portaient les références suivantes : AI167910, AI011720, Rn24957, Mm30219, hs132289, AA236306, hs87296, AA055131, hs151708, AA417809, AA417810, hs61309, hs116424, HUMGS01037, AA835524, hs105242, SHGC17274, hs146128, hs122983, hs87280 et hs13S201. La recherche d'exons putatifs à l'aide du programme informatique GRAIL a permis d'identifier plusieurs exons potentiels, sites de polyadénylation et séquences promotrices.

### Exemple 4 : études de déséquilibre de transmission

12 marqueurs de polymorphisme bialléliques (SNP) ont été identifiés dans une région s'étendant sur environ 250 kb et centrée sur le BAC hb87b10. Ces polymorphismes ont été générés par analyse de la séquence d'une dizaine de malades indépendants atteints de MC. Le séquençage a été le plus souvent réalisé au niveau d'EST connus et positionnés sur le BAC ou à son voisinage. Des exons putatifs, prédits par le programme informatique GRAIL ont aussi été analysés. Les caractéristiques des marqueurs polymorphes ainsi identifiés sont rapportées sur le tableau 2.

**Tableau 2.**

| Caractéristiques de marqueurs de polymorphisme bialléliques étudiés dans la région de IBD1 | | | | | |
|---|---|---|---|---|---|
| I | II | III | IV | V | VI |
| 1 | KIAA0849ex9 | PCR-AS | | SEQ ID N° 88 à 90 | 116 |
| 2 | hb27G11F | PCR-RFLP | *Bsr*I | SEQ ID N° 86, 87 | 185 |
| | | | | | 116 |
| | | | | | 69 |
| 3 | Ctg22Ex1 | PCR-RFLP | *Rsa*I | SEQ ID N° 84, 85 | 381 |
| | | | | | 313 |
| | | | | | 69 |
| 4 | SNP1 | PCR-AS | | SEQ ID N° 81 à 83 | 410 |
| 5 | ctg2931-3ac/ola | LO | | SEQ ID N° 78 à 80 | 51 |
| | | | | | 49 |
| 6 | ctg2931-Sag/ola | LO | | SEQ ID N° 75 à 77 | 44 |
| | | | | | 42 |
| 7 | SNP3-2931 | PCR-AS | | SEQ ID N° 72 à 74 | 245 |
| 8 | Ctg25Ex1 | PCR-RFLP | *Bste*II | SEQ ID N° 70, 71 | 207 |
| | | | | | 122 |
| | | | | | 85 |
| 9 | CTG35 ExA | PCR-AS | | SEQ ID N° 67 à 69 | 333 |
| 10 | ctg35 ExC | PCR-AS | | SEQ ID N° 64 à 66 | 198 |
| 11 | D16S3136 | | | SEQ ID N° 37, 38 | |
| 12 | hbl33DIf | PCR-RFLP | *Taq*I | SEQ ID N° 62, 63 | 369 |
| | | | | | 295 |
| | | | | | 74 |
| 13 | D16S3035 | | | SEQ ID N° 35, 36 | |
| 14 | ADCY7 int7 | PCR-AS | | SEQ ID N° 59 à 61 | 140 |
| PCR-AS : PCR-allèle spécifique ; LO : Ligature d'oligonucléotides | | | | | |

Les 12 marqueurs de polymorphisme bialléliques nouvellement décrits dans ce travail sont répertoriés dans ce tableau. Pour chacun d'eux sont indiqués :
- le locus (colonne I)
- le nom (colonne II)
- la technique de génotypage utilisée (colonne III)
- l'enzyme de restriction éventuellement utilisée (colonne IV)
- les amorces oligonucléotidiques utilisées pour la réaction de polymérisation en chaîne ou pour la ligature (colonne V)
- la taille des produits attendus lors du typage (colonne VI)

199 familles comportant 1 ou plusieurs malades atteints de MC ont été typées pour ces 12 marqueurs de polymorphisme ainsi que pour les marqueurs D16S3035 et D16S3136 localisés sur le BAC hb87b10. Les familles comportant des malades atteints de RCH n'ont pas été prises en compte. Les méthodes de typage des polymorphismes étudiés ont été variables en fonction du type de polymorphisme faisant appel à :
- la technique de PCR-RFLP (amplification suivie de digestion enzymatique du produit de PCR) quand le polymorphisme était situé sur un site de restriction enzymatique.
- PCR avec amorces spécifiques du site polymorphe : amplification différentielle des deux allèles en utilisant des amorces spécifiques de chaque allèle.
- Test de ligation d'oligonucléotides : ligation différentielle utilisant des oligonucléotides spécifiques de chaque allèle, suivie d'électrophorèse en gel de polyacrylamide.

Les données de typage ont ensuite été analysées selon un test de déséquilibre de transmission (programme informatique TDT du logiciel GENEHUNTER version 2). Pour les familles comportant plusieurs apparentés atteints, un seul malade a été pris en compte pour l'analyse. En effet, la prise en compte de plusieurs malades apparentés pose le problème de non indépendance des données dans les calculs statistiques et peut induire une inflation de la valeur du test. Le malade servant à l'analyse a été tiré au sort au sein de chaque famille par une procédure automatique de randomisation. Compte tenu de cette randomisation, la valeur du test statistique obtenu ne représentait qu'un seul échantillon possible issu du groupe de familles étudiées. Afin de ne pas limiter l'analyse à ce seul échantillon possible et pour mieux appréhender la robustesse des résultats obtenus, pour chaque test, une centaine d'échantillons aléatoires ont ainsi été générés et analysés.

Les marqueurs ont été étudiés séparément puis groupés selon leur ordre sur le segment chromosomique (KIAA0849ex9 (locus 1), hb27G11F (locus 2), Ctg22Ex1 (locus 3), SNP1 (locus 4), ctg2931-3ac/ola (locus 5), ctg2931-5ag/ola (locus 6), SNP3-2931 (locus 7), Ctg25Ex1 (locus 8), CTG35ExA (locus 9), ctg35ExC (locus 10), d16s3136 (locus 11), hb133D1f (locus 12), D16S3035 (locus 13), ADCY7int7 (locus 14)) (tableau 2). Les haplotypes comportant 2, 3 et 4 marqueurs consécutifs ont ainsi été analysés en utilisant toujours la même stratégie (100 échantillons aléatoires en prenant pour chaque famille un seul individu atteint).

Pour chaque échantillon testé, il n'a été pris en compte que les génotypes (ou haplotypes) portés par au moins 10 chromosomes parentaux. En moyenne 250 tests différents ont ainsi été réalisés pour chaque échantillon. Il a alors été possible de déduire le nombre de tests attendus positifs pour chaque seuil de signification et de comparer cette distribution à la distribution observée. Pour les sujets sains, la distribution des tests n'est pas différente de celle attendue selon le hasard (χ² = 2,85, ddl=4, p=0,58). Pour les sujets malades, au contraire, il existe un excès de tests positifs témoignant de l'existence d'un déséquilibre de transmission dans la région étudiée.

Les résultats des tests de déséquilibre de transmission pour chaque marqueur de polymorphisme pris isolément et pour les haplotypes montrant les plus forts déséquilibres de transmission ont montré que les marqueurs suivants sont en déséquilibre de liaison avec la maladie: Ctg22Ex1 (locus 3), SNP1 (locus 4), ctg2931-5ag/ola (locus 6), SNP3-2931 (locus 7), Ctg25Ex1 (locus 8) et ctg35ExC (locus 10). Ces marqueurs s'étendent sur une région d'environ 50kb (positions 74736 à 124285 sur la séquence de hb87b10).

Les haplotypes les plus fortement associés avec la maladie de Crohn s'étendent eux aussi sur cette région. Ainsi, pour la majorité des échantillons aléatoires, le test de transmission était positif (p < 0,01) pour des haplotypes combinant les marqueurs suivants :
- locus 5-6, locus 6-7, locus 7-8, locus 8-9, locus 9-10, locus10-11
- locus 5-6-7, locus 6-7-8, locus 7-8-9, locus 8-9-10, locus 9-10-11
- locus 5-6-7-8; locus 6-7-8-9, locus 7-8-9-10,

L'haplotype de susceptibilité le plus à risque est défini par les locus 7 à 10. Il s'agit de l'haplotype 1-2-1-2 (tableau 2).

Les marqueurs testés sont, comme attendu, le plus souvent en déséquilibre de liaison entre eux.

Plus récemment, un nouveau test, le Pedigree Disequilibrium Test (PDT), publié en juillet 2000 (Martin et al., 2000) a été utilisé pour mieux appréhender la signification des résultats obtenus avec le programme informatique TDT. Cette nouvelle statistique permet en effet d'utiliser l'ensemble de l'information disponible dans une famille, tant à partir des sujets malades qu'à partir des sujets sains et de pondérer l'importance de chaque apparenté en une statistique globale pour chaque famille. Les valeurs de p correspondant aux tests PDT et obtenues pour un groupe élargi de 235 familles avec un ou plusieurs apparentés atteints de la maladie de Crohn sont rapportées dans le Tableau 3. Cette nouvelle analyse confirme que la région du BAC hb87b 10 est bien associée avec la maladie de Crohn.

**Tableau 3.**

| Résultat des tests PDT réalisés sur 235 familles atteintes de la maladie de Crohn (NS : non significatif) | |
|---|---|
| **LOCUS** | **VALEUR p DU TEST PDT** |
| KIAA0849ex9 | NS |
| hb27g11f | 0,05 |
| ctg22ex1 | 0,01 |
| SNP1 | 0,001 |
| ctg2931-3ac/ola | NS |
| ctg2931-5ag/ola | 0,0001 |
| SNP3-2931 | 0,0001 |
| ctg25ex1 | 0,0006 |
| ctg35exA | NS |
| ctg35exC | 0,00002 |
| D16S3136 | NS |
| hb133d1f | NS |
| D16S3035 | NS |

### Exemple 5 : Identification du gène IBD1

Les groupements d'EST (références Unigene : Hs 135201, Hs87280, Hs122983, Hs146128, Hs105242, Hs116424, Hs61309, Hs151708, Hs 87296 et Hs132289) publiés et présents sur le BAC hb87b10 ont été étudiés à la recherche d'une séquence d'ADN complémentaire (ADNc) plus complète. Pour IBD1prox, les clones disponibles dans les banques publiques ont été séquencés et les séquences organisées entre elles. Pour IBD1, une banque d'ADN complémentaire de sang périphérique (Stratagene human blood cDNA lambda zapexpress ref 938202) a été criblée par les produits de PCR générés à partir des EST connus selon les modalités proposées par le fabriquant. La séquence des ADNc ainsi identifiés a ensuite servi à un nouveau criblage de la banque d'ADNc et ainsi de suite jusqu'à l'obtention de l'ADNc présenté.

L'EST hs135201 (UniGene) a permis d'identifier un ADNc ne figurant pas sur les bases de données génétiques disponibles (Genbank) Il correspond donc à un nouveau gène humain. La comparaison de la séquence du cDNA et de l'ADN génomique a montré que ce gène est constitué de 11 exons et 10 introns. Un exon supplémentaire, en position 5' par rapport au cDNA identifié est prédit par l'analyse de la séquence avec le logiciel Grail. Ces exons sont très homologues avec les premiers exons du gène CARD4/NOD1. Considérant l'ensemble des exons identifiées et l'exon putatif supplémentaire, ce nouveau gène apparaît avoir une structure génomique très proche de celle de CARD4/NOD1. Par ailleurs, en amont du premier exon putatif figure un site d'initiation de la transcription. Pour l'ensemble de ces raisons, l'exon putatif a été considéré comme participant à ce nouveau gène. L'ADNc reporté en annexe (SEQ ID N° 1) comporte donc l'ensemble de la séquence identifiée plus la séquence prédite par la modélisation informatique, l'ADN complémentaire débutant arbitrairement au premier codon ATG de la séquence codante prédite. Sur cette base, le gène comporterait donc 12 exons et 11 introns. La structure intron-exon du gène est rapportée sur la SEQ ID N° 3.

La séquence protéique déduite de la séquence nucléotidique, comporte 1041 acides aminés (SEQ ID N° 2). Cette séquence n'a pas non plus été retrouvée sur les bases de données biologiques (Genpept, pir, swissprot).

Or, plus récemment, l'exon putatif ci-dessus décrit n'a pas pu être confirmé. Le gène IBD1 ne comporte donc effectivement que 11 exons et 10 introns et code pour une protéine de 1013 acides aminés (c'est-à-dire 28 acides aminés de moins que déterminé initialement).

L'étude de la séquence protéique déduite montre que ce gène contient trois domaines fonctionnels différents (figure 3) :
- Un domaine CARD (Caspase Recruitment Domain) connu pour être impliqué dans l'interaction entre protéines régulatrices de l'apoptose et de l'activation de la voie NFkappa B. Le domaine CARD permet de classer cette nouvelle protéine dans la famille des protéines CARD dont les membres les plus anciens sont CED 4, APAF1 et RICK.
- Un domaine NBD (Nucléotide Binding Domaine) comportant un site de reconnaissance de l'ATP et un site de liaison du Magnésium. La protéine doit donc avoir une activité kinase très probable.
- Un domaine LRR (Leucine Rich Domain) supposé participer à l'interaction entre protéines par analogie avec d'autres domaines protéiques décrits.

Par ailleurs, le domaine LRR de la protéine permet d'affilier la protéine à une famille de protéines impliquées dans la signalisation intracellulaire et présentes tant chez les plantes que chez les animaux.

La comparaison de ce nouveau gène avec les gènes précédemment identifiés et disponibles dans les bases de données publiques montre que celui-ci est très homologue avec CARD4/NOD1 (Bertin et al., 1999 ; Inohara et al., 1999). Cette homologie porte sur la séquence de l'ADN complémentaire, la structure intron-exon du gène et la séquence protéique. L'identité de séquence des 2 ADN complémentaires est de 58%. Une similitude est également observée au niveau de la structure introns-exons. L'homologie de séquence au niveau protéique est de l'ordre de 40%.

La similitude entre ce nouveau gène et CARD4/NOD1 suggère que, comme CARD4/NOD1, la protéine IBD1 est impliquée dans la régulation de l'apoptose et de l'activation de NF-kappa B (Bertin et al., 1999 ; Inohara et al., 1999). La régulation de l'apoptose cellulaire et l'activation de NF-kappa B sont des voies de signalisation intracellulaire essentielles dans les réactions immunitaires. En effet, ces voies de transduction du signal sont les voies effectrices des protéines de la famille du récepteur du TNF (Tumor Necrosis Factor) impliquées dans les interactions cellule-cellule et la réponse cellulaire aux différents médiateurs de l'inflammation (cytokines). Le nouveau gène apparaît donc comme potentiellement important à la réaction inflammatoire, de façon générale.

Plusieurs faisceaux de preuves viennent à l'appui de la dérégulation de NF-kB induit par des bactéries dans la maladie de Crohn. Tout d'abord, la susceptibilité à IBD spontanée chez les souris a été associée à des mutations dans Tlr4, une molécule connue pour se lier aux LPS par l'intermédiaire de son domaine LRR (Poltorak et al., 1998 et Sundberg et al., 1994) et pour être un membre des activateurs de la famille de NF-kB. Deuxièmement, la thérapie antibiotique cause une amélioration provisoire chez les patients atteints de MC accréditant l'hypothèse que les bactéries entériques peuvent jouer un rôle étiologique dans la maladie de Crohn (McKay, 1999). Troisièmement, NF-kB joue un rôle pivot dans les maladies inflammatoires de l'intestin et est activé dans les cellules mononucléées de la lamina propria dans la maladie de Crohn (Schreiber et al., 1998). Quatrièmement, le traitement de la maladie de Crohn est basée sur l'utilisation de la sulfasalazine et des glucocorticoïdes, tous deux connus comme étant des inhibiteurs de NF-kB (Auphan et al., 1995 et Wahl et al., 1998)

Encore plus récemment, il a été montré que le gène candidat IBD1 code pour une protéine très similaire à NOD2, un membre de la superfamille CED4/APAF1 (Ogura et al., 2000). Les séquences nucléotidiques et protéiques de IBD1 et NOD2 ne divergent en réalité que pour une petite portion toute initiale des 2 séquences rapportées. Les expressions tissulaires de Nod2 et IBD1 sont de plus superposables. Ces deux gènes (protéines) peuvent donc être considéré(e)s comme identiques. Il a été démontré que le domaine LRR de Nod2 a une activité de liaison pour les lipopolysaccharides bactériens (LPS) (Inohara et al., 2000) et que sa délétion stimule la voie de NFkB. Ce résultat confirme les données de l'invention.

L'expression tissulaire de IBD1 a été ensuite étudiée par la technique du Northern Blot. Un transcrit de 4.5 kb est visible dans la plupart des tissus humains. La taille du transcrit est conforme avec la taille prédite par l'ADNc. Le transcrit de 4.5 kb semble en très faible abondance dans l'intestin grêle et le colon. Il est par contre très fortement exprimé dans les globules blancs. Ceci est en accord avec des données cliniques sur les transplantations qui suggèrent que la maladie de Crohn est potentiellement une maladie liée aux cellules immunitaires circulantes. En effet, la transplantation intestinale n'empêche pas la récidive sur le greffon dans la maladie de Crohn tandis que la transplantation de moelle osseuse semble avoir un effet bénéfique sur l'évolution de la maladie.

Certaines données font également penser à un épissage alternatif, qui pourrait s'avérer un élément important dans la possibilité de générer des mutants qui pourraient jouer un rôle dans le développement de maladies inflammatoires.

Le promoteur du gène IBD1 n'est actuellement pas identifié avec précision. Il est cependant raisonnable de penser, par analogie avec un très grand nombre de gènes que celui-ci réside, au moins pour partie, immédiatement en amont du gène, dans la portion 5' de celui-ci. Cette région génétique contient des séquences transcrites comme en témoigne la présence d'EST (HUMGS01037, AA835524, hs.105242, SHGC17274, hs.146128, hs.122983, hs.87280). Les clones ATCC contenant ces séquences ont été séquencés et analysés dans le laboratoire, permettant de mettre en évidence une organisation en exons et en introns avec d'éventuels épissages alternatifs. Ces données suggèrent l'existence d'un autre gène (nommé IBD1prox en raison de sa proximité d'IBD1). La séquence partielle de l'ADN complémentaire de IBD1prox est rapportée (SEQ ID N° 4) de même que sa structure intron-exon sur la SEQ ID N° 6.

La traduction des ADNc correspondant à IBD1prox aboutit à une protéine contenant une homéobox. L'analyse de plusieurs ADNc du gène suggère cependant l'existence d'épissages alternatifs. IBD1prox, selon un des épissages alternatifs possibles correspond à l'EST anonyme HUMGS01037 dont l'ARN est exprimé de manière plus importante dans les lignées leucocytaires différenciées que dans les lignées non différenciées.

Ainsi, il est possible que ce gène puisse avoir un rôle dans l'inflammation et la différentiation cellulaire. Il peut donc lui aussi être considéré comme un bon candidat pour la susceptibilité aux MICI. L'association entre MC et le polymorphisme ctg35 ExC localisé sur la séquence codante de IBD1prox renforce cette hypothèse même si ce polymorphisme n'entraîne pas de variation de séquence au niveau protéique.

Enfin, plus récemment, l'existence d'une liaison génétique dans les familles atteintes de la maladie de Crohn et ne comportant pas de mutation du gène IBD1 suggère elle aussi que IBD1 prox a un rôle additionnel à IBD1 dans la prédisposition génétique à la maladie.

La relation fonctionnelle entre IBD1 et IBD1prox n'est actuellement pas établie. Toutefois, la forte proximité entre les deux gènes pourrait refléter une interaction entre ceux-ci. Dans ce cas, la localisation « tête -bêche » de ces gènes suggère qu'ils puissent avoir des modes de régulation communs ou interdépendants.

### Exemple 6: identifications de mutations du gène IBD1 dans les maladies inflammatoires

Afin de confirmer le rôle de IBD1 dans les maladies inflammatoires, la séquence codante et les jonctions intron-exon du gène ont été séquencées de l'exon 2 à l'exon 12 inclus chez 70 sujets indépendants, à savoir : 50 malades atteints de MC, 10 malades atteints de RCH, 1 malade atteint de syndrome de Blau et 9 témoins sains. Les malades étudiés étaient pour la plupart des formes familiales de la maladie et étaient souvent porteurs de l'haplotype de susceptibilité défini par les études de déséquilibre de transmission. Les témoins sains étaient d'origine caucasienne.

24 variants de séquence ont ainsi pu être identifiés sur ce groupe de 70 personnes non apparentées(tableau 3).

La nomenclature des mutations rapportées fait référence à la séquence initiale de la protéine comportant 1041 acides aminés. La nomenclature plus récemment proposée est aisément déduite en retirant 28 acides aminés à la séquence initiale, et correspond donc à une protéine comprenant 1013 acides aminés (cf exemple 5).

**Tableau 4.**

| Mutations observées dans le gène IBD1 | | | | | |
|---|---|---|---|---|---|
| Exon | Variant nucléotidique | Variant protéique | Maladie de Crohn | Rectocolite hémorragique | Témoins sains |
| 1 | non testé | | | | |
| 2 | G417A | silencieux | | | |
| 2 | C537G | silencieux | | | |
| 3 | aucun | | | | |
| 4 | T805C | S269P | 48/100 | 6/20 | 3/18 |
| 4 | A869G | N290S | 0 | 0 | 1/18 |
| 4 | C905T | A302V | 1/100 | 0 | 0 |
| 4 | C1283T | P428L | 1/100 | 0 | 0 |
| 4 | C1284A | silencieux | | | |
| 4 | C1287T | silencieux | | | |
| 4 | T1380C | silencieux | | | |
| 4 | T1764G | silencieux | | | |
| 4 | G1837A | A613T | 1/100 | 0 | 0 |
| 4 | C2107T | R703W | 10/10 | 1/20 | 1/18 |
| 4 | C2110T | R704C | 4/10 | 1/20 | 0 |
| 5 | G2365A | R792Q | 1/100 | 0 | 0 |
| 5 | G2370A | V794M | 0 | 1/20 | 0 |
| 5 | G2530A | E844K | 1/10 | 0 | 0 |
| 6 | A2558G | N853S | 1/100 | 0 | 0 |
| 6 | A2590G | M864V | 1/100 | 0 | 0 |
| 7 | aucun | | | | |
| 8 | G2725C | G909R | 7/100 | 0 | 0 |
| 8 | C2756A | A919D | 1/100 | 0 | 0 |
| 9 | G2866A | V9561 | 2/100 | 1/20 | 3/18 |
| 10 | C2928T | silencieux | | | |
| 11 | 3022insC | stop | 20/100 | 0 | 0 |
| 12 | aucun | | | | |

Les mutations autres que silencieuses observées dans chaque exon sont rapportées. Elles sont indiquées par la variation de la chaîne peptidique. Pour chaque mutation et pour chaque phénotype étudié, il est indiqué le nombre de fois où la mutation est observé, rapporté au nombre de chromosomes testés.

Aucun variant de séquence fonctionnel n'a été identifié dans les exons 1 à 3 (correspondants au domaine CARD de la protéine). Les exons 7 et 12 n'ont pas non plus montré de variation de séquence. Certains variants correspondaient à des polymorphismes déjà identifiés et typés pour les études de déséquilibre de transmission, à savoir :
- Snp3-2931 : variant nucléotidique T805C, variant protéique S269P
- ctg2931-5ag/ola : variant nucléotidique T1380C (silencieux)
- ctg2931-3ac/ola : variant nucléotidique T1764G (silencieux)
- SNP1 : variant nucléotidique C2107T, variant protéique R703W

Plusieurs variations de séquence étaient silencieuses (G417A, C537G, C1284A, C1287T, T1380C, T1764G, C2928T) et n'entraînaient pas de modification de la séquence protéique. Elles n'ont pas été étudiées davantage ici.

Pour les 16 variations de séquence non silencieuses, il a été observé des variants de séquence protéique chez 43/50 MC contre 5/9 témoins sains et 6/10 RCH. L'existence d'une ou plusieurs variation(s) de séquence apparaissait associée au phénotype MC. Il existait souvent plusieurs variations de séquence chez un même individu atteint de MC suggérant un effet parfois récessif du gène pour la MC. A l'inverse, aucun homozygote ou hétérozygote composite n'était observé parmi les patients atteints de RCH ou parmi les témoins sains.

Certains variants non silencieux étaient présents à la fois chez les malades atteints de RCH ou de MC et chez les sujets sains. Il s'agissait des variants S269P, N290S, R703W et V956I situés dans les exons 2, 4 et 9. Un complément d'information semble donc nécessaire avant de retenir un éventuel rôle fonctionnel à ces variants de séquence.

V956I est une variation de séquence conservative (acides aminés aliphatiques).

Le variant de séquence S269P correspond à une variation de classe d'acide aminé (hydroxylé en immunoacide) au début du domaine liant les nucléotides. Il en déséquilibre de transmission avec la MC. Il s'agit en effet du polymorphisme Snp3 (Cf. supra).

R703W aboutit à une modification de la classe de l'acide aminé (aromatique au lieu de basique). Cette modification survient dans la région intermédiaire entre les domaines NBD et LRR, région conservée entre IBD1 et CARD4/NOD1. Un rôle fonctionnel peut donc être suspecté pour ce polymorphisme. Cette variation de séquence (correspondant au site polymorphe Snp1) est plus souvent transmise au malades atteints de MC que ne le veut le hasard (Cf supra) confirmant que ce polymorphisme est associé à la MC. Il est possible que la présence de ce mutant chez les sujets sains témoigne d'une pénétrance incomplète de la mutation comme cela est attendu pour les maladies génétiques complexes telles que les maladies inflammatoires chroniques de l'intestin.

Le variant R704C, situé immédiatement à coté de R703W a pu être identifié à la fois dans la MC et dans la RCH. II correspond lui aussi à une variation non conservative de la protéine (acide aminé soufré au lieu de basique) sur la même région protéique, suggérant un effet fonctionnel aussi important pour R704C que pour R703W.

D'autres variations de séquence sont spécifiques de la MC de la RCH ou du syndrome de Blau.

Certaines variations de séquence sont au contraire rares, présentes chez un ou quelques malades (A613T, R704C, E844K, N853S, M864V, A919D). Il s'agit toujours de variations entraînant des modifications non conservatives de la protéine dans des domaines leucine riches, à des positions importantes au sein de ces domaines. Ces différents éléments suggèrent que ces variations ont un rôle fonctionnel.

Deux variations de séquence (G909R, L1008P*) sont retrouvées chez un assez grand nombre de maladies de Crohn (respectivement 7/50 et 16/50) alors qu'elles ne sont pas détectées chez les témoins ou chez les malades atteints de RCH.

La délétion/insertion d'une guanosine au niveau du codon 1008 aboutit à une transformation de la troisième leucine de l'hélice alpha du dernier LRR en proline suivie d'un codon STOP (L1008P*). Cette variation de séquence entraîne donc une modification importante de la protéine : réduction de taille de la protéine (protéine possédant un domaine LRR tronqué) et altération d'un acide aminé très conservé (Leucine). Cette modification de séquence est associée à la MC comme en témoigne une étude de déséquilibre de transmission dans 16 familles porteuses de la mutation (P=0,008).

La mutation G909R survient sur le dernier acide aminé du sixième motif LRR. Il remplace un acide aminé aliphatique en acide aminé basique. Cette variation est potentiellement importante compte tenu du caractère habituellement neutre ou polaire des acides aminés en position terminale des motifs leucine riche (tant pour IBD1 que pour NOD1/CARD4) et du caractère conservé de cet acide aminé sur les protéines IBD1 et NOD1/CARD4.

Dans le syndrome de Blau, les malades (n=2) de la famille étudiée étaient porteurs d'une variation de séquence spécifique (L470F), localisée dans l'exon 4 et correspondant au domaine NBD de la protéine. Dans cette série, ce variant de séquence était spécifique du syndrome de Blau.

Dans la RCH, plusieurs variants de séquence non retrouvés chez les sujets sains ont aussi été identifiés. La proportion de malades porteurs d'une mutation était plus modeste que pour la MC, comme attendu compte tenu de la liaison moins fortement établie entre IBD1 et RCH et du caractère supposé moins génétique de cette dernière maladie. Des variations de séquence étaient communes à la MC et à la RCH (R703W, R704C). D'autres au contraires apparaissaient spécifiques de la RCH (V794M). Cette observation permet de confirmer que MC et RCH sont des maladies partageant au moins en partie la même prédisposition génétique. Elle pose les bases d'une classification nosologique des MICI.

L'étude des variants de séquence du gène IBD1 a donc permis d'identifier plusieurs variants ayant un effet fonctionnel très probable (ex : protéine tronquée) et associés à la maladie de Crohn, à la RCH et au syndrome de Blau.

Le promoteur du gène n'est actuellement pas déterminé. Selon toute vraisemblance cependant, celui-ci est probablement situé dans la région 5' en amont du gène. Selon cette hypothèse, les variants de séquence observés dans cette région peuvent avoir un effet fonctionnel. Ceci pourrait expliquer la très forte association entre MC et certains locus polymorphes tels que ctg35 ExC ou Ctg25Ex1.

L'invention fournit ainsi la première description de mutations dans la famille des gènes contenant un domaine CARD chez l'homme. La fréquence de ces mutations dans des maladies inflammatoires variées montre que le gène IBD1 a un rôle essentiel dans le processus inflammatoire normal et pathologique. Cette invention fournit de nouvelles voies de compréhension et de recherche dans le domaine de la physiopathologie des processus inflammatoires normaux et pathologiques. Elle permet de ce fait d'envisager le développement de nouvelles molécules pharmaceutiques régulant les voies effectrices contrôlées par IBD1 et utiles dans le traitement des maladies inflammatoires et la régulation du processus inflammatoire en général.

### Exemple 7: bases d'un diagnostic biologique de susceptibilité à la maladie de Crohn

Plus récemment, 457 patients indépendants atteints de la maladie de Crohn, 159 patients indépendants atteints de rectocolite hémorragique et 103 témoins sains ont été étudiés à la recherche de mutations. Ce travail a permis de confirmer les mutations précédemment rapportées et d'identifier des mutations supplémentaires rapportées sur la figure 4. Les mutations principales ont ensuite été génotypées dans 235 familles atteintes de la maladie de Crohn. Ce travail plus récent est exposé en utilisant comme référence la séquence protéique plus courte (1013 acides aminés, voir exemple 5) mais la nomenclature antérieure des mutations est aisément déduite à partir de cette dernière en ajoutant 28 au chiffre indiquant la position des acides aminés.

Parmi les 5 mutations les plus fréquences, la mutation conservative V928I (anciennement V956I) n'est pas significativement associée à l'une ou l'autre des maladies inflammatoires de l'intestin et ne semble donc pas avoir de rôle important dans la maladie.

La mutation S241P (anciennement S269P) est en déséquilibre de liaison avec les autres mutations principales et ne semble pas jouer par elle-même un rôle important dans la susceptibilité aux maladies inflammatoires de l'intestin (données non montrées).

A l'inverse, les 3 autres mutations R675W (anciennement R703W), G881R (anciennement G909R) et 980fs (anciennement L1008P*) sont significativement associées à la maladie de Crohn mais pas à la rectocolite hémorragique (cf infra). La localisation dans le LRR ou à sa proximité immédiate des 3 mutations fréquentes plaide très fortement pour un mécanisme fonctionnel impliquant ce domaine protéique, probablement par un défaut de régulation négative de NFkB par la protéine mutée. Les autres mutations sont plus rares (figure 4). Ces mutations cumulées sont présentes chez 17% des sujets atteints de la maladie de Crohn contre respectivement 4 % et 5 % les sujets sains ou atteints de rectocolite hémorragique. Un grand nombre des mutations rares sont aussi localisées dans le LRR.

Les études intrafamiliales des trois polymorphismes les plus fréquents dans la maladie de Crohn montrent qu'ils sont tous trois associés à la maladie (tableau 5). Comme attendu, pour une mutation supposée très délétère, le polymorphisme le plus fortement associé est la mutation tronquante. Ces trois polymorphismes sont associés de manière indépendante à la maladie de Crohn puisqu'il n'a pas été possible d'identifier sur 235 familles des chromosomes porteurs de plus d'une de ces trois mutations. Le caractère indépendant de ces associations renforce considérablement l'hypothèse que le gène IBD1 est bien impliqué dans la prédisposition génétique à la maladie de Crohn.

**Tableau 5 :**

| étude des 3 polymorphismes fréquents de IBD1 dans 235 familles atteintes de la maladie de Crohn | |
|---|---|
| **MUTATION** | **VALEUR p DU TEST PDT** |
| R675W | 0,001 |
| G881R | 0,003 |
| 980fs | 0,000006 |

Les études de cas-témoin confiment cette association (tableau 6). Ils montrent que les mutations les plus fréquentes dans la maladie de Crohn ne sont pas fréquentes dans la rectocolite hémorragique.

**Tableau 6 :**

| étude de cas-témoin des 3 polymorphismes fréquents de IBD1 dans les maladies inflammatoires de l'intestin | | | | | |
|---|---|---|---|---|---|
| **MUTATION** | **NB DE CHROMOSOMES ETUDIES** | **FREQUENCE DE L'ALLELE A RISQUE R675W** | **FREQUENCE DE L'ALLELE A RISQUE G881R** | **FREQUENCE DE L'ALLELE A RISQUE 980fs** | **TOTAL ALLELES A RISQUE** |
| Témoins sains | 206 | 0,04 | 0,01 | 0,02 | 0,07 |
| Rectocolite H. | 318 | 0,03 | 0,00 | 0,01 | 0,05 |
| M Crohn | 936 | 0,11 | 0,06 | 0,12 | 0,29 |

L'étude de l'effet dose de ces mutations montre que les sujets porteurs d'une mutation à l'état homozygote ou hétérozygote composite présentent un bien plu grand risque de développer la maladie que les sujets non porteurs ou hétérozygotes pour ces mutations (tableau 7).

**Tableau 7 :**

| risque relatif et absolu de la maladie de Crohn attribuable en fonction du génotype de IBD1 | | | | |
|---|---|---|---|---|
| Dans la population générale, un risque de la maladie de Crohn de 0,001 a été pris comme référence et les mutations ont été supposées en équilibre de Hardy-Weinberg. | | | | |

| **DISTRIBUTION** | **GENOTYPE** | | | |
|---|---|---|---|---|
| | **AUCUN VARIANT** | **SIMPLE HETEROZYGOTE** | **HOMOZYGOTE** | **HETEROZYGOTE COMPOSITE** |
| Sains | 88 | 15 | 0 | 0 |
| Rectocolite H | 145 | 13 | 1 | 0 |
| M Crohn | 267 | 133 | 28 | 40 |
| Risque attribuable de MC: | | | | |
| Risque relatif | 1 | 3 | 38 | 44 |
| Risque absolu | 0,0007 | 0,002 | 0,03 | 0,03 |

Les travaux cités ci-dessus confirment les données préliminaires antérieures et apportent les bases détaillées d'un diagnostic biologique de la maladie de Crohn par l'étude des variants de IBD1. En effet, ce travail :
1) définit les mutations dont la fréquence est supérieure à 0,001 dans une population caucasienne mélangée,
2) définit la fréquence des mutations observées et permet de définir 3 mutations principales associées à la maladie de Crohn. Ainsi, il est possible, grâce à ce travail, de définir une stratégie d'étude du gène pour la recherche de variants morbides à savoir : premièrement typage des 3 mutations principales, deuxièmement recherche de mutations dans les 7 derniers exons, troisièmement recherche d'autres variants de séquence.
3) définit les modalités pratiques de recherche de ces mutations en signalant leur position et leur nature. En effet, il est ensuite aisé à l'homme du métier de mettre au point des méthodes de typage et de séquençage selon son expertise personnelle. On peut citer en particulier la possibilité de faire les génotypages des 3 mutations principales par PCR suivie de digestion enzymatique et électrophorèse, étude des profils de migration par dHPLC, DGGE ou SSCP, oligoligation, microséquençage, etc.
4) démontre l'indépendance des mutations les plus fréquentes qui ne sont pas observées sur le même chromosome dans cette population étendue et variée. Cette information permet de classer de façon fiable les sujets en hétérozygotes composites (ayant deux mutations) comme porteur à une double dose de variations intragéniques.
5) démontre que la plus grande proportion des mutations n'entraîne qu'un effet nul ou minime sur le risque de rectocolite hémorragique. Ce résultat permet d'envisager d'aider le clinicien dans le diagnostic différentiel entre ces deux maladies. En effet, dans environ 10 % des cas, les maladies inflammatoires de l'intestin restent inclassées malgré les examens biologiques, radiologiques et endoscopiques.
6) définit un risque relatif et absolu de la maladie pour les génotypes les plus fréquents. Ce résultat pose les bases d'un diagnostic prédictif potentiellement utile dans une démarche de suivi ou d'intervention préventive dans les populations à risque, en particulier, les apparentés de malades.
7) démontre l'existence d'un effet dose pour le gène IBD1 et confirme le caractère en partie récessif de la prédisposition génétique à la maladie de Crohn. Il permet donc de poser les bases d'un conseil génétique et d'un diagnostic préclinique intrafamilial.

Notons enfin qu'une mutation supplémentaire du domaine NBD a été isolée dans une deuxième famille porteuse d'un syndrome de Blau. La rareté des deux événements dans 2 familles différentes suffit à confirmer l'implication de ce gène dans le syndrome de Blau et dans les maladies granulomateuses en générale.

L'ensemble de ces données apporte un outil diagnostique directement applicable et utile au praticien dans sa pratique quotidienne.

Le gène IBD1prox, situé dans la région promotrice de IBD1, et dont la séquence partielle est dévoilée dans la présente invention, peut lui aussi avoir un rôle important dans la régulation de l'apoptose cellulaire et du processus inflammatoire, comme suggéré par son expression différentielle dans les cellules matures du système immunitaire. La forte association rapportée dans ce travail entre le marqueur de polymorphisme ctg35ExC (situé dans la région transcrite du gène) et la maladie de Crohn, plaide aussi très fortement en faveur de cette hypothèse.

Les maladies inflammatoires de l'intestin sont des maladies génétiques complexes pour lesquelles, à ce jour, aucun gène de susceptibilité n'avait été identifié avec certitude. L'invention a permis de l'identification du premier gène de susceptibilité à la maladie de Crohn, par une démarche de clonage positionnel (ou génétique reverse). Il s'agit là de la première localisation génétique obtenue par une telle approche pour une maladie génétique complexe, ce qui démontre son utilité et sa faisabilité, au moins dans certains cas dans les maladies génétiques complexes.

La présente invention concerne aussi un acide nucléique purifié ou isolé caractérisé en ce qu'il code pour un polypeptide possédant un fragment continu d'au moins 200 acides aminés d'une protéine choisie parmi SEQ ID N° 2 et SEQ ID N° 5.

### Références

Auphan et al. (1995) Science 270, 286-90.
Asakawa et al. (1997), Gene, 191,69
Becker et al. (1998), Proc Natl Acad Sci USA, 95, 9979
Bertin et al. (1999), J Biol Chem, 274, 12955
Buckholz, (1993), Curr. Op. Biotechnology 4, 538.
Carter, (1993) Curr. Op. Biotechnology 3, 533.
Cho et al. (1998), Proc Natl Acad Sci USA, 95, 7502.
Duck et al. (1990), Biotechniques, 9, 142.
Edwards et Aruffo (1993), Curr. Op. Biotechnology, 4, 558.
Epstein (1992) Médecine/Sciences, 8, 902.
Guatelli et al. (1990), Proc. Natl. Acad. Sci. USA 87: 1874.
Hugot et al. (1996), Nature, 379, 821.
Inohara et al. (1999) J Biol Chem, 274, 14560.
Inohara et al. (2000) J. Biol. Chem.
Kievitis et al. (1991), J. Virol. Methods, 35, 273.
Kim et al., (1996) Genomics, 34, 213.
Köhler et Milstein. (1975) Nature 256, 495.
Kwoh, et al. (1989), Proc. Natl. Acad. Sci. USA, 86, 1173.
Landegren et al. (1988) Science 241, 1077.
Lander et Kruglyak (1995) Nat Genet, 11, 241.
Luckow (1993), Curr. Op. Biotechnology 4, 564.
Martin et al. (2000), Am. J. Hum. Genet. 67 : 146-54.
Matthews et al. (1988), Anal. Biochem., 169, 1-25.
McKay (1999) Gastroenterol. 13, 509-516.
Miele et al. (1983), J. Mol. Biol., 171,281.
Neddleman et Wunsch (1970) J. Mol. Biol. 48 : 443
Ogura et al. (2000), J. Biol. Chem.
Olins et Lee (1993), Curr. Op. Biotechnology 4 : 520.
Perricaudet et al. (1992). La Recherche 23 : 471.
Pearson et Lipman (1988) Proc. Natl. Acad. Sci. USA 85 : 2444
Poltorak et al. (1998) Sciences 282, 2085-8.
Rioux et al. (1998) Gastroenterology, 115: 1062.
Rohlmann et al. (1996) Nature Biotech. 14 : 1562.
Rolfs, A. et al. (1991), Berlin : Springer-Verlag.
Rouquier et al. (1994), Anal Biochem 217, 205.
Sambrook et al. (1989) Molecular cloning : a laboratory manual. 2^{nd} Ed. Cold Spring Harbor Lab., Cold Spring Harbor, New York.
Satsangi et al. (1996), Nat Genet, 14: 199.
Schreiber et al. (1998) Gut 42, 477-84.
Segev, (1992), Kessler C. Springer Verlag, Berlin, New-York, 197-205.
Smith et Waterman (1981) Ad. App. Math. 2 : 482
Stewart et Yound (1984), Solid phase peptides synthesis, Pierce Chem. Company, Rockford, 111, 2ème éd., (1984).
Spielman et al. (1993) Am J Hum Genet, 52, 506.
Sundberg et al. (-1994) Gastroenterology 107, 1726-35.
Temin, (1986) Retrovirus vectors for gene transfer. In Kucherlapati R, ed. Gene Transfer, New York, Plenum Press, 149-187.
Tromp et al. (1996) Am J Hum Genet, 59 : 1097.
Wahl et al. (1998) B. J. Clin. Invest.> 101, 1163-74.
Walker (1992), Nucleic Acids Res. 20 : 1691.

## Revendications

1. Acide nucléique purifié ou isolé, **caractérisé en ce qu'**il comprend une séquence nucléique choisie dans le groupe de séquences suivantes :
a) SEQ ID N° 1 et SEQ ID N° 3 ;
b) la séquence complémentaire ou la séquence d'ARN correspondant à une séquence telle que définie en a).

2. Acide nucléique purifié ou isolé selon la revendication 1, **caractérisé en ce qu'**il comprend ou est constitué de SEQ ID N° 1, la séquence complémentaire ou la séquence d'ARN correspondant à cette séquence.

3. Polypeptide isolé **caractérisé en ce qu'**il comprend un polypeptide correspondant à SEQ ID N° 2.

4. Polypeptide selon la revendication 3, **caractérisé en ce qu'**il est constitué de SEQ ID N° 2.

5. Vecteur de clonage et/ou d'expression comprenant un acide nucléique selon l'une des revendications 1 et 2 ou codant pour un polypeptide selon l'une des revendications 3 et 4.

6. Cellule hôte **caractérisée en ce qu'**elle est transformée par un vecteur selon la revendication 5.

7. Animal, excepté l'homme, **caractérisé en ce qu'**il comprend une cellule selon la revendication 6.

8. Utilisation *in vitro* d'un acide nucléique selon l'une des revendications 1 et 2 comme nucléotide sens ou antisens.

9. Utilisation d'une séquence d'acide nucléique selon l'une des revendications 1 et 2 pour la production d'un polypeptide recombinant.

10. Procédé d'obtention d'un polypeptide recombinant **caractérisé en ce que** l'on cultive une cellule selon la revendication 6 dans des conditions permettant l'expression dudit polypeptide et que l'on récupère ledit polypeptide recombinant.

11. Anticorps monoclonal ou polyclonal **caractérisé en ce qu'**il lie sélectivement un polypeptide selon la revendication 4.

12. Procédé de détection d'un polypeptide selon l'une des revendications 3 et 4, **caractérisé en ce qu'**il comprend les étapes suivantes :
a) mise en contact d'un échantillon biologique avec un anticorps selon la revendication 11 ;
b) mise en évidence du complexe antigène-anticorps formé.

13. Trousse de réactifs pour la mise en oeuvre d'un procédé selon la revendication 12, **caractérisée en ce qu'**elle comprend :
a) un anticorps monoclonal ou polyclonal selon la revendication 11;
b) éventuellement des réactifs pour la constitution d'un milieu propice à la réaction immunologique ;
c) les réactifs permettant la détection du complexe antigène-anticorps produit lors de la réaction immunologique.

14. Méthode de diagnostic et/ou d'évaluation pronostique d'une maladie inflammatoire et/ou immune ou d'un cancer **caractérisée en ce qu'**on détermine à partir d'un prélèvement biologique d'un patient la présence d'au moins une mutation et/ou une altération d'expression du gène correspondant à SEQ ID N° 1 ou SEQ ID N° 3 par l'analyse de tout ou partie d'une séquence nucléique correspondant audit gène.

15. Puce à ADN **caractérisée en ce qu'**elle contient une séquence nucléique selon l'une des revendications 1 et 2.

16. Puce à protéines **caractérisée en ce qu'**elle contient un polypeptide selon l'une des revendications 3 et 4, ou un anticorps selon la revendication 11.

17. Procédé de détection et/ou de dosage d'un acide nucléique selon l'une des revendications 1 et 2 dans un échantillon biologique, **caractérisé en ce qu'**il comprend les étapes suivantes :
a) mise en contact d'un polynucléotide selon l'une des revendications 1 et 2, marqué ;
b) détection et/ou dosage de l'hybride formé entre ledit polynucléotide et l'acide nucléique de l'échantillon biologique.

18. Procédé de détection et/ou de dosage d'un acide nucléique selon l'une des revendications 1 et 2 dans un échantillon biologique, **caractérisé en ce qu'**il comprend une étape d'amplification des acides nucléiques dudit échantillon biologique à l'aide d'amorces correspondant à des fragments d'au moins 15 nucléotides consécutifs de SEQ ID N° 1 ou SEQ ID N° 3.

19. Procédé de criblage de composés capables de se fixer à un polypeptide de séquence SEQ ID N° 2, **caractérisé en ce qu'**il comprend les étapes de mise en contact d'un polypeptide selon l'une des revendications 3 et 4, d'une cellule selon la revendication 6, ou d'un mammifère selon la revendication 7, avec un composé candidat et de détection de la formation d'un complexe entre ledit composé candidat et ledit polypeptide.

20. Procédé de criblage de composés capables d'interagir *in vitro* ou *in vivo* avec un acide nucléique selon l'une des revendications 1 et 2, **caractérisé en ce qu'**il comprend les étapes de mise en contact d'un acide nucléique selon l'une des revendications 1 et 2, d'une cellule selon la revendication 6, ou d'un mammifère selon la revendication 7, avec un composé candidat et de détection de la formation d'un complexe entre ledit composé candidat et ledit acide nucléique

21. Composé **caractérisé en ce qu'**il est choisi parmi
a) un acide nucléique selon l'une des revendications 1 et 2 ;
b) un polypeptide selon l'une des revendications 3 et 4 ;
c) un vecteur selon la revendication 5 ;
d) une cellule selon la revendication 6 ; et
e) un anticorps selon la revendication 11 ;
à titre de médicament.

22. Composé selon la revendication 21, pour la prévention et/ou le traitement d'une maladie inflammatoire et/ou immune ou d'un cancer associé à la présence d'au moins une mutation du gène correspondant à SEQ ID N° 1.

## Patentansprüche

1. Gereinigte oder isolierte Nukleinsäure, **dadurch gekennzeichnet, dass** sie eine Nukleinsäuresequenz umfasst, welche ausgewählt wird aus der Gruppe von folgenden Sequenzen:
a) SEQ ID NO.1 und SEQ ID NO.3;
b) der komplementären Sequenz oder der RNA-Sequenz, welche einer Sequenz, wie in a) definiert, entspricht.

2. Gereinigte oder isolierte Nukleinsäure nach Anspruch 1, **dadurch gekennzeichnet, dass** sie folgendes umfasst oder aus folgendem besteht: SEQ ID NO. 1, die komplementäre Sequenz, oder die dieser Sequenz entsprechende RNA-Sequenz.

3. Isoliertes Polypeptid, **dadurch gekennzeichnet, dass** es ein Polypeptid, welches SEQ ID NO.2 entspricht, umfasst.

4. Polypeptid nach Anspruch 3, **dadurch gekennzeichnet, dass** es aus SEQ ID NO.2 besteht.

5. Klonierungs- und/oder Expressionsvektor, welcher eine Nukleinsäure nach einem der Ansprüche 1 und 2 oder eine, die ein Polypeptid nach einem der Ansprüche 3 und 4 kodiert, umfasst.

6. Wirtszelle, **dadurch gekennzeichnet, dass** sie durch einen Vektor nach Anspruch 5 transformiert ist.

7. Tier, mit Ausnahme des Menschen, **dadurch gekennzeichnet, dass** es eine Zelle nach Anspruch 6 umfasst.

8. *In vitro*-Verwendung einer Nukleinsäure nach einem der Ansprüche 1 und 2 als Sinn- oder Antisinn-Nukleotid.

9. Verwendung einer Nukleinsäuresequenz nach einem der Ansprüche 1 und 2 für die Herstellung eines rekombinanten Polypeptids.

10. Verfahren zur Herstellung eines rekombinanten Polypeptids, **dadurch gekennzeichnet, dass** man eine Zelle nach Anspruch 6 unter Bedingungen, welche die Expression des Polypeptids erlauben, kultiviert und dass man das rekombinante Polypeptid gewinnt.

11. Monoklonaler oder polyklonaler Antikörper, **dadurch gekennzeichnet, dass** er selektiv ein Polypeptid nach Anspruch 4 bindet.

12. Verfahren zum Nachweis eines Polypeptids nach einem der Ansprüche 3 und 4, **dadurch gekennzeichnet, dass** es die folgenden Schritte umfasst:
a) Inkontaktbringen einer biologischen Probe mit einem Antikörper nach Anspruch 11;
b) Nachweisen des gebildeten Antigen-Antikörper-Komplexes.

13. Reagenzienkit für das Ausführen eines Verfahrens nach Anspruch 12, **dadurch gekennzeichnet, dass** er umfasst:
a) einen monoklonalen oder polyklonalen Antikörper nach Anspruch 11;
b) gegebenenfalls Reagenzien für die Bildung eines Mediums, welches für die immunologische Reaktion günstig ist;
c) die Reagenzien, die den Nachweis des während der immunologischen Reaktion erzeugten Antigen-Antikörper-Komplexes erlauben.

14. Diagnose- und/oder prognostisches Bewertungsverfahren einer entzündlichen und/oder das Immunsystem betreffenden Erkrankung oder einer Krebserkrankung, **dadurch gekennzeichnet, dass** man ausgehend von einer biologischen Probe eines Patienten das Vorhandensein von wenigstens einer Mutation und/oder eine Veränderung der Expression des SEQ ID NO.1 oder SEQ ID NO.3 entsprechenden Gens durch die Analyse der Gesamtheit oder eines Teils einer Nukleinsäuresequenz, welche dem Gen entspricht, bestimmt.

15. DNA-Chip, **dadurch gekennzeichnet, dass** er eine Nukleinsäuresequenz nach einem der Ansprüche 1 und 2 enthält.

16. Chip mit Proteinen, **dadurch gekennzeichnet, dass** er ein Polypeptid nach einem der Ansprüche 3 und 4 oder einen Antikörper nach Anspruch 11 enthält.

17. Verfahren zum Nachweis und/oder zur quantitativen Bestimmung einer Nukleinsäure nach einem der Ansprüche 1 und 2 in einer biologischen Probe, **dadurch gekennzeichnet, dass** es die folgenden Schritte umfasst:
a) Inkontaktbringen eines markierten Polynukleotids nach einem der Ansprüche 1 und 2;
b) Nachweis und/oder quantitative Bestimmung des zwischen dem Polynukleotid und der Nukleinsäure der biologischen Probe gebildeten Hybrids.

18. Verfahren zum Nachweis und/oder zur quantitativen Bestimmung einer Nukleinsäure nach einem der Ansprüche 1 und 2 in einer biologischen Probe, **dadurch gekennzeichnet, dass** es einen Amplifizierungsschritt der Nukleinsäuren der biologischen Probe mit Hilfe von Primern, die Fragmenten von wenigstens 15 aufeinanderfolgenden Nukleotiden von SEQ ID NO.1 oder SEQ ID NO.3 entsprechen, umfasst.

19. Verfahren zum Screenen auf Verbindungen, die in der Lage sind, an ein Polypeptid mit der Sequenz SEQ ID NO.2 zu binden, **dadurch gekennzeichnet, dass** es die Schritte umfasst, ein Polypeptid nach einem der Ansprüche 3 und 4, eine Zelle nach Anspruch 6 oder ein Säugetier nach Anspruch 7 mit einer Kandidatenverbindung in Kontakt zu bringen und die Bildung eines Komplexes zwischen der Kandidatenverbindung und dem Polypeptid nachzuweisen.

20. Verfahren zum Screenen auf Verbindungen, die zur Wechselwirkung *in vitro* oder *in vivo* mit einer Nukleinsäure nach einem der Ansprüche 1 und 2 in der Lage sind, **dadurch gekennzeichnet, dass** es die Schritte umfasst, eine Nukleinsäure nach einem der Ansprüche 1 und 2, eine Zelle nach Anspruch 6 oder ein Säugetier nach Anspruch 7 mit einer Kandidatenverbindung in Kontakt zu bringen und die Bildung eines Komplexes zwischen der Kandidatenverbindung und der Nukleinsäure nachzuweisen.

21. Verbindung, **dadurch gekennzeichnet, dass** sie ausgewählt wird aus
a) einer Nukleinsäure nach einem der Ansprüche 1 und 2;
b) einem Polypeptid nach einem der Ansprüche 3 und 4;
c) einem Vektor nach Anspruch 5;
d) einer Zelle nach Anspruch 6; und
e) einem Antikörper nach Anspruch 11;
als Arzneimittel.

22. Verbindung nach Anspruch 21 für die Verhütung und/oder die Behandlung einer entzündlichen und/oder das Immunsystem betreffenden Erkrankung oder einer Krebserkrankung, welche mit dem Vorhandensein von wenigstens einer Mutation des SEQ ID NO.1 entsprechenden Gens verbunden ist.

## Claims

1. Purified or isolated nucleic acid, **characterized in that** it comprises a nucleic acid sequence chosen from the following group of sequences:
a) SEQ ID No. 1 and SEQ ID No. 3;
b) the complementary sequence or the RNA sequence corresponding to a sequence as defined in a).

2. Purified or isolated nucleic acid according to Claim 1, **characterized in that** it comprises or consists of SEQ ID No. 1, or the complementary sequence or the RNA sequence corresponding to this sequence.

3. Isolated polypeptide, **characterized in that** it comprises a polypeptide corresponding to SEQ ID No. 2.

4. Polypeptide according to Claim 3, **characterized in that** it consists of SEQ ID No. 2.

5. Cloning and/or expression vector, comprising a nucleic acid according to either of Claims 1 and 2 or encoding a polypeptide according to either of Claims 3 and 4.

6. Host cell, **characterized in that** it is transformed with a vector according to Claim 5.

7. Animal, except a human, **characterized in that** it comprises a cell according to Claim 6.

8. Use, *in vitro*, of a nucleic acid according to either of Claims 1 and 2, as a sense or antisense nucleotide.

9. Use of a nucleic acid sequence according to either of Claims 1 and 2, for producing a recombinant polypeptide.

10. Method for obtaining a recombinant polypeptide, **characterized in that** a cell according to Claim 6 is cultured under conditions which allow the expression of said polypeptide, and **in that** said recombinant polypeptide is recovered.

11. Monoclonal or polyclonal antibody, **characterized in that** it selectively binds a polypeptide. according to Claim 4.

12. Method for detecting a polypeptide according to either of Claims 3 and 4, **characterized in that** it comprises the following steps:
a) bringing a biological sample into contact with an antibody according to Claim 11;
b) demonstrating the antigen-antibody complex formed.

13. Kit of reagents for carrying out a method according to Claim 12, **characterized in that** it comprises:
a) a monoclonal or polyclonal antibody according to Claim 11;
b) optionally, reagents for constituting a medium suitable for the immunoreaction;
c) the reagents for detecting the antigen-antibody complex produced during the immunoreaction.

14. Method of diagnosis and/or of prognostic assessment of an inflammatory and/or immune disease or of a cancer, **characterized in that** the presence of at least one mutation and/or a deleterious modification of expression of the gene corresponding to SEQ ID No. 1 or SEQ ID No. 3 is determined, using a biological specimen from a patient, by analysing all or part of a nucleic acid sequence corresponding to said gene.

15. DNA chip, **characterized in that** it contains a nucleic acid sequence according to either of Claims 1 and 2.

16. Protein chip, **characterized in that** it contains a polypeptide according to either of Claims 3 and 4, or an antibody according to Claim 11.

17. Method for detecting and/or assaying a nucleic acid according to either of Claims 1 and 2, in a biological sample, **characterized in that** it comprises the following steps:
a) bringing a polynucleotide according to either of Claims 1 and 2, that has been labelled, into contact;
b) detecting and/or assaying the hybrid formed between said polynucleotide and the nucleic acid of the biological sample.

18. Method for detecting and/or assaying a nucleic acid according to either of Claims 1 and 2, in a biological sample, **characterized in that** it comprises a step of amplification of the nucleic acids of said biological sample, using primers corresponding to fragments of at least 15 consecutive nucleotides of SEQ ID No. 1 or SEQ ID No. 3.

19. Method for screening compounds capable of attaching to a polypeptide of sequence SEQ ID No. 2, **characterized in that** it comprises the steps of bringing a polypeptide according to either of Claims 3 and 4, a cell according to Claim 6 or a mammal according to Claim 7 into contact with a candidate compound, and detecting the formation of a complex between said candidate compound and said polypeptide.

20. Method for screening compounds capable of interacting, *in vitro* or *in vivo,* with a nucleic acid according to either of Claims 1 and 2, **characterized in that** it comprises the steps of bringing a nucleic acid according to either of Claims 1 and 2, a cell according to Claim 6 or a mammal according to Claim 7 into contact with a candidate compound, and detecting the formation of a complex between said candidate compound and said nucleic acid.

21. Compound, **characterized in that** it is chosen from
a) a nucleic acid according to either of Claims 1 and 2;
b) a polypeptide according to either of Claims 3 and 4;
c) a vector according to Claim 5;
d) a cell according to Claim 6; and
e) an antibody according to Claim 11,
as a medicinal product.

22. Compound according to Claim 21, for preventing and/or treating an inflammatory and/or immune disease, or a cancer, associated with the presence of at least one mutation of the gene corresponding to SEQ ID No. 1.
